# EUROPEAN PATENT APPLICATION

(11) **EP 4 624 472 A1**
(43) Date of publication of application: **01.10.2025**
(21) Application number: 23895047.1
(22) Date of filing: 22.11.2023
(51) Int. Cl.: C07D 487/04, A61K 47/55, A61P 35/00, C07D 413/14, C07D 403/10, A61K 31/5377

(54) **DEGRADER INCLUDING NOVEL C-MET PROTEIN LIGAND AND PHARMACEUTICAL COMPOSITION INCLUDING SAME**

(30) Priority: 22.11.2022 KR 20220157345
(71) Applicant: Innocure Therapeutics, Inc., Seongnam-si Gyeonggi-do 13488 (KR)
(72) Inventor: YOO, Hye Dong, Seongnam-si Gyeonggi-do 13488 (KR); SHIN, Young Jun, Seongnam-si Gyeonggi-do 13488 (KR); KIM, Bo Kyoung, Seongnam-si Gyeonggi-do 13488 (KR); YANG, Dong Sik, Seongnam-si Gyeonggi-do 13488 (KR); KIM, Sung Jin, Seongnam-si Gyeonggi-do 13488 (KR); KOOK, Seung Hoon, Seongnam-si Gyeonggi-do 13488 (KR)
(74) Representative: Potter Clarkson
(86) International application number: PCT/KR2023/018997
(87) International publication number: WO 2024/112120

(57) **Abstract**

A TPD compound is disclosed.

The present invention relates to a novel PROTAC compound binding to a cMET protein and a pharmaceutical composition comprising the same. The present invention relates to a PROTAC compound that binds to a cMET protein, and since it can bind to a cMET protein and degrade it, it can be useful for the treatment or prevention of diseases related to cMET protein. The compound of the present invention has an excellent anticancer effect and can also induce the degradation of cMET proteins, which can show a therapeutic effect on cMET and related diseases.

## Description

### [Technical Fields]

The present invention relates to a novel PROTAC compound binding to a cMET protein and a pharmaceutical composition comprising the same.

### [Background Skills]

A PROTAC is a heterodimer molecule in which a ligand of a target protein and a ligand that binds to an E3 ligase are linked via a linker. PROTAC binds to both proteins simultaneously, bringing the target protein very close to E3 ligase, which recognizes the target protein as a substrate and triggers polyubiquitination and subsequent proteasome degradation. Since this principle can effectively remove specific proteins from cells, PROTAC can be used as a chemical probe to study the function of target proteins, and furthermore, it has a high potential as a treatment for diseases. The term TPD (Targeted Protein Degradation) is sometimes used instead of the term PROTAC.

In the present invention, a novel target protein-binding moiety binding to cMET proteins was prepared and a novel E3 ligase-binding moiety was prepared, and a new E3 ligase-binding moiety and a novel cMET protein-targeted binding moiety were connected through a linker to complete a PROTAC.

### [Detailed explanation of the invention]

### [Technical Challenges]

The present invention is intended to provide a PROTAC coupled with a novel E3 ligase binding moiety and a novel cMET protein-targeted binding moiety via a linker and a pharmaceutical composition comprising the same.

### [Technical Solution]

In order to accomplish the technical task, the present invention provides a TPD compound represented by the following Chemical Formula 1:
Compounds represented by the following Chemical Formula 1:

[Chemical Formula 1] cMET Targeted Protein-Binding Moiety (P)-{Linker (L)}p-E3 Ligase-Binding Moiety (E)

wherein,
E3 Ligase-Binding Moiety (E) is a compound represented by the following Chemical Formula 2; and
cMET Targeted Protein-Binding Moiety (P) is a compound represented with any of the following formulas 3 to 7; and
p is an integer of 0 or 1. 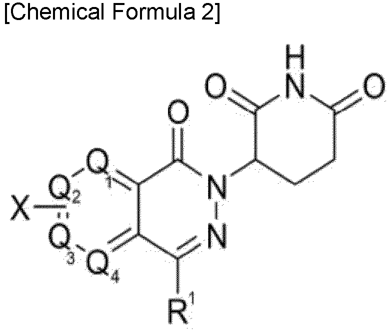 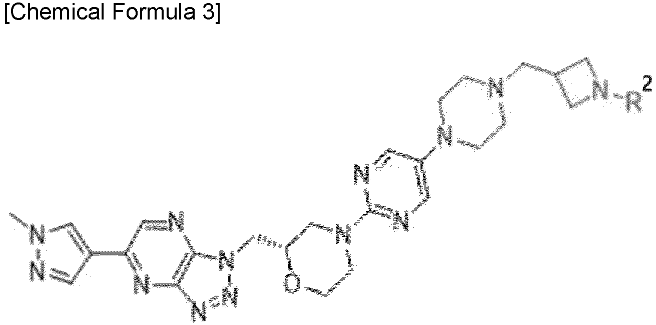 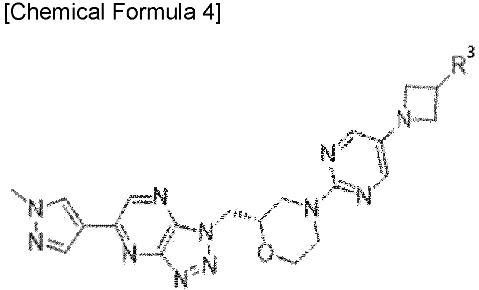 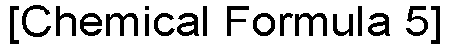 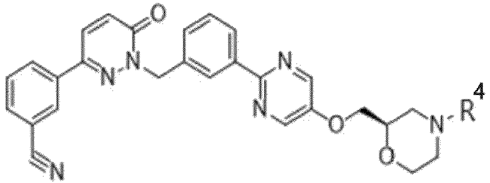 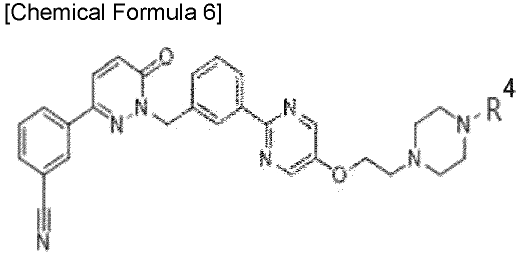 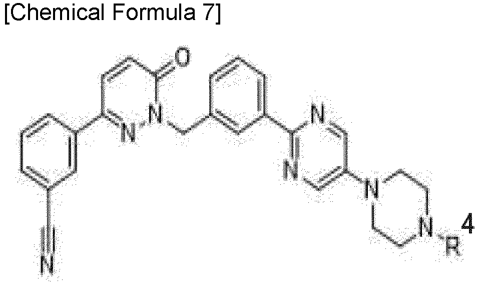 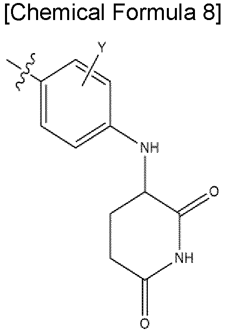
wherein,
X is hydrogen, halogen, amino, nitro, hydroxy, C1 to C6 straight or branched alkyl, or a 4 to 8 atom heterocyclic containing oxygen or nitrogen;
Q₁ to Q₄ are each independently C-F, C-Cl, C-H, C-X, or N, and at least any one of Q₁ to Q₄ is C-X;
Y is hydrogen, halogen, or a C1 to C6 a straight, branched or cyclic alkyloxy unsubstituted or substituted with halogen;
R¹ is hydrogen, nitro, amino, carbonyl, C1 to C6 straight, branched or cyclic alkyl, or C1 to C6 straight, branched, or cyclic alkyl substituted with halogen;
R² and R³ are each independently hydrogen, t-butoxycarbonyl, benzyloxycarbonyl, Tosyl, or unsubstituted or substituted C1 to C6 straight, branched or cyclic alkyl group; and
R⁴ is hydrogen, t-butoxycarbonyl group or benzyloxycarbonyl group.

In the present invention, one end of the linker (L) may be (i) connected to a compound in Chemical Formula 1 by substituting X in a compound of Chemical Formula 2; or
(ii) connected to nitrogen or oxygen atom of X in a compound of Chemical Formula 2.

In the present invention, the other end of the linker (L) may be (i) connected to the substituents R², R³ or R⁴ of any of the compounds of the Chemical Formulas 3 to 7 and connected to any one of the Chemical Formulas 3 to 7, or (ii) connected to the benzene ring of the structure of the Chemical Formula 8.

In one embodiment of the present invention, R² and R³ are each independently C1 to C6 straight, branched chain or cyclic alkyl group unsubstituted or substituted with halogen or R⁵,
wherein, R⁵ may be (i) a piperazine unsubstituted or substituted t-butoxycarbonyl, benzyloxycarbonyl, or Tosyl, (ii) a C1 to C6 alkoxy group unsubstituted or substituted with a benzyl, phenyl, pyridine, or Tosyl group substituted with R⁶ (wherein, R⁶ is C1 to C6 alkoxy, amino, t-butoxycarbonyl group, benzyloxycarbonyl group, or amino substituted with Tosyl group), or (iii) hydroxy group.

In another embodiment of the present invention, R² and R³ are each independently a C1 to C6 straight, branched or cyclic alkyl group unsubstituted or substituted with halogen or R⁵,
wherein, R⁵ may be (i) a piperazine unsubstituted or substituted with t-butoxycarbonyl or benzyloxycarbonyl group, (ii) a C1 to C6 alkoxy group unsubstituted or substituted with abenzyl, phenyl, pyridine, or Tosyl group substituted with R⁶ (wherein, R⁶ is C1 to C4 alkoxy, amino, t-butoxycarbonyl group, benzyloxycarbonyl group, or amino substituted with Tosyl group), or (iii) hydroxy group.

In another embodiment of the present invention, R² is hydrogen or a C1 to C6 straight, branched alkyl chain or cyclic alkyl group; and
R³ is a straight or branched, or cyclic alkyl group of C1 to C4 unsubstituted or substituted with R⁵,
wherein, R⁵ may be (i) a piperazine, (ii) an C1 to C4 alkoxy group unsubstituted or substituted with a benzyl, phenyl, or pyridine unsubstituted or substituted with R⁶ (wherein, R⁶ is an C1 to C4 alkoxy group or amino group of), or (iii) a hydroxy group.

In the present invention, the Linker (L) is
-(CH₂)ₘ-R⁷-(CH₂)ₙ- or
a formula selected from: 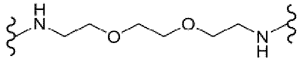 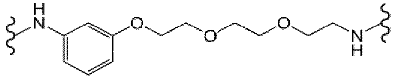 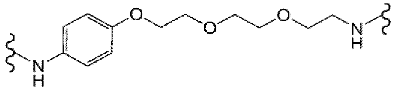 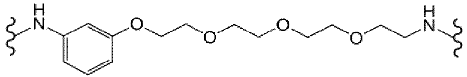 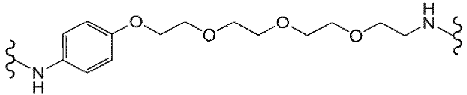 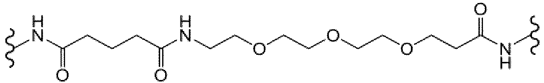
wherein, R⁷ is piperazine, piperidine, azetidine, benzene, triazole or pyrrolidine; and
m and n are each independently an integer selected from 0 to 5.

In another embodiment of the present invention, the linker L is selected from: 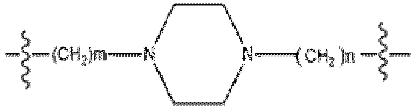 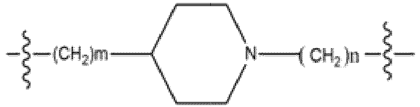 or 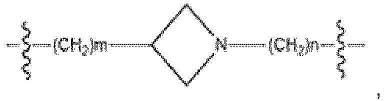 wherein, m and n are each independently an integer selected from 0 to 5.

In addition, the present invention relates to a compound with the following Chemical Formulas 3 or 4 or a pharmaceutically acceptable salt thereof: 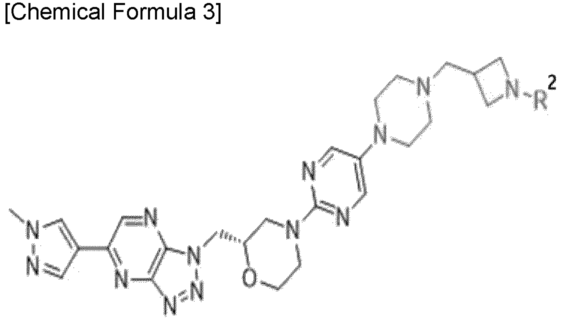 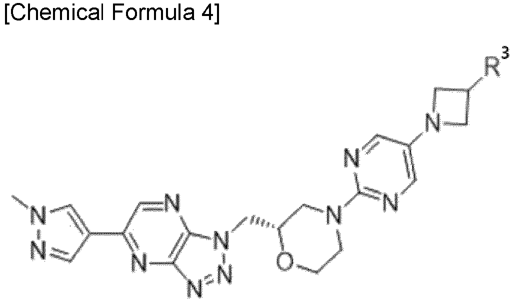 wherein,
R² and R³ are each independently hydrogen, t-butoxycarbonyl, benzyloxycarbonyl, Tosyl or a C1 to C6 straight, branched, or cyclic alkyl group unsubstituted or substituted.

In one embodiment of the present invention, R² and R³ are each independently a C1 to C6 straight, branched or cyclic alkyl group unsubstituted or substituted with halogen or R⁵,
wherein, R⁵ may be (i) a piperazine unsubstituted or substituted with t-butoxycarbonyl, benzyloxycarbonyl, or Tosyl, (ii) a C1 to C6 alkoxy group unsubstituted or substituted with a benzyl, phenyl, pyridine, or Tosyl group unsubstituted or substituted with R⁶ (wherein, R⁶ is an alkoxy group of C1 to C6, an amino group or an amino group substituted by a t-butoxycarbonyl group, benzyloxycarbonyl group, or a Tosyl group), or (iii) a hydroxy group.

In addition, in one embodiment of the present invention, R² and R³ are each independently a C1 to C6 straight, branched or cyclic alkyl group unsubstituted or substituted with halogen or R⁵,
wherein, R⁵ may be (i) a piperazine unsubstituted or substituted with t-butoxycarbonyl or benzyloxycarbonyl group, (ii) a C1 to C6 alkoxy group of unsubstituted or substituted with a benzyl, phenyl, pyridine, or Tosyl group substituted with R⁶ (wherein, R⁶ is C1 to C4 alkoxy, amino, t-butoxycarbonyl group, benzyloxycarbonyl group, or amino substituted with Tosyl group), or (iii) hydroxy group.

In addition, in an embodiment of the present invention, R² is hydrogen, or C1 to C6 a straight, branched or cyclic alkyl group; and
R³ is a straight, branched, or cyclic alkyl group of C1 to C4 unsubstituted or substituted with R⁵,
wherein, R⁵ may be (i) a piperazine, (ii) a C1 to C4 alkoxy group of unsubstituted or substituted with benzyl, phenyl or pyridine unsubstituted or substituted with R⁶ (wherein, R⁶ is C1 to C4 alkoxy or amino), or (iii) hydroxy group.

In addition, the present invention relates to any one of the following Chemical Formulas 5 to 7 or a pharmaceutically acceptable salt thereof: 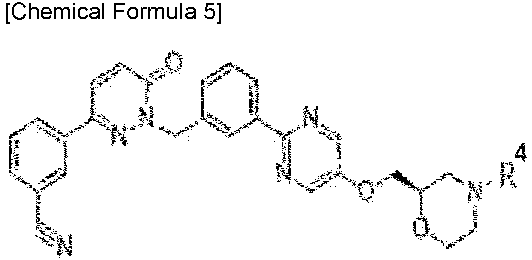 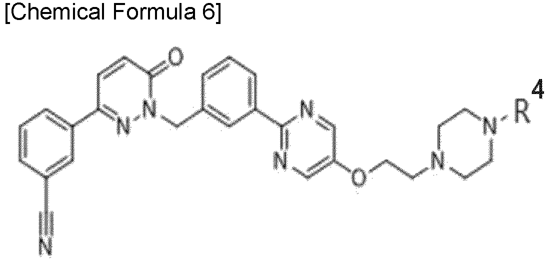 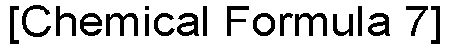 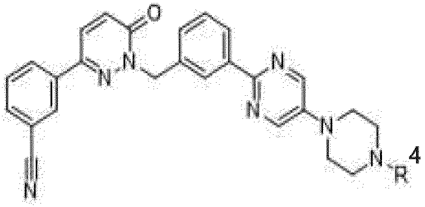 wherein,
R⁴ is hydrogen, t-butoxycarbonyl group or benzyloxycarbonyl group.

In addition to the TPD compounds of the Chemical Formula 1 of the present invention, compounds with Chemical Formulas 3 to 7 can bind to the cMET protein and provide an anticancer effect. Thus, the present invention provides a pharmaceutical composition for the treatment of cancer comprising any one of the Chemical Formulas 1 to 7.

### [Effect of Invention]

The present invention relates to a PROTAC compound that binds to a cMET protein, and since it can bind to a cMET protein and degrade it, it can be useful for the treatment or prevention of diseases related to cMET protein. The compound of the present invention has an excellent anticancer effect and can also induce the degradation of cMET proteins, which can show a therapeutic effect on cMET related diseases.

### [Brief description of drawing]

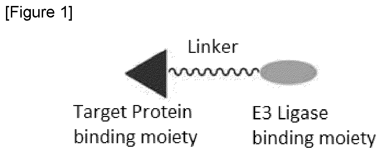

Figure 1 is a schematic of a PROTAC compound consisting of an E3 ligase-binding moiety, a linker, and a target protein-binding moiety.

### [Mode for Practicing the Invention]

The present invention is described in more detail below. However, the present invention can be implemented in various different forms, and the present invention is not limited by the embodiments described herein.

The terms used herein are used merely to describe a specific embodiment and are not intended to qualify the present invention. Expressions in the singular include plural expressions, unless the context clearly means otherwise. The 'inclusion' of a component in the specification of the present invention means that it may include more other components rather than excluding other components, unless there is a specifically contrary statement.

PROTAC according to the present invention may be a compound represented by the formula 1:

[Chemical Formula 1] E3 Ligase-Binding Moiety (E)-Linker (L)-cMET Target Protein-Binding Moiety (P)

The basic structure of the E3 ligase binding moiety according to the present invention can be prepared by the following Reaction Equation 1 or Reaction Equation 2.

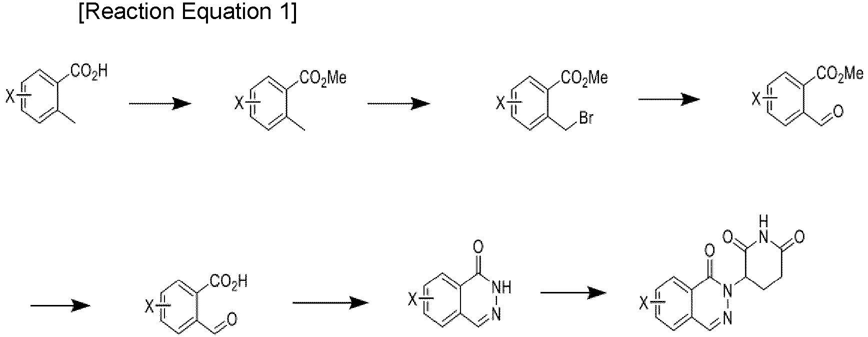

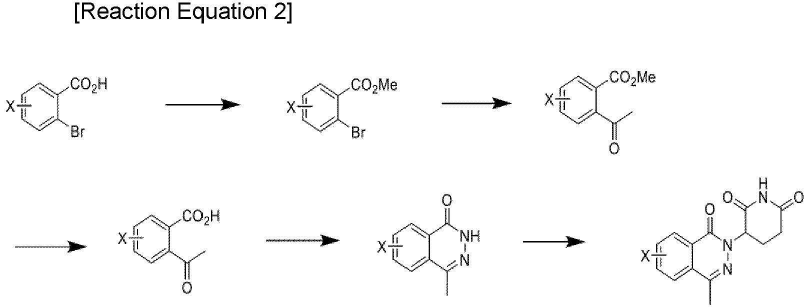

In the above Reaction Equations 1 or 2, X is hydrogen, halogen, amino, nitro, hydroxy, piperazine group or an C1 to C4 alkoxy. However, for the convenience of explanation, in the above equation, the substituent that can be bound to carbon in Q₁ to Q₄ of the compounds of Chemical Formula 1 is not shown, and only simple substituents such as hydrogen or methyl are shown among the R¹ substituents as an example.

In the following, a specific example of an E3 ligase-bound moiety compound is prepared using an embodiment.

### Example A: Preparation of an E3 ligase-coupled moiety compound

### Example A-1: Preparation of a compound

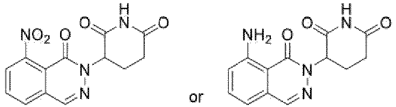

### (prepared according to Equation 1)

### 1-1) Preparation of methyl 2-methyl-6-nitrobenzoate

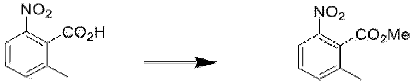

At room temperature, K2CO3 (19.1 g, 138 mmol) was added to a 2-methyl-6-nitrobenzoic acid (5 g, 27.6 mmol) solution of acetone (100 ml). After stirring for 30 minutes, methane iodide (19.6 g, 138 mmol) was added to the reactant and heated to 60oC for 6 hours. After cooling, the reactants were filtered and concentrated under reduced pressure. The product was used in the next reaction without additional purification. (5.45g, 98%) MS (ESI, m/z): [M+1]⁺ = [195.2].

### 1-2) Preparation of methyl 2-(bromomethyl)-6-nitrobenzoate

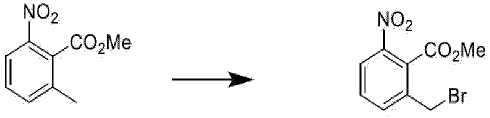

At room temperature, 1-bromomerolidine-2,5-dione (7.39 g, 41.4 mmol) and benzoyl benzene carboroxoate (0.67 g, 7.26 mmol) were sequentially added to a solution of methyl 2-methyl-6-nitrobenzoate (5.39 g, 27.6 mmol) in ClCH₂CH₂Cl (100 ml). The reactants were heated and refluxed for 3 hours. The point when the red color disappears is the time when the reaction is complete. After cooling, the reactants were washed with water, dried with MgSO₄, and concentrated under reduced pressure. The crude product was used in the next reaction without additional purification. (7.34g, 98%) MS (ESI, m/z): [M+1]⁺ = [274.4].

### 1-3) Preparation of methyl 2-formyl-6-nitrobenzoate

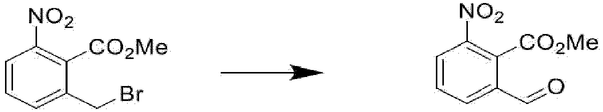

At room temperature, NMO (N-methylmorpholine N-oxide) (561 mg, 4.87 mmol) and 4Å molecular sieve were sequentially added to methyl 2-(bromomethyl)-6-nitrobenzoate (580 mg, 2.12 mmol) solution in DCM (30 ml). The reactants were stirred at room temperature for 2 hours. Molecular sieve was filtered and washed with DCM (10ml). The DCM layer was washed with water (50ml), dried with MgSO₄, and concentrated under reduced pressure. The residue was purified by column chromatography, and the title compound was obtained as a white solid. (350mg, 79.07%) MS (ESI, m/z): [M+1]⁺ = [210.0].

### 1-4) Preparation of 2-formyl-6-nitrobenzoic acid

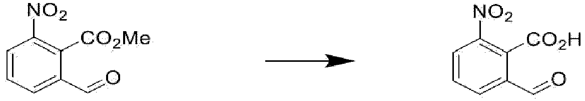

At room temperature, an aqueous solution of lithium (+1) hydroxyside (1.15 g, 47.8 mmol) was added to a solution of methyl 2-formyl-6-nitrobenzoate (2 g, 9.56 mmol) in THF (10 ml). After 2 h of agitation, the reactants were decompressively concentrated to dry the THF. After cooling to 0°C, the reactants were acidified with 1N HCl and the pH was adjusted to 4. The reactants were extracted twice with ethyl acetate (50ml). The combined ethyl acetate layer was dried with MgSO₄, and concentrated under reduced pressure to obtain white crystals (1.50g, 80.3%). MS (ESI, m/z): [M+1]⁺ = [195.2].

### 1-5) 8-Nitroptalazine-1(2H)-one Preparation

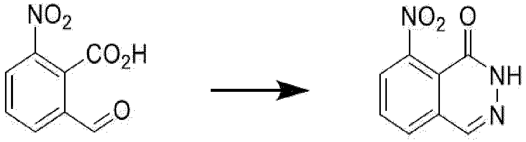

At room temperature, NH2NH2 monohydrate (417 mg, 8.33 mmol) was added to a 2-formyl-6-nitrobenzoic acid (1.2 g, 6.15 mmol) solution in methanol (10 ml). After stirring for 30 minutes, the reactants were heated to 80°C for 2 hours. The reactants were cooled to room temperature and concentrated under reduced pressure. The residue was poured into water (50ml) and extracted twice with ethyl acetate (50ml). The combined ethyl acetate layer was dried with MgSO₄, concentrated under reduced pressure, and white crystals were obtained (1g, 85.07%). MS (ESI, m/z): [M+1]⁺ = [191.8].

### 1-6) 3-(8-nitro-1-oxopthalazine-2(1H)-yl)piperidine-2,6-dione preparation

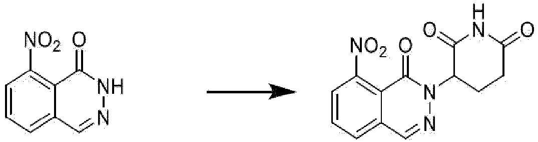

3-bromoperidin-2,6-dione (90 mg, 0.471 mmol) and K₂CO₃ (129 mg, 0.942 mmol) were sequentially added to 8-nitro-1,2-dihydrodroplasine-1-one (60 mg, 0.314 mmol) solution in DMF (1 ml). The reactants were heated to 85°C for 5 hours. After cooling, the reactants were poured into water (5 ml) and extracted twice with ethyl acetate (5 ml). The combined ethyl acetate layer was dried with MgSO₄ and concentrated under reduced pressure. The residue was purified by column chromatography, and the title compound was obtained as a white crystal (68 mg, 71.7%). MS (ESI, m/z): [M+1]⁺ = [302.6].

### 1-7) 3-(8-nitro-1-oxopthalazine-2(1H)-yl)piperidine-2,6-dione preparation

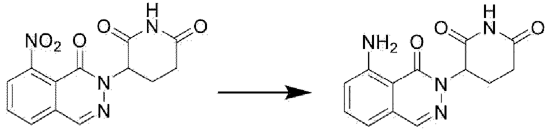

20 mg of 10% Pd/C (wet) (5 mg) of 20 mg was added to 3-(8-nitro-1-oxo-1,2-dihydrophoptalazine-2-yl-2-yl) solution of 3-(8-nitro-1-oxo-1,2-dihydrophoptalazine-2-yl) in methanol (5 ml) and stirred for 1 hour under hydrogen in the balloon. The solids were filtered, the filtrate was decompressively concentrated, and the title compound was obtained at a yield of 99%. MS (ESI, m/z): [M+1]⁺ = [272.3].

[NMR] ¹H NMR (400 MHz, DMSO-d₆) δ 10.95 (s, 1H), 8.10 (s, 1H), 7.46 (t, J = 7.83 Hz, 1H), 7.22 (br s, 2H), 6.86 (d, J = 7.83 Hz, 1H), 6.81(d, J = 7.34 Hz, 1H), 5.61 (br dd, J = 5.2, 11.92 Hz, 1H), 2.77 - 2.89 (m, 1H), 2.46 - 2.57 (m, 2H), 1.95 - 2.08 (m, 1H)

### Example A-2: Preparation of a compound

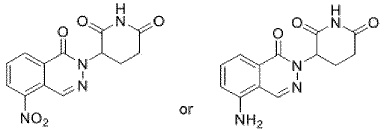

### 2-1) Preparation of methyl 2-methyl-3-nitrobenzoate

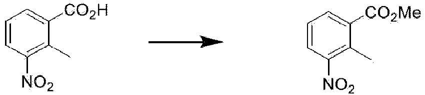

At room temperature, K₂CO₃ (19.1 g, 138 mmol) was added to a solution of 2-methyl-3-nitrobenzoic acid (5 g, 27.6 mmol) in acetone (100 ml). After stirring for 30 minutes, methane iodide (19.6 g, 138 mmol) was added to the reactant and heated to 60°C for 6 hours. After cooling, the reactants were filtered and concentrated under reduced pressure. The product was used in the next reaction without additional purification. (5.45g, 98%) MS (ESI, m/z): [M+1]⁺ = [195.3].

### 2-2) Preparation of methyl 2-(bromomethyl)-3-nitrobenzoate

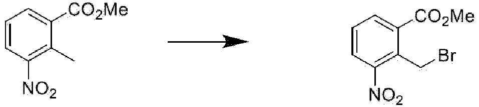

At room temperature, 1-bromophilidine-2,5-dione (7.39 g, 41.4 mmol) and benzoyl benzene carboforoxoate (0.67 g, 7.26 mmol) were sequentially added to a solution of methyl 2-methyl-3-nitrobenzoate (5.39 g, 27.6 mmol) in ClCH₂CH₂Cl (100 ml). The reactants were heated and refluxed for 3 hours. The point when the red color disappears is the time when the reaction is complete. After cooling, the reactants were washed with water, dried with MgSO₄, and concentrated under reduced pressure. The crude product was used in the next reaction without additional purification. (7.34g, 98%) MS (ESI, m/z): [M+1]⁺ = [274.4].

### 2-3) Preparation of methyl 2-formyl-3-nitrobenzoate

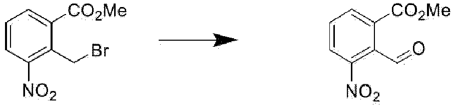

At room temperature, NMO (561mg, 4.87mmol) and 4Å molecular sieve were sequentially added to methyl 2-(bromomethyl)-3-nitrobenzoate (580mg, 2.12mmol) solution in DCM (30ml). The reactants were stirred at room temperature for 2 hours. Molecular sieve was filtered and washed with DCM (10ml). The DCM layer was washed with water (50ml), dried with MgSO₄, and concentrated under reduced pressure. The residue was purified by column chromatography, and the title compound was obtained as a white solid. (350mg, 79.07%) MS (ESI, m/z): [M+1]⁺ = [210.0].

### 2-4) Preparation of 2-formyl-3-nitrobenzoic acid

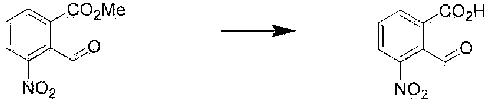

At room temperature, an aqueous solution of lithium (+1) hydroxyside (1.15 g, 47.8 mmol) was added to a solution of methyl 2-formyl-3-nitrobenzoate (2 g, 9.56 mmol) in THF (10 ml). After 2 hours of agitation, the reactants were concentrated under reduce pressure to remove THF. After cooling to 0°C, the residue were acidified with 1N HCl and the pH was adjusted to 4. The reactants were extracted twice with ethyl acetate (50ml). The combined ethyl acetate layer was dried with MgSO₄, and concentrated under reduced pressure to obtain white crystals (1.50g, 80.3%). MS (ESI, m/z): [M+1]⁺ = [195.2].

### 2-5) Preparation of 5-Nitroptalazine-1(2H)-one

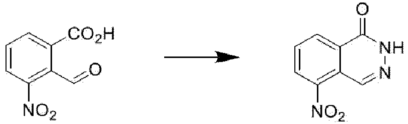

At room temperature, NH₂NH₂ monohydrate (417 mg, 8.33 mmol) was added to a 2-formyl-3-nitrobenzoic acid (1.2 g, 6.15 mmol) solution in methanol (10 ml). After stirring for 30 minutes, the reactants were heated to 80°C for 2 hours. The reactants were cooled to room temperature and concentrated under reduced pressure. The residue was poured into water (50ml) and extracted twice with ethyl acetate (50ml). The combined ethyl acetate layer was dried with MgSO₄, concentrated under reduced pressure, and white crystals were obtained (1g, 85.07%). MS (ESI, m/z): [M+1]⁺ = [191.8].

### 2-6) Preparation of 3-(5-nitro-1-oxopthalazine-2(1H)-yl)piperidine-2,6-dione

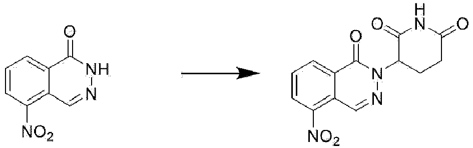

3-bromoperidin-2,6-dione (90 mg, 0.471 mmol) and K₂CO₃ (129 mg, 0.942 mmol) were sequentially added to 5-nitropthalazine-1(2H)-one (60 mg, 0.314 mmol) solution in DMF (1 ml). The reactants were heated to 85°C for 5 hours. After cooling, the reactants were poured into water (5 ml) and extracted twice with ethyl acetate (5 ml). The combined ethyl acetate layer was dried with MgSO₄ and concentrated under reduced pressure. The residue was purified by column chromatography, and the title compound was obtained as a white crystal (68 mg, 71.7%). MS (ESI, m/z): [M+1]⁺ = [302.4].

### 2-7) Preparation of 3-(5-amino-1-oxopthalazine-2(1H)-yl)piperidine-2,6-dione

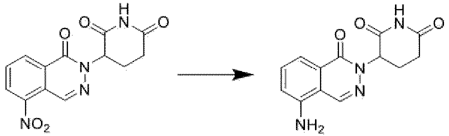

20 mg of 10% Pd/C (wet) (5 mg) of 20 mg was added to 3-(5-nitro-1-oxopthalazine-2(1H)-yl)piperidine-2,6-dione (25 mg, 0.82 mmol) solution in methanol (5 ml) and stirred for 1 hour under hydrogen in the balloon. The solids were filtered, the filtrate was decompressively concentrated, and the title compound was obtained at a yield of 99%. MS (ESI, m/z): [M+1]⁺ = [272.3].

[NMR] ¹H NMR (400 MHz, DMSO-d₆) δ 10.95 (s, 1H), 8.20 -8.41 (m, 3H), 8.08 (s, 1H), 7.83 (d, J = 8.93 Hz, 1H), 6.95 (d, J = 10.5 Hz, 1H), 5.64 (br dd, J = 4.83, 11.31 Hz, 1H), 2.62 - 2.89 (m, 1H), 2.52 - 2.60 (m, 2H), 1.86 - 2.07 (m, 1H)

### Example A-3: Preparation of a compound

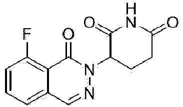

### 3-1) Preparation of methyl 2-fluoro-6-methylbenzoate

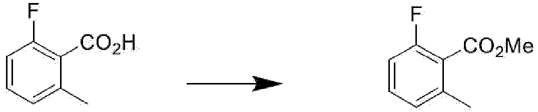

At room temperature, K₂CO₃ (44.8 g, 324 mmol) was added to a solution of 2-fluoro-6-methylbenzoic acid (10 g, 64.9 mmol) in acetone (200 ml). After stirring for 30 minutes, methane iodide (46 g, 324 mmol) was added to the reactant and heated to 60°C for 6 hours. After cooling, the reactants were filtered and concentrated under reduced pressure. The product was used in the next reaction without additional purification. (11.2g, yield 103%) MS (ESI, m/z): [M+1]⁺ = [168.3].

### 3-2) Preparation of methyl 2-(bromomethyl)-6-fluorobenzoate

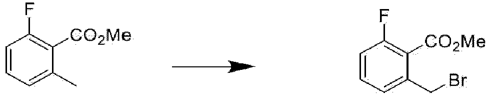

At room temperature, 1-bromomerrolidine-2,5-dione (24.7 g, 139 mmol) and benzoyl benzene carboforoxoate (1.53 g, 6.30 mmol) were sequentially added to a solution of methyl 2-fluoro-6-methylbenzoate (21.2 g, 126 mmol) in ClCH₂CH₂Cl (250 ml). The reactants were heated and refluxed for 16 hours. The point when the red color disappears is the time when the reaction is complete. After cooling, the reactants were washed with water, dried with MgSO4, and concentrated under reduced pressure. The crude products were used in the following reactions without additional purification. (35g, yield 112%) MS (ESI, m/z): [M+1]⁺ = [245.9].

### 3-3) Preparation of methyl 2-fluoro-6-formylbenzoate

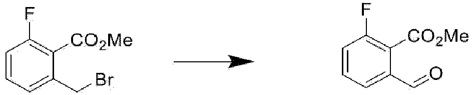

At room temperature, NMO (24.9 g, 212 mmol) was added to methyl 2-(bromomethyl)-6-fluorobenzoate (35 g, 142 mmol) solution in DCM (280 ml). The reactants were stirred at room temperature for 4 hours. The DCM layer was washed with water (200ml), dried with MgSO4, and concentrated under reduced pressure. The residue was purified by column chromatography, and the title compound was obtained as a white solid. (11.52g, yield 45%) MS (ESI, m/z): [M+1]⁺ = [183.1].

### 3-4) Preparation of 2-fluoro-6-formylbenzoic acid

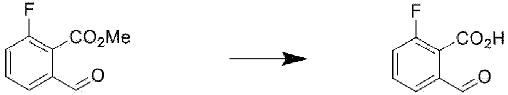

At room temperature, an aqueous solution (41 ml) of lithium (+1) hydroxide (7.57 g, 180 mmol) was added to a solution of methyl 2-fluoro-6-formylbenzoate (11.5 g, 63.2 mmol) in THF (82 ml). After 4 h of agitation, the reactants were decompressively concentrated and the THF was dried. After cooling to 0°C, the reactants were acidified with 1N HCl and the pH was adjusted to 4. The reactants were extracted twice with ethyl acetate (100 ml). The combined ethyl acetate layer was dried with MgSO₄, concentrated under reduced pressure, and white crystals were obtained (10.4g, 97.8%). MS (ESI, m/z): [M+1]⁺ = [168.8].

### 3-5) Preparation of 8-Fluoroptalazine-1(2H)-one

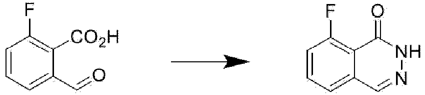

At room temperature, NH₂NH₂ monohydrate (3.72 mg, 61.9 mmol) was added to a solution of 2-fluoro-6-formylbenzoic acid (10.4 g, 61.9 mmol) in methanol (120 ml) and stirred at room temperature for 16 hours. The white sediment was filtered and washed with methanol. The obtained white crystals were slurried with 100ml of ethyl acetate (EA) and filtered to obtain white crystals (3.05g, 30%). MS (ESI, m/z): [M+1]⁺ = [164.8].

### 3-6) Preparation of 3-(8-fluoro-1-oxopthalazine-2(1H)-yl)piperidine-2,6-dione

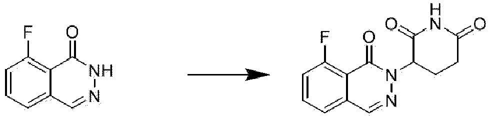

NaH (60%, 53.6mg, 1.34mmol) was added to 8-fluoroptalazine-1(2H)-one (200mg, 1.22mmol) solution in DMF (5ml) at 0°C and stirred for 30 minutes. 3-bromoperidin-2,6-dione (468 mg, 2.44 mmol) was added to the solution and stirred for 6 hours. The reaction was terminated with water and extracted twice with ethyl acetate (20ml). The mixed ethyl acetate was dried with MgSO₄ and concentrated under reduced pressure. The residues were purified by column chromatography, and the title compound was obtained as white crystals (70 mg, 20.8%). MS (ESI, m/z): [M+1]⁺ = [276.1].
[NMR] ¹H NMR (400 MHz, DMSO-d₆) δ 11.06 (s, 1H), 8.48 (s, 1H), 7.99 (ddd, J = 4.6, 7.2, 8.2 Hz, 1H), 7.80 (d, J = 7.2 Hz, 1H), 7.67 (dd, J = 8.2, 11.4 Hz, 1H), 5.77 (dd, J=5.3, 12.0 Hz, 1H), 2.99-2.77 (m, 1H), 2.68-2.51 (m, 2H), 2.23-2.02 (m, 1H)

### Example A-4: Preparation of a compound

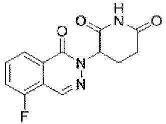

### 4-1) Preparation of methyl 5-fluoro-6-methylbenzoate

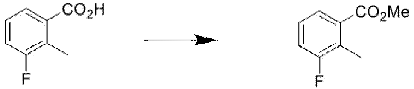

At room temperature, sulfuric acid (2 ml) was added to a solution of 3-fluoro-2-methylbenzoic acid (10 g, 64.9 mmol) in methanol (60 ml). Heated to 80°C and stirred overnight. After cooling to room temperature, the reactants were evaporated and extracted with NaHCO₃ and ethyl acetate (EA). Dried with MgSO₄. The crude product was used in the next reaction without additional purification. (10.1g, yield 92%) MS (ESI, m/z): [M+1]⁺ = [168.3].

### 4-2) Preparation of methyl 2-(bromomethyl)-3-fluorobenzoate

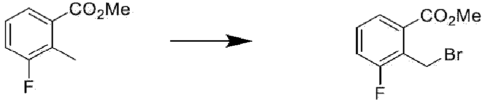

At room temperature, 1-bromomerolidin-2,5-dione (15.9 g, 89.2 mmol) and benzoyl benzene carboforoxoate (0.72 g, 2.97 mmol) were sequentially added to a solution of methyl 3-fluoro-2-methylbenzoate (10 g, 59.5 mmol) in ClCH₂CH₂Cl (250 ml). The reactants were heated and refluxed for 16 hours. The point when the red color disappears is the time when the reaction is complete. After cooling, the reactants were washed with water, dried with MgSO₄, and concentrated under reduced pressure. The crude product was used in the next reaction without additional purification. (10.5g, yield 71%) MS (ESI, m/z): [M+1]⁺ = [245.9].

### 4-3) Preparation of methyl 3-fluoro-2-formylbenzoate

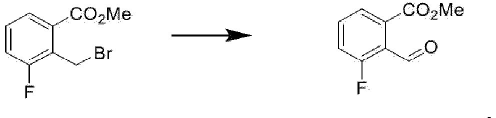

At room temperature, NMO (10.4 g, 89 mmol), 4Å molecular sieve was added to methyl 2-(bromomethyl)-3-fluorobenzoate (10 g, 40.5 mmol) solution in DCM (200 ml). The reactants were stirred at room temperature for 4 hours. The DCM layer was washed with water (200ml), dried with MgSO₄, and concentrated under reduced pressure. The residue was purified by column chromatography to obtain the heading compound. (5.5g, 75%) MS (ESI, m/z): [M+1]⁺ = [183.1].

### 4-4) Preparation of 3-fluoro-2-formylbenzoic acid

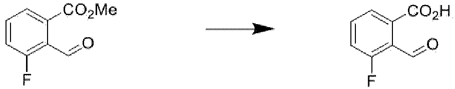

At room temperature, an aqueous solution (41 ml) of lithium (+1) hydroxyside monohydrate (2.88 g, 68.6 mmol) was added to a solution of methyl 3-fluoro-2-formylbenzoate (2.5 g, 13.7 mmol) in THF (68 ml). After 4 hours of agitation, the reactants were decompressively concentrated and THF was removed. After cooling to 0°C, the reactants were acidified with 1N HCl and the pH was adjusted to 4. The reactants were extracted twice with ethyl acetate (100 ml). The combined ethyl acetate layer was dried with MgSO₄, concentrated under reduced pressure to obtain white crystals (2.5g, 108%). MS (ESI, m/z): [M+1]⁺ = [168.8].

### 4-5) Preparation of 5-Fluoro-1,2-dihydrophthalazine-1-one

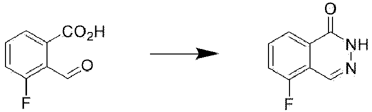

At room temperature, NH₂NH₂ monohydrate (1.22 mg, 16.4 mmol) was added to a 3-fluoro-2-formylbenzoic acid (2.5 g, 14.9 mmol) solution in THF:water=1:1 (70 ml) and stirred for 16 hours. The mixture was acidified to pH 4 and the solid was filtered and washed with hexane. Vacuum drying yielded the title compound (1.3 g, 53%). MS (ESI, m/z): [M+1]⁺ = [165.3]

### 4-6) Preparation of 3-(5-fluoro-1-oxo-1,2-dihydroptalazine-2-yl)piperidine-2,6-dione

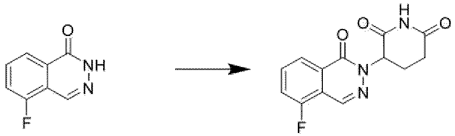

LDA (lithium diisopropylamide, 2.19 mmol) was added to 5-fluoropleptalazine-1-one (300 mg, 1.83 mmol) solution in DMF (10 ml) at 0°C and stirred for 30 minutes. 3-bromoperidin-2,6-dione (526 mg, 2.74 mmol) was added to the solution and stirred at 80°C for 6 hours. Once the reaction was completed, the reactants were cooled to room temperature, poured into water (100ml), pH was adjusted to 3~4 with 6N HCl, then extracted with ethyl acetate, dried with MgSO₄, and concentrated under reduced pressure. The product was recrystallized into hexane, vacuum-dried, and the title compound was obtained. MS (ESI, m/z): [M+1]⁺ = [276.1].
[NMR] ¹H NMR (400 MHz, DMSO-d₆) δ 11.03 (s, 1H), 8.53 (s, 1H), 8.05 (d, J=7.82Hz, 1H), 7.76-7.90 (m, 2H), 5.78 (dd, J=12.23, 5.26Hz, 1H), 2.80-2.93 (m, 1H), 2.47-2.61 (m, 2H), 2.01-2.16 (m, 1H)

### Example A-5: Preparation of a compound

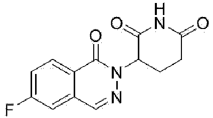

### 5-1) Preparation of methyl 4-fluoro-6-methylbenzoate

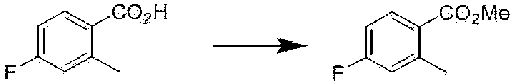

In accordance with the preparation method of Example 4-1, methyl 4-fluoro-2-methylbenzoate was prepared from 4-fluoro-2-methylbenzoate.

### 5-2) Preparation of methyl 2-(bromomethyl)-4-fluorobenzoate

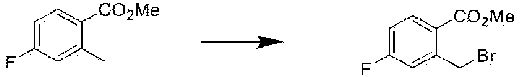

At room temperature, 1-bromophilolidin-2,5-dione (31.8 g, 178 mmol) and benzoyl benzeborocarbon (1.92 g, 5.95 mmol) were sequentially added to a solution of methyl 4-fluoro-2-methylbenzoate (20 g, 119 mmol) in ClCH₂CH₂Cl (100 ml). The reactants were heated and refluxed for 3 hours. The point when the red color disappears is the time when the reaction is complete. After cooling, the reactants were washed with water, dried with MgSO₄, and concentrated under reduced pressure. The product was purified with MPLC (HX/EA EA 0 -> 5%) (22 g, yield 75%). MS (ESI, m/z): [M+1]⁺ = [248.4].

### 5-3) Preparation of methyl 3-fluoro-2-formylbenzoate

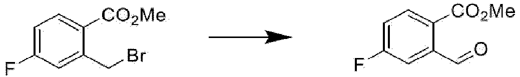

At room temperature, NMO (15.6 mg, 134 mmol) and 4Å molecular sieve were sequentially added to methyl 2-(bromomethyl)-4-fluorobenzoate (22 g, 89 mmol) solution in DCM (100 ml). The reactants were stirred at room temperature for 2 hours. The molecular sieve was filtered and washed with DCM (50ml). The DCM layer was washed with water (200ml), dried with MgSO₄, and concentrated under reduced pressure. The residue was purified by column chromatography, and the title compound was obtained as a white solid. (12g, 73.98%) MS (ESI, m/z): [M+1]⁺ = [183.2].

### 5-4) Preparation of 4-fluoro-2-formylbenzoic acid

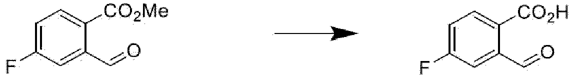

At room temperature, an aqueous solution (50 ml) of lithium (+1) hydroxyside (13.8 g, 329 mmol) was added to a solution of methyl 4-fluoro-6-formylbenzoate (12 g, 65.9 mmol) in THF (50 ml). After 2 h of agitation, the reactants were decompressively concentrated to dry the THF. After cooling to 0°C, the reactants were acidified with 1N HCl and the pH was adjusted to 4. The reactants were extracted twice with ethyl acetate (50ml). The combined ethyl acetate layer was dried with MgSO₄, concentrated under reduced pressure, and white crystals were obtained (10g, 90.3%). MS (ESI, m/z): [M+1]⁺ = [169.2].

### 5-5) Preparation of 6-fluoro-1,2-dihydrophthalazine-1-one

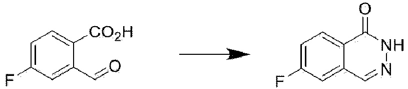

At room temperature, NH₂NH₂ monohydrate (2.02 g, 40.3 mmol) was added to a 4-fluoro-2-formylbenzoic acid (6.78 g, 40.3 mmol) solution in methanol (50 ml). After stirring for 30 minutes, the reactants were heated to 80°C for 2 hours. The reactants were cooled to room temperature and concentrated under reduced pressure. The residue was poured into water (150ml) and extracted twice with ethyl acetate (150ml). The combined ethyl acetate layer was dried with MgSO4, concentrated under reduced pressure to obtain white crystals (5.5g, 83.07%). MS (ESI, m/z): [M+1]⁺ = [165.3].

### 5-6) 3-(6-fluoro-1-oxopthalazine-2(1H)-yl)piperidine-2,6-dione preparation

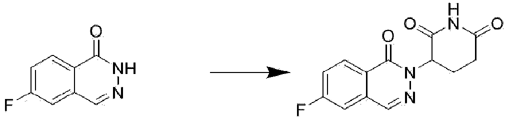

Sodium 2-methylpropane-2-oleate (175 mg, 0.914 mmol) was added to a 6-fluorophthalazine-1-one (100 mg, 0.609 mmol) solution in DMF (2 ml) at 0°C. After stirring for 30 minutes, 3-bromoperidin-2,6-dione (90 mg, 0.471 mmol) was added to the reaction mixture and stirred at room temperature for 6 hours. Water (20ml) was poured into the reaction mixture, and ethyl acetate (20ml) was extracted twice. The mixed ethyl acetate was dried with MgSO₄ and concentrated under reduced pressure. The residues were purified by chromatography, and the title compound was obtained as white crystals (110 mg, 65.5%). MS (ESI, m/z): [M+1]⁺ = [276.6].
[NMR] ¹H NMR (400 MHz, DMSO-d₆) δ 11.11 (s, 1H), 8.50 (s, 1H), 8.38 (dd, J=8.86, 5.44 Hz, 1H), 7.72 - 7.89 (m, 2H), 5.70 - 5.90 (m, 11H), 2.56 - 2.70 (m, 2H), 2.11 - 2.25 (m, 1H)

### Example A-6: Preparation of a compound

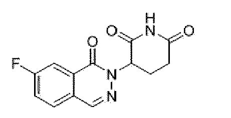

### 6-1) Preparation of methyl 4-fluoro-6-methylbenzoate

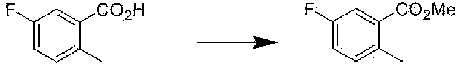

In accordance with the preparation method of Example 4-1, methyl 5-fluoro-2-methylbenzoate was prepared from 5-fluoro-2-methylbenzoate.

### 6-2) Preparation of methyl 2-(bromomethyl)-5-fluorobenzoate

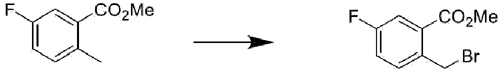

At room temperature, 1-bromophilidine-2,5-dione (31.8 g, 178 mmol) and benzoyl benzene carboroxoate (1.92 g, 5.95 mmol) were sequentially added to a solution of methyl 5-fluoro-2-methylbenzoate (20 g, 119 mmol) in ClCH₂CH₂Cl (100 ml). The reactants were heated and refluxed for 3 hours. The point when the red color disappears is the time when the reaction is complete. After cooling, the reactants were washed with water, dried with MgSO₄, and concentrated under reduced pressure. The product was purified with MPLC (HX/EA EA 0 - > 5%) (29 g, yield 98.8%). MS (ESI, m/z): [M+1]⁺ = [248.4].

### 6-3) Preparation of methyl 5-fluoro-2-formylbenzoate

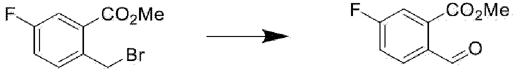

At room temperature, NMO (20.6mg, 176mmol) and 4Å molecular sieve were sequentially added to methyl 2-(bromomethyl)-5-fluorobenzoate (29g, 117mmol) solution in DCM (100ml). The reactants were stirred at room temperature for 2 hours. The molecular sieve was filtered and washed with DCM (50ml). The DCM layer was washed with water (200ml), dried with MgSO₄, and concentrated under reduced pressure. The residue was purified by column chromatography, and the title compound was obtained as a white solid. (15g, yield 70.16%) MS (ESI, m/z): [M+1]⁺ = [183.2].

### 6-4) Preparation of 5-fluoro-2-formylbenzoic acid

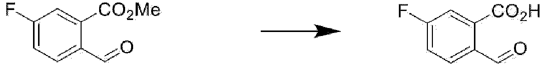

At room temperature, an aqueous solution (50 ml) of lithium (+1) hydroxyside (17.3 g, 412 mmol) was added to a solution of methyl 5-fluoro-2-formylbenzoate (15 g, 82.3 mmol) in THF (50 ml). After 2 hour of agitation, the reactants were decompressively concentrated to dry the THF. After cooling to 0°C, the reactants were acidified with 1N HCl and the pH was adjusted to 4. The reactants were extracted twice with ethyl acetate (50ml). The combined ethyl acetate layer was dried with MgSO₄, concentrated under reduced pressure to obtain white crystals (12g, 86.67%). MS (ESI, m/z): [M+1]⁺ = [169.2].

### 6-5) Preparation of 7-Fluoro-1,2-dihydroptalazine-1-one

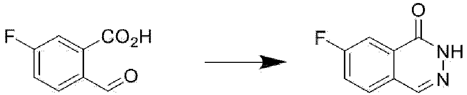

At room temperature, NH₂NH₂ monohydrate (3.74 g, 74.8 mmol) was added to a 5-fluoro-2-formylbenzoic acid (8.16 g, 48.5 mmol) solution in methanol (50 ml). After stirring for 30 minutes, the reactants were heated to 80°C for 2 hours. The reactants were cooled to room temperature and concentrated under reduced pressure. The residue was poured into water (150ml) and extracted twice with ethyl acetate (150ml). The combined ethyl acetate layer was dried with MgSO₄, concentrated under reduced pressure to obtain white crystals (6.5g, 81.59%). MS (ESI, m/z): [M+1]⁺ = [165.3].

### 6-6) Preparation of 3-(7-fluoro-1-oxopthalazine-2(1H)-yl)piperidine-2,6-dione

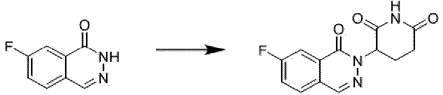

Sodium 2-methylpropane-2-oleate (586 mg, 6.09 mmol) was added to a 7-fluorine-1,2-dihydrophoptalazine-1-one (500 mg, 3.05 mmol) solution in DMF (5 ml) at 0°C. After stirring for 30 minutes, 3-bromoperidin-2,6-dione (1.05 mg, 5.48 mmol) was added to the reaction mixture and stirred at room temperature for 6 hours. The reaction mixture was poured into water (50ml) and extracted twice with ethyl acetate (50ml). The mixed ethyl acetate was dried with MgSO₄ and concentrated under reduced pressure. The residue was purified by column chromatography, and the title compound was obtained as a white crystal (300 mg, 35.78%). MS (ESI, m/z): [M+1]⁺ = [276.6].
[NMR] ¹H NMR (400 MHz, DMSO-d₆) δ 11.09 (s, 1H), 8.53 (s, 1H), 8.13 (dd, J=8.74, 5.20 Hz, 1H), 7.87 - 8.00 (m, 2H), 5.76 - 5.88 (m, 11H), 2.54 - 2.68 (m, 2H), 2.10 - 2.20 (m ,1H)

### Example A-7: Preparation of a compound

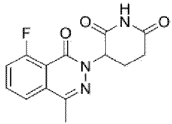

### (prepared according to Equation 2)

### 7-1) Preparation of methyl 2-acetyl-6-fluorobenzoate

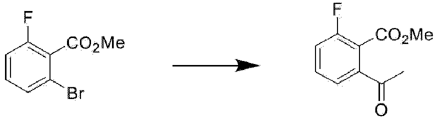

Tetrakis (triphenylphosphine)-palladium (0) (557 mg, 0.48 mmol) was added to a solution of methyl 2-acetyl-6-fluorobenzoate (1.12 g, 4.81 mmol) and tributyl (1-ethoxyvinyl) tin (1.91 g, 5.29 mmol) in toluene (20 ml) and stirred at 100°C for 16 hours. After cooling, 5 ml of 1N-HCl was added and stirred for 1 hour. The organic layer was dried with MgSO₄ and concentrated under reduced pressure. The residue was purified by silica column chromatography, and the heading compound was obtained as yellow oil. (820mg, yield 87%) MS (ESI, m/z): [M+1]⁺ = [196.8].

### 7-2) Preparation of 2-acetyl-6-fluorobenzoic acid

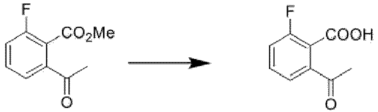

LiOH (494 mg, 20.6 mmol) was added to a solution of methyl 2-acetyl-6-fluorobenzoate (810 mg, 4.13 mmol) in THF (20 ml) and water (10 ml) and stirred for 20 hours at room temperature. The solution was acidified with 1N-HCl so that the pH was about 3. 100ml EA was applied, the organic layer was dried with MgSO₄, and the title compound was obtained by decompression concentration. (810mg yield 108%) MS (ESI, m/z): [M+1]⁺ = [183.1].

### 7-3) Preparation of 8-fluoro-4-methylphthalazine-1(2H)-one

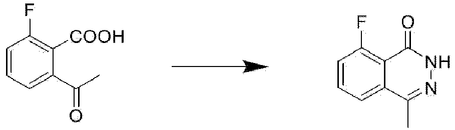

Hydrazine monohydrate (261 mg, 5.20 mmol) was added to a 2-acetyl-6-fluorobenzoic acid (790 mg, 4.34 mmol) solution in methanol (23 ml) and stirred at room temperature for 16 hours. The sediment was filtered, washed with ACN (acetonitrile), and the title compound was obtained as a white solid. (612mg, yield 79.2%) MS (ESI, m/z): [M+1]⁺ = [179.1].

### 7-4) Preparation of 3-(8-fluoro-4-methyl-1-oxopthalazine-2(1H)-yl)piperidine-2,6-dione

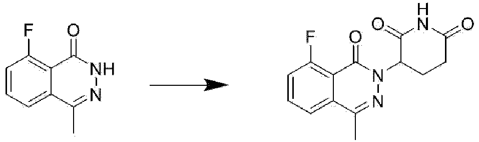

1M-Lithium diisopropylamide (3.6 ml, 3.6 mmol) was added to 8-fluoro-4-methylphthalazine-1(2H)-one (534 mg, 3 mmol) solution in THF (30 ml) at 0°C and stirred for 20 min. 3-brobopiperidine-2,6-dione (863 mg, 4.5 mmol) was added to the solution and stirred at 80°C for 2 hours. The reactants were depressure-concentrated and dried. Water (10 ml) was added and stirred for 1 hour, and acidified with 1N-HCl to adjust the pH to 4. The precipitate was filtered, washed with water, and the title compound was obtained as a white solid (760 mg, yield: 86.5%). MS (ESI, m/z): [M+1]⁺ = [289.8].
[NMR] ¹H NMR (400 MHz, DMSO-d₆) δ 11.05 (s, 1H), 8.04-7.94 (m, 1H), 7.79 (d, J = 7.9 Hz, 1H), 7.69 (dd, J = 7.9, 11.3 Hz, 1H), 5.71 (be dd, J = 4.9, 12.1 Hz, 1H), 3.0 - 2.83 (m, 1H), 2.64 - 2.56 (m, 2H), 2.55 (s, 3H), 2.17 - 2.05 (m, 1H).

### Example A-8: Preparation of a compound

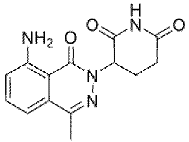

### Preparation of 8-1) 3-(8-((2,4-dimethoxybenzyl)amino)-4-methyl-1-oxopthalazine-2(1H)-yl)piperidine-2,6-dione

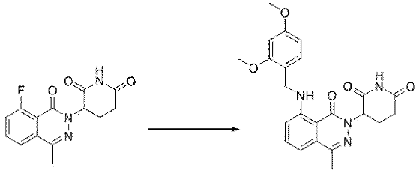

3-(8-fluoro-4-methyl-1-oxotalamine-2(1H)-yl) piperidine-2,6-dione (0.104 mmol), 2,4-dimethoxybenzylamine (0.207 mmol) and DIPEA (N,N-diisopropylethylamine) (0.312 mmol) solutions in NMP (N-methyl-pyrrolidone) (1 mL) were stimulated with microwaves for 2 hours at 120°C. The reaction mixture was purified by reversed-phase chromatography (C18, water (0.1 % FA) / ACN (0.1% FA), gradient), and 33 mg of white solid was obtained (33 mg, yield 72%). MS (ESI, m/z): [M+1]⁺ = [437.0]

### 8-2) Preparation of 3-(8-amino-4-methyl-1-oxopthalazine-2(1H)-yl)piperidine-2,6-dione

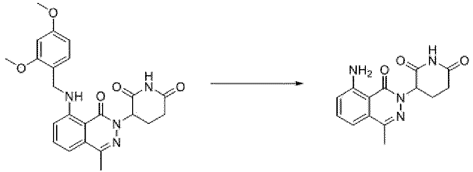

0.3ml of TFA (trifluoroacetic acid) was added to 3-(8-((2,4-dimethoxybenzyl)amino)4-methyl-1-oxopthalazine-2(1H)-yl)piperidine-2,6-dione (14mg, 0.032mmol) in toluene (0.7mL) and stirred at 80°C for 1 hour. The reaction mixture was purified by reversed-phase column chromatography (C18, water (0.1% FA) / ACN (0.1% FA), gradient) yielded 7.5 mg of white solid (7.5 mg, yield 82%). MS (ESI, m/z): [M+1]⁺ = [287.0]

[NMR] 1H NMR (400 MHz, DMSO-d6) δ10.98 (s, 1H), 7.54 (t, J = 8.0 Hz, 1H), 7.37 (brs, 2H), 6.95 (d, J = 8.2 Hz, 1H), 6.88 (d, J = 7.6 Hz, 1H), 5.62 (br dd, J = 5.3, 12.0 Hz, 1H), 3.0-2.82 (m, 1H), 2.66 - 2.52 (m, 2H), 2.39 (s, 3H), 2.11 - 2.02 (m, 1H)

### Example A-9: Preparation of a compound

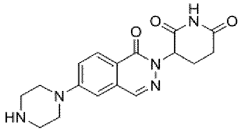

### 9-1) Preparation of tert-butyl 4-(2-(2,6-dioxopiperidine-3-yl)-1-oxo-1,2-dihydroptalazine-6-yl)piperazine-1-carboxylate

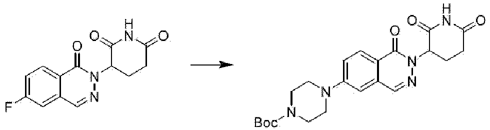

6-Fluoro-1,2-dihydroptalazine-1-one (100 mg, 0.36 mmol) and tert-butylpiperazine-1-carboxylate (81.2 mg, 0.36 mmol) were dissolved in NMP (1 mL), DIPEA (5 equivalents) was added to the reaction mixture, and stirred overnight at 120°C. The reaction of the reaction mixture was terminated with water, extracted with EA, and washed with NH4Cl saturated aqueous solution and Brine solution. The organic layer was dried with MgSO₄. The reaction mixture was loaded into silica, separated by MPLC (HX/EA 30% -> 50% for 10 min), and the oil product was obtained (110 mg, yield 66%).

### 9-2) Preparation of 3-(1-oxo-6-(piperazine-1-yl)phthalazine-2(1H)-yl)piperidine-2,6-dione

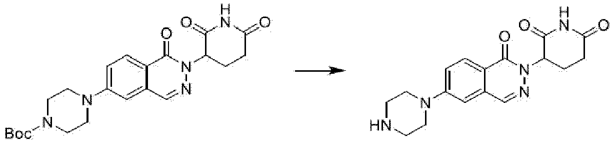

Tert-butyl 4-[2-(2,6-dioxopiperidine-3-yl)-1-oxo-1,2-dihydroptalazine-6-yl]piperazine-1-carboxylate was dissolved in a 20% TFA solution in DCM (1 ml) and reacted for 2 hours at room temperature. The reaction was terminated with an aqueous solution saturated with NaHCO3 and extracted with EA. The organic layer was dried with MgSO₄ and evaporated. The reaction mixture was purified with MPLC (MC/ME Me 0 -> 10%). White solid product (40 mg, yield 86%). MS (ESI, m/f): [M+1]⁺ = [342.2].

[NMR] 1H NMR (400 MHz, DMSO-d6) δ 8.24 (s, 1 H) 8.02 (d, J=9.05 Hz, 1 H) 7.47 (dd, J=8.93, 2.57 Hz, 1 H) 7.31 (s, 0.5 H) 7.23 (d, J=2.45 Hz, 1 H) 7.13 (s, 0.5 H) 5.34 (dd, J=8.93, 4.52 Hz, 1 H) 3.28 - 3.44 (m, 6 H) 2.85 - 2.95 (m, 4 H) 2.30 - 2.40 (m, 1 H) 2.16 - 2.30 (m, 2 H)

### Example A-10: Preparation of a compound

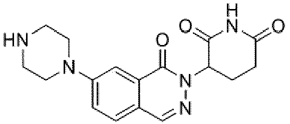

### 10-1) Preparation of tert-butyl 4-(3-(2,6-dioxopiperidine-3-yl)-4-oxo-3,4-dihydroptalazine-6-yl)piperazine-1-carboxylate

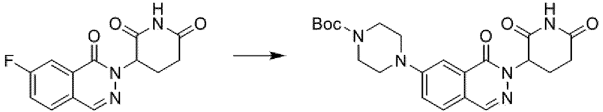

3-(7-fluoro-1-oxo-1,2-dihydroptalazine-2-yl)piperidine-2,6-dione (100 mg, 0.36 mmol) and tert-butylpiperazine-1-carboxylate (81.2 mg, 0.44 mmol) were dissolved in NMP (1 mL), DIPEA (5 equivalents) was added to the reaction mixture, and stirred overnight at 120°C. The reaction of the reaction mixture was terminated with water, extracted with EA, and washed with NH4Cl saturated aqueous solution and Brine solution. The organic layer was dried with MgSO₄, the reaction mixture was loaded into silica, separated by MPLC (HX/EA 30% -> 50% for 10 min), and the product was obtained as oil (yield: 110 mg, 66%).

### 10-2) Preparation of 3-(1-oxo-7-(piperazine-1-yl)phthalazine-2(1H)-yl)piperidine-2,6-dione

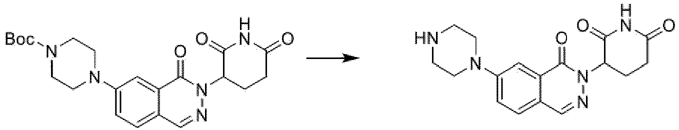

Tert-butyl 4-[3-(2,6-dioxopiperidine-3-yl)-4-oxo-3,4-dihydroptalazine-6-yl]piperazine-1-carboxylate (60 mg, 0.14 mmol) was dissolved in 20% TFA in DCM (1 ml) and reacted for 2 hours at room temperature. The reaction was terminated with an aqueous solution saturated with NaHCO₃ and extracted with EA. The organic layer was dried with MgSO₄ and evaporated. The reaction mixture was purified with MPLC (MC/ME Me 0 -> 10%) and a white solid was obtained as the product (yield: 40 mg/ 86%). MS (ESI, m/f): [M+1]⁺ = [342.2].

[NMR] 1H NMR (400 MHz, DMSO-d6) δ 8.21 - 8.29 (m, 1 H) 7.76 (d, J=8.80 Hz, 1 H) 7.58 (dd, J=8.93, 2.57 Hz, 1 H) 7.47 (d, J=2.45 Hz, 1 H) 7.28 (s, 0.5 H) 7.13 (s, 0.5 H) 5.34 - 5.44 (m, 1 H) 3.26 - 3.33 (m, 4 H) 2.81 - 2.91 (m, 3 H) 2.62 - 2.69 (m, 1 H) 2.55 - 2.62 (m, 1 H) 2.31 - 2.44 (m, 1 H) 2.15 - 2.31 (m, 2 H)

### Example A-11: preparation of 3-(8-methoxy-1-oxopthalazine-2(1H)-yl)piperidine-2,6-dione

Preparation of 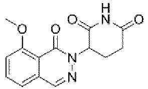 compounds

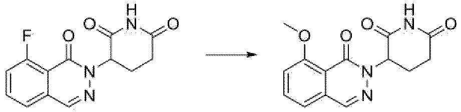

3-(6-fluoro-1-oxo-1,2-dihydroptalazine-2-yl)piperidine-2,6-dione (100 mg, 0.36 mmol) was dissolved in DMSO (1 ml), and NaOMe (19.6 mg, 0.36 mmol) was added to the reaction mixture. The reaction mixture was reacted at room temperature for 1 hour. The organic solvent was removed, and extracted with EA and water. The mixture was purified with MPLC, and the product was obtained as a white solid (yield: 80 mg / 76%). MS (ESI, m/f): [M+1]⁺ = [288.2].

[NMR] 1H NMR (400 MHz, DMSO-d6) δ 11.04 (br. s., 1 H) 8.40 (s, 1 H) 8.14 - 8.25 (m, 1 H) 7.42 - 7.49 (m, 2 H) 5.80 (dd, J=12.23, 5.38 Hz, 1 H) 3.94 (s, 3 H) 2.86 - 2.99 (m, 1 H) 2.52 - 2.67 (m, 2 H) 2.07 - 2.17 (m, 1 H)

### Example A-12: Preparation of 3-(7-methoxy-1-oxopthalazine-2(1H)-yl)piperidine-2,6-dione

### Preparation of compound

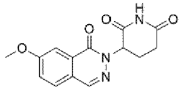

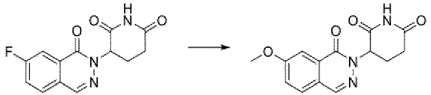

3-(7-fluoro-1-oxopthalazine-2(1H)-yl)piperidine-2,6-dione (100 mg, 0.36 mmol) was dissolved in DMSO (1 ml), and NaOMe (19.6 mg, 0.36 mmol) was added to the reaction mixture. The half-heung mixture was reacted at room temperature for 1 hour. The organic solvent was removed, and extracted with EA and water. The mixture was purified with MPLC, and the product was obtained as a white solid. (Yield: 78mg / 75%) MS (ESI, m/f): [M+1]⁺ = [288.2].

[NMR] 1H NMR (400 MHz, DMSO-d6) δ 11.01 - 11.11 (m, 1 H) 8.38 - 8.45 (m, 1 H) 7.90 - 7.96 (m, 1 H) 7.65 (d, J=2.69 Hz, 1 H) 7.56 (dd, J=8.68, 2.57 Hz, 1 H) 5.77 - 5.85 (m, 1 H) 3.93 - 3.98 (m, 3 H) 2.87 - 3.00 (m, 1 H) 2.53 - 2.70 (m, 2 H) 2.06 - 2.18 (m, 1 H)

### Example A-13: Preparation of 3-(6-methoxy-1-oxopthalazine-2(1H)-yl)piperidine-2,6-dione

### Preparation of compound

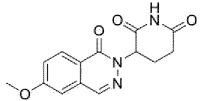

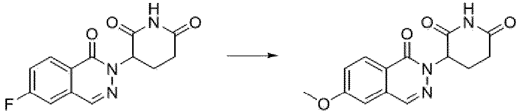

3-(6-fluoro-1-oxopthalazine-2(1H)-yl)piperidine-2,6-dione (100 mg, 0.36 mmol) was dissolved in DMSO (1 ml), and NaOMe (19.6 mg, 0.36 mmol) was added to the reaction mixture. The half-heung mixture was reacted at room temperature for 1 hour. The organic solvent was removed, and extracted with EA and water. The mixture was purified with MPLC, and the product was obtained as a white solid. (Yield: 80mg / 76%) MS (ESI, m/f): [M+1]⁺ = [288.2].

[NMR] 1H NMR (400 MHz, DMSO-d6) δ 11.01 (s, 1 H) 8.32 (s, 1 H) 7.88 (t, J=8.01 Hz, 1 H) 7.40 (d, J=8.31 Hz, 1 H) 7.44 (d, J=7.70 Hz, 1 H) 5.64 (dd, J=11.49, 4.52 Hz, 1 H) 3.90 (s, 3 H) 2.83 - 2.96 (m, 1 H) 2.52 - 2.66 (m, 2 H) 2.03 - 2.13 (m, 1 H)

### Example A-14: Preparation of 3-(5-methoxy-1-oxopthalazine-2(1H)-yl)piperidine-2,6-dione

### Preparation of compound

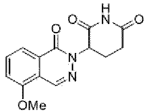

### 14-1) Preparation of Methyl 2-(Bromomethyl)-3-Methoxybenzoate

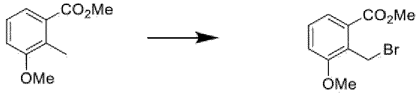

1-bromophilidine-2,5-dione (11.5 g, 64.94 mmol) and benzoyl benzene carboperoxate (786 mg, 3.24 mmol) were sequentially added to a solution of methyl 2-methyl-3-methoxybenzoate (11.7 g, 64.9 mmol) in ClCH₂CH₂Cl (250 ml) at room temperature. The reaction mixture was heated and refluxed for 3 hours. The point at which the red color disappears is the time when the reaction is completed. After cooling, the reactants were washed with water, dried with MgSO₄, and concentrated under reduced pressure. The crude product was used in the next reaction without additional purification. (16.5 g, 98.2%) MS (ESI, m/z): [M+1]⁺ = [259.4].

### 14-2) Preparation of Methyl 2-Formyl-3-Methoxybenzoate

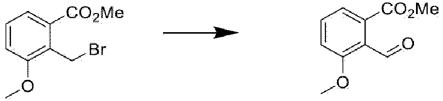

NMO (12.6 g, 108.1 mmol) and 4Å molecular sieve (50 g) were sequentially added to methyl 2-(bromomethyl)-3-methoxybenzoate (14.1 g, 54.1 mmol) solution in DCM (300 mL) at room temperature. The reactants were stirred at room temperature for 2 hours. The molecular sieve was filtered and washed with DCM (100 mL). The DCM layer was washed with water (250 mL), dried with MgSO₄, and concentrated under reduced pressure. The residue was purified by column chromatography, and the title compound was obtained as a white solid. (8.3 g, 80.01%) MS (ESI, m/f): [M+1]⁺ = [195.0].

### 14-3) Preparation of 2-formyl-3-methoxybenzoic acid

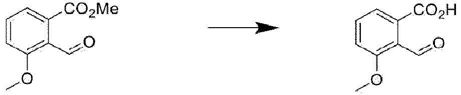

Lithium(1+) hydroxyside (2.4 g, 100.5 mmol) in H2O (100 mL) was added to a solution of methyl 2-formyl-3-methoxybenzoate (6.5 g, 33.6 mmol) in THF (100 mL) at room temperature. After stirring for 2 hours, the reactants were decompressively concentrated to evaporate the THF. After cooling to 0°C, the reactants were acidified with 1N HCl and the pH was adjusted to 4. The reactants were extracted twice with ethyl acetate (250 mL). The combined ethyl acetate layer was dried with MgSO₄, concentrated under reduced pressure, and white crystals were obtained. (11.2g, 82.6%) MS (ESI, m/f): [M+1]⁺ = [199.2].

### 14-4) Preparation of 5-methoxyphthalazine-1(2H)-one

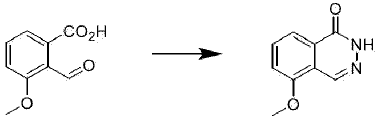

NH₂NH₂ monohydrate (10.3 g, 342 mmol) was added to a solution of 2-formyl-3-methoxybenzoic acid (8.2 g, 45.5 mmol) in MeOH (20 mL) at room temperature. After stirring for 2 hours, the reactants were concentrated under reduced pressure, poured into water (50 ml), and extracted twice with ethyl acetate (150 ml). The combined ethyl acetate layer was dried with MgSO4 and concentrated under reduced pressure to obtain white crystals. (9.2g, 76.35%) MS (ESI, m/f): [M+1]⁺ = [176.9].

### 14-5) Preparation of 3-(5-methoxy-1-oxopthalazine-2(1H)-yl)piperidine-2,6-dione

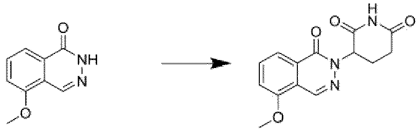

1.0 M LDA (37.4 mL) was dripped at 0°C in some suspension of 5-methoxyphthalazine-1(2H)-one (5.1 g, 28.9 mmol) in THF (250 mL). After stirring for 30 min, 3-bromoperidin-2,6-dione was partially added to the reactants. The reactants were heated to 80°C and stirred for 2 hours. Once the reaction was finished, the reactant was cooled to room temperature, poured into water (100 ml), pH was adjusted to 3~4 with 6N HCl, extracted with ethyl acetate, dried with MgSO₄, and concentrated under reduced pressure. The residue was purified with MPLC and the title compound was obtained as a white crystal. (7.2g, yield 86.5%) MS (ESI, m/f): [M+1]⁺ = [288.3].

[NMR] 1H NMR (400 MHz, DMSO-d6) δ11.06 (s, 1H), 8.51 (s, 1H), 7.87-7.77 (m, 2H), 7.54-7.51 (m, 1H), 5.85 (m, 1H), 4.0 (s, 3H), 2.98 - 2.90 (m, 1H), 2.65 - 2.55 (m, 2H), 2.14 - 2.09 (m, 1H).

### Example A-15: Preparation of 3-(6-bromo-1-oxopthalazine-2(1H)-yl)piperidine-2,6-dione

### Preparation of compound

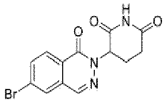

### 15-1) Preparation of 5-Bromo-3-hydroxyisobenzofuran-1(3H)-one

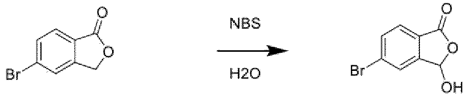

AIBN (401 mg, 2.44 mmol) was added to a mixture of 5-bromophthalide (5.2 g, 24.4 mmol) and N-bromosuccinicimide (5.6 g, 31.7 mmol) in 200 ml of ClCH₂CH₂Cl and reflucated for 8 hours. Following the response, TLC was carried out. The succinimide was filtered and the cake was washed with ClCH₂CH₂Cl (50 mL). The solvent was removed with vacuum, leaving 5.2g of residue, to which 50 ml of water was added. The mixture was stirred for 4 hours to reflux, the mixture was cooled, the product was filtered, neutralized washed with water, and dried, and pale yellowish-white crystals were obtained. (4.85g, yield 86.7%) MS (ESI, m/z): [M+1]⁺ = [229.6] and [230.5]

### 15-2) Preparation of 6-bromophthalazine-1(2H)-one

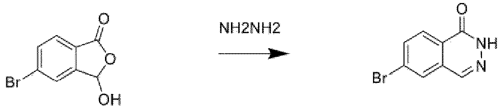

NH₂NH₂ H2O (315 mg, 9.82 mmol) was added to 5-bromo-3-hydroxy-1,3-dihydro-2-benzofuran-1-one (1.5 g, 6.55 mmol) solution in MeOH (50 mL) at room temperature and stirred for 30 minutes. The reaction was refluctuated for 5 hours. The reactants were cooled to room temperature and concentrated under reduced pressure. The resulting solid was tritured with ethyl acetate to obtain white crystals. (1.31g, 5.82 mmol) MS (ESI, m/f): [M+1]⁺ = [226.3].

### 15-3) Preparation of 3-(6-bromo-1-oxopthalazine-2(1H)-yl)piperidine-2,6-dione

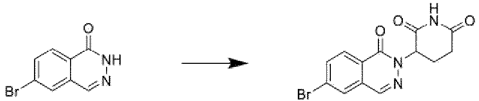

1.0 M LDA (7.33 mL) was dripped at 0°C in a 6-bromo-1,2-dihydroptalazine-1-one (1.31 g, 5.82 mmol) suspension of THF (150 mL). After stirring for 30 min, 3-bromoperidin-2,6-dione was partially added to the reactants. The reactants were heated to 80°C and stirred for 2 hours. Once the reaction was over, the reactants were cooled to room temperature, poured into water (100 ml), pH was adjusted to 3-4 with 6N HCl, extracted with ethyl acetate, dried with MgSO₄, and concentrated under reduced pressure. The resulting solids were filtered, washed with ethyl acetate, and white crystals were obtained. (1.73g, 5.15 mmol) MS (ESI, m/f): [M+1]⁺ = [337.2].

[NMR] 1H NMR (400 MHz, DMSO-d6) δ11.08 (s, 1H), 8.45 (s, 1H), 8.28 (s, 1H), 8.18-8.16 (m, 1H), 8.06-8.04 (m, 1H), 5.84-5.80 (m, 1H), 2.93 - 2.90 (m, 1H), 2.65 - 2.54 (m, 2H), 2.15 - 2.12 (m, 1H).

### Example A-16: Preparation of 3-(1-oxo-8-(piperazine-1-yl)phthalazine-2(1H)-yl)piperidine-2,6-dione dihydrochloride

### Preparation of compound

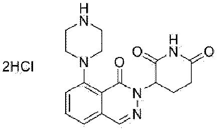

### 16-1) Preparation of 3-(1-oxo-8-(piperazine-1-yl)phthalazine-2(1H)-yl)piperidine-2,6-dione dihydrochloride

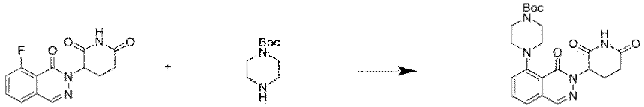

tert-butyl piperazine-1-carboxylate (244 mg, 1.13 mmol) and ethylbis (propane-2-yl)amine (423 mg, 3.24 mmol) were sequentially added to 3-(8-fluoro-1-oxo-1,2-dihydrophoptalazine-2-yl)piperidine-2,6-dione (0.3 g, 1.09 mmol) solution in DMA (4 mL). The mixture was stirred at 120°C for 5 hours. The reaction mixture was diluted with water and extracted with ethyl acetate. The organic layer was washed with brine solution, dried with Na2SO4, and concentrated. The residue was purified by column chromatography to obtain a pale yellowish-white oil. (415 mg, 86.2%) MS (ESI, m/f): [M+1]⁺ = [442.4]

### 16-2) Preparation of 3-(1-oxo-8-(piperazine-1-yl)phthalazine-2(1H)-yl)piperidine-2,6-dione dihydrochloride

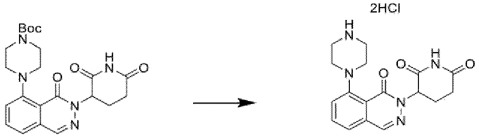

3-bromoperidin-2,6-dione (90 mg, 0.471 mmol) and K₂CO₃ (129 mg, 0.942 mmol) were sequentially added to 8-nitro-1,2-dihydrophthalazine-1-one (60 mg, 0.314 mmol) solution in DMF (1 mL). The reaction was heated to 85°C for 5 hours. After cooling, the reaction mixture was poured into water (5 mL) and extracted twice with ethyl acetate (5 mL). The mixed ethyl acetate was extracted with MgSO₄ and concentrated under reduced pressure. The residue was purified by column chromatography, and the title compound was obtained as white crystals. (68.0 mg, yield 71.7%) MS (ESI, m/f): [M+1]⁺ = [342.6].

[NMR] 1H NMR (400 MHz, DMSO-d6) δ11.02 (s, 1H), 8.35 (s, 1H), 7.87-7.83 (m, 1H), 7.53-7.51 (m, 1H), 7.39-7.41 (m, 1H), 5.55 (m, 1H), 3.31-3.27 (br, 8H), 2.88 - 2.80 (m, 1H), 2.64 - 2.57 (m, 2H), 2.50 (br, 1H), 2.13 - 2.08(m, 1H).

### Example B: Preparation of a cMET target ligand compound based on ABN derivatives

For the preparation of ABN base-cMET target ligand compounds, Intermediates with the following formula
(S)-4-(5-bromofimidine-2-yl)-2 ((6-(1-methyl-1H-pyrazol-4-yl)-1H-[1,2,3]triazolo[4,5-b]pyrazine-1-yl)methyl)morpholine (chemical formula name is written in accordance with the U.S. patent) with reference to the U.S. registered patent (US 9,403,831 B2), which is incorporated by reference herein, and
(2S)-4-(5-bromoprimidine-2-yl)-2((5-(1-methyl-1H-pyrazole-4-yl)-3a,4a-dihydro-1 H-[1,2,3]triazolo[4,5-b]pyrazine-1-yl)methyl)morpholine
were synthesized, and this intermediate was used to prepare compounds of Examples B-1 to B-13 in the following embodiments.

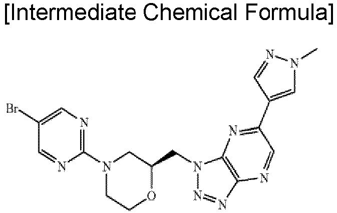

### Example B-1: Preparation of tert-butyl(S)-3-((4-(2-(2-((5-(1-methyl-1H-pyrazole-4-yl)-1H-[1,2,3]triazolo[4,5-b]pyrazine-1-yl)methyl)morpholino)pyrimidine-5-il)ppiperazine-1-il)methyl)azetidine-1-carboxylate

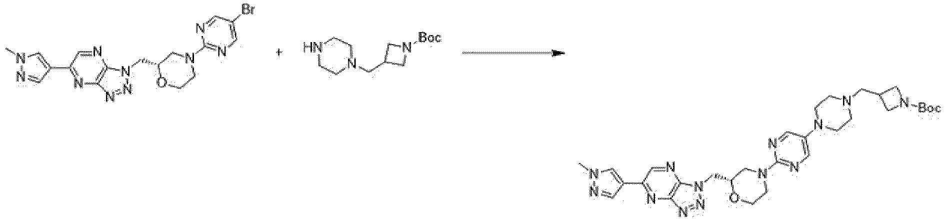

Pd₂(dba)₃ (60.1 mg, 0.07 mmol) were added to (2S)-4-(5-bromoprimidine-2-yl)-2 ((5-(1-(1-methyl-1H-pyrazol-4-yl)-3a,4a-dihydro-1H-[1,2,3]triazolo[4,5-b]pyrazine-1-yl)methyl)morpholine (100 mg, 0.22 mmol) and t-butyl 3-[(piperazine-1-yl)methyl]azetidine-1-carboxylate (134 mg, 0.53 mmol) in toluene (3.0 ml). The reactants were stirred at 90°C for 3 hours. After the end of the reaction, the reactants were poured into water, extracted with DCM (twice), dried with MgSO₄, and concentrated under reduced pressure. The residues were purified by DCM/MeOH (0~5%) column chromatography to obtain the title compound (70 mg). MS (ESI, m/z): [M+H]⁺ = 632.6.
¹H NMR (500 MHz, DMSO-*d*₆) δ ppm 9.21 (s, 1 H) 8.65 (s, 1 H) 8.31 (s, 1 H) 8.22 (s, 2 H) 6.54 (s, 1 H) 4.84 - 4.99 (m, 3 H) 4.11 - 4.26 (m, 1 H) 3.95 (s, 4 H) 3.90 (br. s., 5 H) 3.37 - 3.54 (m, 3 H) 2.88 - 3.03 (m, 3 H) 2.74 (br. s., 1 H) 1.30 - 1.41 (m, 15 H)

### Example B-2: Preparation of (S)-4-(5-(4-(azetidine-3-yl)piperazine-1-yl)pyrimidine-2-yl)-2-((5-(1-methyl-1H-pyrazole-4-yl)-1H-[1,2,3]triazolo[4,5-b]pyrazine-1-yl)methyl)morpholine

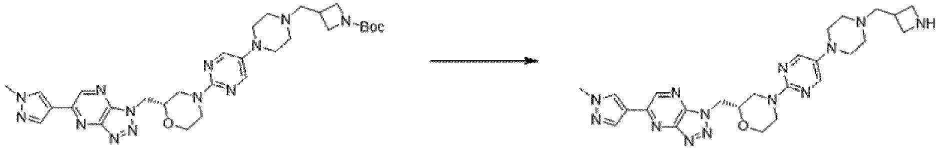

30% TFA was added to a solution of t-butyl 3-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-il}piperazine-1-yl)methyl]azetidine-1-carboxylate (70 mg, 0.11 mmol) solution. The reactants were stirred at room temperature for 2 hours. After the reaction was completed, the reaction mixture was concentrated under reduced pressure. The residue was purified by DCM/MeOH (0~5%) amine column chromatography to obtain the title compound (50mg). MS (ESI, m/z): [M+H]⁺ = 532.7.

### Example B-3: Preparation of (S)-2-((5-(1-methyl-1H-pyrazole-4-yl)-1H-[1,2,3]triazolo[4,5-b]pyrazine-1-yl)methyl)-4-(5-(4-((1-methylazetidine-3-yl)methyl)piperazine-1-il)pyrimidine-2-yl)morpholine

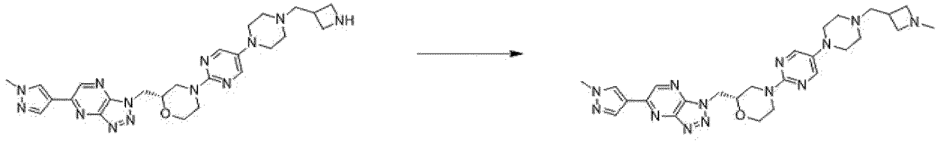

(2S)-4-(5-{4-[(Azetidine-3-yl)methyl]piperazine-1-il}pyrimidine-2-yl)-2-{[5-(1-methyl-1H-pyrazole-4-yl)-1H-[1,2,3]triazolo[4,5-b]pyrazine-1-yl]methyl}morpholine (14 mg, 0.026 mmol) solution was dissolved in methanol (1% acetic acid) (1 ml), formaldehyde (3.95 mg, 0.13 mmol) in a 35% aqueous solution and NaBH₄ (2.5 mg, 0.07 mmol equivalent) were added. The reaction mixture was stirred at room temperature for 1 hour. After the reaction was completed, the reaction mixture was concentrated under reduced pressure. The residue was purified by DCM/MeOH (0~5%) amine column chromatography to obtain the title compound (50mg). MS (ESI, m/z): [M+H]⁺ = 546.7.
¹H NMR (500 MHz, DMSO-*d*₆) δ ppm 9.21 (s, 1 H) 8.65 (s, 1 H) 8.31 (s, 1 H) 8.19 - 8.29 (m, 1 H) 8.16 (s, 1 H) 6.56 (br. s., 1 H) 4.84 - 4.96 (m, 2 H) 4.78 (br. s., 1 H) 4.50 (d, J=13.12 Hz, 1 H) 4.32 (br. s., 1 H) 4.18 (br. s., 2 H) 3.83 - 3.99 (m, 3 H) 3.81 (br. s., 1 H) 3.53 (d, J= 11.44 Hz, 2 H) 3.45 (d, J=8.24 Hz, 3 H) 3.11 (d, J=5.34 Hz, 2 H) 3.05 (d, J=6.41 Hz, 2 H) 2.91 - 2.98 (m, 2 H) 2.30 - 2.38 (m, 2 H) 2.27 (br. s., 2 H) 1.90 - 2.01 (m, 3 H)

### Example B-4: Preparation of benzyl (S)-4-((1-(2-(2-((5-(1-(1-methyl-1H-pyrazol-4-yl)-1H-[1,2,3]triazolo[4,5-b]pyrazine-1-yl)methyl)morpholino)pyrimidine-5-il)azetidine-3-yl)methyl)piperazine-1-carboxylate

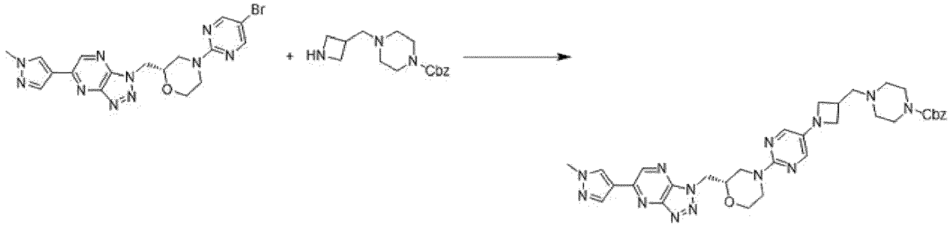

Pd2 (dba)3 (60.1 mg, 0.07 mmol), Xantphos (38.1 mg, 0.07 mmol), and NaOt-Bu (214 mg, 66 mmol) were added to a solution of (2S)-4-(5-(5-methyl-1H-pyrazol-4-yl)-1H-{[1,2,3]triazolo[4,5-b]pyrazine-1-yl]methyl}morpholine (100 mg, 0.22 mmol) and benzyl 4-[(azetidine-3-yl)methyl]piperazine-1-carboxylate (190 mg, 0.66 mmol). The reactants were stirred at 90°C for 3 hours. After the end of the reaction, the reactants were poured into water, extracted with DCM (twice), dried with MgSO₄, and concentrated under reduced pressure. The residue was purified by DCM/MeOH (0~5%) column chromatography to obtain the title compound (65mg). MS (ESI, m/z): [M+H]⁺ = 666.6.
¹H NMR (500 MHz, DMSO-*d*₆) δ ppm 9.13 (s, 1 H) 8.57 (s, 1 H) 8.24 (s, 1 H) 7.76 (s, 1 H) 7.22 - 7.37 (m, 6 H) 4.98 - 5.06 (m, 2 H) 4.75 - 4.88 (m, 2 H) 4.38 (d, J=12.21 Hz, 1 H) 4.04 (d, J=13.12 Hz, 1 H) 3.88 (s, 3 H) 3.76 - 3.86 (m, 2 H) 3.31 - 3.46 (m, 16 H) 2.77 - 2.90 (m, 1 H) 1.81 - 1.95 (m, 1 H)

### Example B-5: Preparation of (S)-2-((5-(1-methyl-1H-pyrazole-4-yl)-1H-[1,2,3]triazolo[4,5-b]pyrazine-1-yl)methyl)-4-(5-(3-(piperazine-1-ylmethyl)azetidine-1-il)pyrimidine-2-yl)morpholine

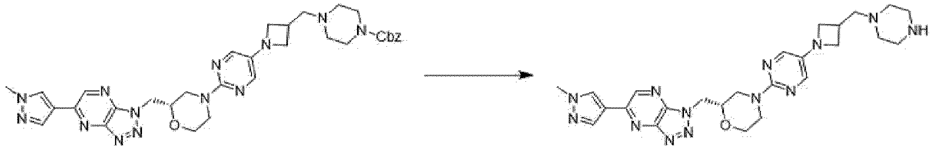

Pd/C (10 wt%) was added to benzyl 4-[(1-{2-[(2S)-2-{[5-(1-methyl-1H-pyrazole-4-yl)-1H-[1,2,3]triazolo[4,5-b]pyrazine-1-yl]methyl}morpholine-4-yl]pyrimidin-5-il}azetidine-3-yl)methyl]piperazine-1-carboxylate (50 mg, 0.07 mmol) solution. The reactants were stirred under H2 gas at room temperature for 12 hours. After the reaction was completed, the reactants were filtered and concentrated under depressure. The residue was purified by DCM/MeOH (0-10%) amine column chromatography to obtain the title compound (15mg). MS (ESI, m/z): [M+H]⁺ = 532.6.
¹H NMR (500 MHz, DMSO-*d*₆) δ ppm 9.14 (s, 1 H) 8.57 (s, 1 H) 8.24 (s, 1 H) 7.76 (s, 2 H) 4.76 - 4.88 (m, 1 H) 4.38 (d, J=12.05 Hz, 1 H) 4.04 (d, J=13.28 Hz, 1 H) 3.88 (s, 3 H) 3.74 - 3.85 (m, 2 H) 3.18 - 3.44 (m, 11) 2.97 (br. s., 1 H) 2.76 - 2.90 (m, 2 H) 2.52 - 2.60 (m, 1 H) 1.81 - 1.93 (m, 4 H)

### Example B-6: Preparation of (S)-4-(5-(3-((benzyloxy)methyl)azetidine-1-il)pyrimidine-2-yl)-2-((5-(1-methyl-1H-pyrazole-4-yl)-1H-[1,2,3]triazolo[4,5-b]pyrazine-1-yl)methyl)morpholine

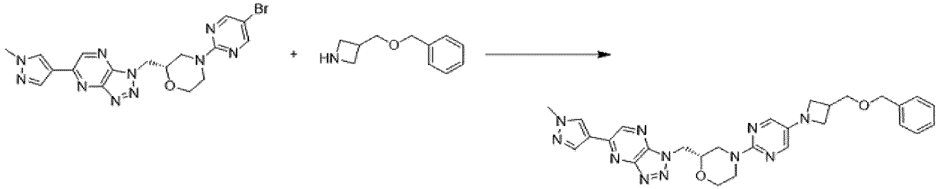

Pd2(dba)3 (0.84 g, 0.92 mmol), Xantphos (0.53 g, 0.92 mmol), and NaOt-Bu (0.89 g, 9.18 mmol) were added to a solution of (2S)-4-(5-(5-methyl-1H-pyrazol-4-yl)-1H-[1,2,3]triazolo[4,5-b]pyrazine-1-yl]methyl}morpholine (1.4 g, 3.1 mmol) and 3-[(benzyloxy)methyl]azetidine (0.98 g, 5.51 mmol) in toluene (40 ml). The reactants were stirred at 90°C for 3 hours. After the end of the reaction, the reactants were poured into water, extracted with DCM (twice), dried with MgSO₄, and concentrated under reduced pressure. The residues were purified by DCM/MeOH (0~5%) column chromatography to obtain the title compound (0.8g). MS (ESI, m/z): [M+H]⁺ = 554.6.
¹H NMR (500 MHz, DMSO-*d*₆) δ ppm 9.21 (s, 1 H) 8.65 (s, 1 H) 8.31 (s, 1 H) 7.83 (s, 2 H) 7.23 - 7.40 (m, 5 H) 4.82 - 4.95 (m, 2 H) 4.39 - 4.54 (m, 3 H) 4.11 (d, J=13.12 Hz, 2 H) 3.95 (s, 3 H) 3.82 - 3.91 (m, 2 H) 3.63 (d, J=6.71 Hz, 2 H) 3.54 (t, J=6.33 Hz, 2 H) 3.42 - 3.51 (m, 1 H) 3.32 - 3.39 (m, 2 H) 2.83 - 3.02 (m, 2 H)

### Example B-7: Preparation of (S)-(1-(2-(2-((5-(1-methyl-1H-pyrazole-4-yl)-1H-[1,2,3]triazolo[4,5-b]pyrazine-1-yl)methyl)morpholino)pyrimidine-5-il)azetidine-3-yl)methanol

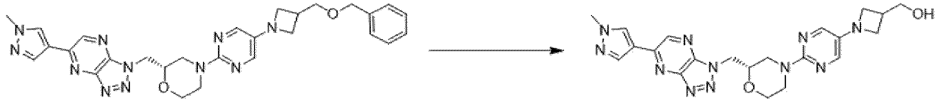

MsOH (0.5 g, 0.91 mmol) was added to the (2S)-4-(5-{3-[(benzyloxy)methyl]azetidin-1-yl}pyrimidine-2-yl)-2-{[5-(1-methyl-1H-pyrazol-4-yl)-1H-[1,2,3]triazolo[4,5-b]pyrazine-1-yl]methyl}morpholine (0.5 g, 0.91 mmol) in DCM (100 ml). The reactants were stirred at 0°C~room temperature for 2 hours. After the reaction was completed, the reaction mixture was concentrated under reduced pressure. The residues were purified by MC/Me (0-10%) column chromatography to obtain a heading compound (0.3 g). MS (ESI, m/z): [M+H]⁺ = 464.5.
¹H NMR (500 MHz, DMSO-*d*₆) δ ppm 9.21 (s, 1 H) 8.64 (s, 1 H) 8.31 (s, 1 H) 7.82 (s, 2 H) 4.81 - 4.95 (m, 2 H) 4.74 (t, J=5.26 Hz, 1 H) 4.45 (d, J=12.51 Hz, 1 H) 4.15 - 4.25 (m, 1 H) 4.11 (d, J=13.28 Hz, 1 H) 3.91 - 3.98 (m, 3 H) 3.88 (d, J=11.60 Hz, 1 H) 3.80 (t, J=7.48 Hz, 2 H) 3.42 - 3.60 (m, 6 H) 2.83 - 2.98 (m, 2 H) 2.79 (dt, J=13.01, 6.69 Hz, 1 H)

### Example B-8: Preparation of (S)-(1-(2-(2-((5-(1-methyl-1H-pyrazole-4-yl)-1H-[1,2,3]triazolo[4,5-b]pyrazine-1-yl)methyl)morpholino)pyrimidine-5-il)azetidine-3-yl)methyl 4-methylbenzenesulfonate

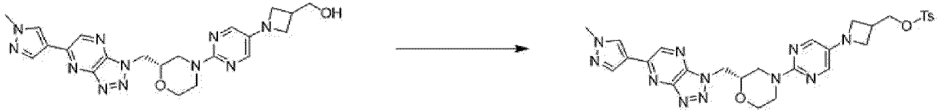

TEA (96.2 ul, 0.69 mmol) was added to a solution of (1-{2-[(2S)-2-{[5-(1-methyl-1H-pyrazol-4-yl)-1H-[1,2,3]triazolo[4,5-b]pyrazine-1-yl]methyl}morpholine-4-yl]pyrimidin-5-yl}azetidine-3-yl)methanol (160 mg, 0.36 mmol) in DCM (5 ml). 4-methylbenzene-1-sulfonyl chloride (132 mg, 0.69 mmol) was added to the reaction mixture, and stirred at room temperature for 3 hours. After the end of the reaction, the reactants were poured into water, extracted with DCM (twice), dried with MgSO₄, and concentrated under reduced pressure. The residues were purified by HX/EA (80->95%) column chromatography to obtain the title compound (155 mg). LC-MS (ESI, m/z): [M+H]⁺ = 618.4.
¹H NMR (500 MHz, DMSO-*d*₆) δ ppm 9.13 (s, 1 H) 8.57 (s, 1H) 8.24 (s, 1 H) 7.69 - 7.78 (m, 3 H) 7.35 - 7.47 (m, 3 H) 4.75 - 4.87 (m, 2 H) 4.38 (d, J=12.51 Hz, 1 H) 4.08 - 4.21 (m, 1H) 4.04 (d, J=12.97 Hz, 2 H) 3.88 (s, 3 H) 3.80 (d, J=10.83 Hz, 2 H) 3.73 (t, J=7.63 Hz, 2 H) 3.34 - 3.44 (m, 2 H) 2.78 - 2.96 (m, 2 H) 2.45 (s, 3H) 2.36 (s, 2 H)

### Example B-9: Preparation of tert-butyl(S)-4-((1-(2-(2-((5-(1-(1H-pyrazole-4-yl)-1H-[1,2,3]triazolo[4,5-b]pyrazine-1-yl)methyl)morpholino)pyrimidine-5-il)azetidine-3-il)methyl)piperazine-1-carboxylate

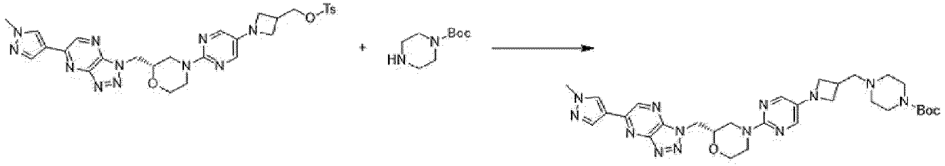

Potassium carbonate (13.4 mg, 0.09 mmol)) and sodium iodide (4.8 mg, 0.03 mmol) were added to a solution of (1-{2-[(2S)-2-{[5-(1-methyl-1H-pyrazol-4-yl)-1H-[1,2,3]triazolo[4,5-b]pyrazine-1-yl]methyl}morpholine-4-yl]pyrimidine-5-yl}azetidine-3-yl)methyl 4-methylbenzene-1-sulfonate (20 mg, 0.03 mmol). The reactants were stirred at 80 °C overnight. After the reaction was completed, the reactants were concentrated under decompression. The residues were purified by DCM/Me (0~3%) column chromatography to obtain the title compound (10 mg). MS (ESI, m/z): [M+H]⁺ = 632.4.
¹H NMR (500 MHz, DMSO-*d*₆) δ ppm 9.14 (s, 1 H) 8.57 (s, 1 H) 8.24 (s, 1 H) 7.75 (s, 2 H) 4.75 - 4.89 (m, 2 H) 4.38 (d, J=12.66 Hz, 1H) 4.12 (br. s., 1 H) 4.04 (d, J=12.82 Hz, 1 H) 3.88 (s, 3 H) 3.81 (t, J=7.25 Hz, 3 H) 3.32 - 3.41 (m, 2 H) 2.82 - 2.88 (m, 2 H) 2.79 (t, J=4.50 Hz, 3 H) 2.29 (br. s., 2H) 2.24 (br. s., 3 H) 1.80 - 1.94 (m, 4 H) 1.30 - 1.38 (m, 9 H)

### Example B-10: Preparation of (S)-2-((5-(1-methyl-1H-pyrazole-4-yl)-1H-[1,2,3]triazolo[4,5-b]pyrazin-1-yl)methyl)-4-(5-(3-((4-methylpiperazine-1-yl)methyl)azetidine-1-il)pyrimidine-2-yl)morpholine

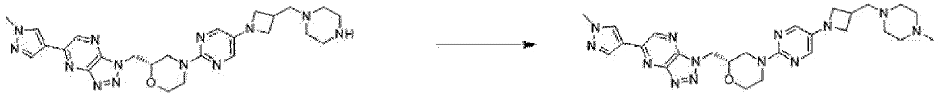

Methanol (1% acetic acid) (1 ml) was added to (2S)-2-{[5-(1-methyl-1H-pyrazole-4-yl)-1H-[1,2,3]triazolo[4,5-b]pyrazine-1-yl]methyl}-4-(5-{3-[(piperazine-1-yl)methyl]azetidine-1-day}pyrimidin-2-yl)morpholine (20 mg, 0.04 mmol) in a solution of morpholine (20 mg, 0.04 mmol) with formaldehyde in H₂O (3.95 mg, 0.13 mmol) and NaBH₄ (2.5 mg, 0.07 mmol). The reactants were stirred at room temperature for 1 hour. After the reaction was completed, the reactants were concentrated under decompression. The residue was purified by DCM/MeOH (0~5%) amine column chromatography, and the title compound (15mg) was obtained. MS (ESI, m/z): [M+H]⁺ = 546.7.
¹H NMR (500 MHz, DMSO-*d*₆) δ ppm 9.14 (s, 1 H) 8.57 (s, 1 H) 8.24 (s, 1 H) 7.76 (s, 2 H) 4.76 - 4.88 (m, 1 H) 4.38 (d, J=12.05 Hz, 1 H) 4.04 (d, J=13.28 Hz, 1H) 3.88 (s, 3 H) 3.74 - 3.85 (m, 2 H) 3.18 - 3.44 (m, 11) 2.97 (br. s., 1 H) 2.76 - 2.90 (m, 2 H) 2.52 - 2.60 (m, 1 H) 1.81 - 1.93 (m, 4H) 2.14 (s, 3 H)

### Example B-11: Preparation of (S)-4-(5-(3-(((4-methoxybenzyl)oxy)methyl)azetidine-1-il)pyrimidine-2-yl)-2-((5-(1-methyl-1H-pyrazole-4-yl)-1H-[1,2,3]triazolo[4,5-b]pyrazine-1-yl)methyl)morpholine

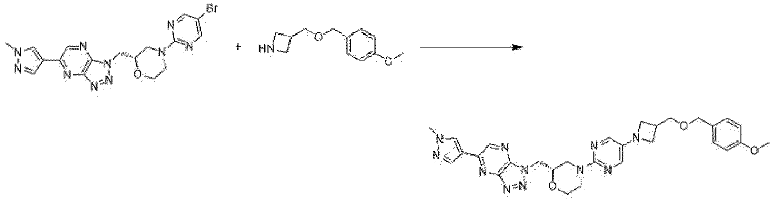

Pd₂(dba)₃ (0.4 g, 5.44 mmol), Xantphos (0.26 g, 0.44 mmol), and NaOt-Bu (0.54 g, 5.47 mmol) were added to a solution of (2S)-4-(5-(5-methyl-1H-pyrazol-4-yl)-1H-[1,2,3]triazolo[4,5-b]pyrazine-1-yl]methyl}morpholine (1.0 g, 2.2 mmol), 3-{[(4-methoxyphenyl)methoxy]methyl}azetidine (0.7 g, 3.28 mmol), Xantphos (0.26 g, 0.44 mmol), and NaOt-Bu (0.54 g, 5.47 mmol). The reactants were stirred at 90 °C for 3 hours. After the end of the reaction, the reactants were poured into water, extracted with DCM (twice), dried with MgSO₄, and concentrated under reduced pressure. The residue was purified by DCM/MeOH (0~5%) column chromatography to obtain the title compound (0.85g). MS (ESI, m/z): [M+H]⁺ = 584.6.
¹H NMR (500 MHz, DMSO-*d*₆) δ ppm 9.13 (s, 1 H) 8.57 (s, 1 H) 8.24 (s, 1 H) 7.74 (s, 2 H) 7.17 (m, J=8.39 Hz, 2 H) 6.83 (m, J=8.39 Hz, 2 H) 4.73 - 4.89 (m, 2 H) 4.31 - 4.41 (m, 3 H) 4,07 - 4.16 (m, 1 H) 4.03 (d, J=12.82 Hz, 1 H) 3.88 (s, 3 H) 3.72 - 3.83 (m, 3 H) 3.67 (s, 3 H) 3.51 (d, J=6.56 Hz, 2 H) 3.34 - 3.47 (m, 3 H) 2.75 - 2.91 (m, 3 H)

### Example B-12: Preparation of tert-butyl(S)-(4-(((1-(2-(2-((5-(1H-pyrazole-4-yl)-1H-[1,2,3]triazolo[4,5-b]pyrazine-1-yl)methyl)morpholino)pyrimidine-5-il)azetidine-3-il)methoxy)methyl)phenyl)carbamate

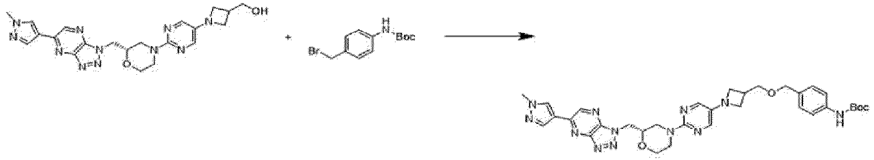

Sodium hydride (40.0 mg, 0.24 mmol) and t-butyl N-[4-(bromomethyl)phenyl]carbamate (123 mg, 0.43 mmol) were added to a solution of (1-{2-[(2S)-2-{[5-(1S)-2-{5-(1-methyl-1H-pyrazole-4-yl)-1H-[1,2,3]triazolo[4,5-b]pyrazine-1-yl}methyl-4-yl}methyl-4-yl-methyl-1-yl-methyl (1,2,3 mmol) in THF (5 ml). The reactants were stirred at 50°C for 3 hours. After the end of the reaction, the reactants were poured into water, extracted with EA (twice), dried with MgSO₄, and concentrated under reduced pressure. The residue was purified by chromatography and the title compound (100mg) was obtained. MS (ESI, m/z): [M+H]⁺ = 670.5.
¹H NMR (500 MHz, DMSO-*d*₆) δ ppm 9.13 (s, 1 H) 8.52 - 8.61 (m, 1 H) 8.24 (s, 1 H) 7.98 (s, 1 H) 7.68 - 7.79 (m, 1 H) 7.29 (d, J=8.24 Hz, 1 H) 6.90 - 7.11 (m, 2 H) 4.73 - 4.84 (m, 1 H) 4.48 (dd, J=7.71, 6.03 Hz, 1 H) 4.37 (d, J=12.82 Hz, 1 H) 4.23 (s, 1 H) 4.13 - 4.20 (m, 1H) 4.03 (d, J=13.12 Hz, 1 H) 3.88 (s, 3 H) 3.79 (d, J=10.99 Hz, 1 H) 3.47 (d, J=7.32 Hz, 1 H) 3.33 - 3.41 (m, 6 H) 3.11 - 3.22 (m, 1 H) 2.76 - 2.89 (m, 1 H) 1.35 - 1.45 (m, 11 H)

### Example B-13: Preparation of tert-butyl(S)-(5-(((1-(2-((5-(1-(2-((5-(1H-pyrazole-4-yl)-1H-[1,2,3]triazolo[4,5-b]pyrazine-1-yl)methyl)morpholino)pyrimidine-5-il)azetidine-3-il)methoxy)methyl)pyridine-2-il)carbamate

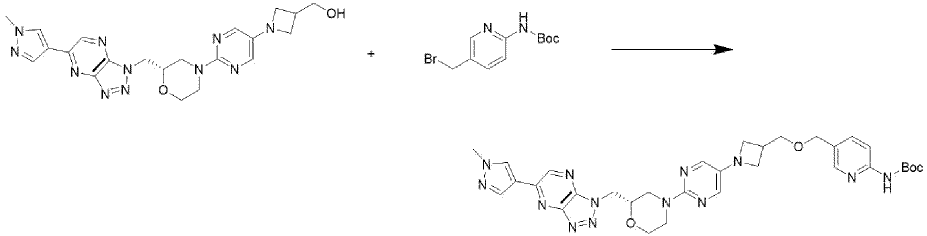

Sodium hydride (40.0 mg, 1.66 mmol) and t-butyl N-[5-(5-(bromomethyl)pyridine-2-yl)-1H-[1,2,3]triazolo[4,5-b]pyrazine-1-yl]methyl}morpholine-4-yl]pyrimidin-5-yl}azetidine-3-yl) methanol (110 mg, 0.24 mmol) in THF (5 ml) and t-butyl N-[5-(bromomethyl)pyridine-2-yl]carbamate (123 mg, 0.43 mmol) were added. The reactants were stirred at 50 °C for 3 hours. After the end of the reaction, the reactants were poured into water, extracted with EA (twice), dried with MgSO₄, and concentrated under reduced pressure. The residue was purified by chromatography and the title compound (100mg) was obtained. MS (ESI, m/z): [M+H]⁺ = 669.5.
¹H NMR (500 MHz, DMSO-*d*₆) δ ppm 9.55 - 9.67 (m, 1 H) 9.13 (s, 1 H) 8.57 (s, 1H) 8.24 (s, 1 H) 8.13 - 8.22 (m, 1H), 7.97 - 8.07 (m, 2 H) 7.44 - 7.65 (m, 3 H) 4.84 - 4.96 (m, 2 H) 4.74 - 4.84 (m, 2 H) 4.72 (br. s., 1 H) 4.43 - 4.52 (m, 1 H) 4.39 (d, J=12.97 Hz, 1 H) 4.31 (br. s., 1 H) 4.25 (s, 1 H) 4.17 (t, J=6.03 Hz, 1 H) 4.03 - 4.13 (m, 1H) 3.88 (s, 3 H) 3.79 (d, J=11.60 Hz, 1 H) 3.45 (d, J=7.17 Hz, 1 H) 3.32 - 3.42 (m, 1 H) 3.09 - 3,20 (m, 1 H) 2.79 - 2.92 (m, 1 H) 1.80 - 1.94 (m, 1 H) 1.33 - 1.43 (m, 9 H)

### Example C: Preparation of a cMET target ligand compound based on a tepotinib derivative Example C

### Example C-1: Preparation of tert-butyl(2R)-2-(((2-(3-((3-(3-cyanophenyl)-6-oxopyridazin-1(6H)-yl)methyl)phenyl)pyrimidine-5-yl)oxy)methyl)methyl-4-carboxylate

### 1-1) Preparation of t-butyl(2R)-2-[({2-[3-(hydroxymethyl)phenyl]pyrimidine-5-yl}oxy)methyl]morpholine-4-carboxylate

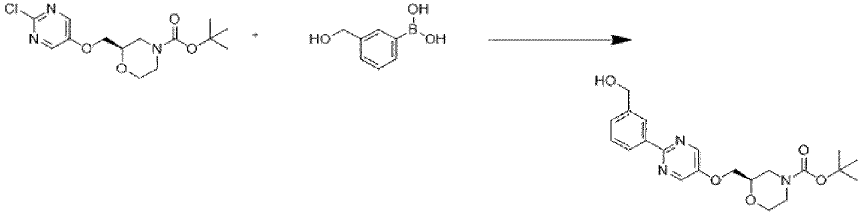

Under nitrogen atmospheric conditions, a mixture of 1.4-dioxane and t-butyl 2-{[(2-chlorpyrimidine-5-yl)oxy]methyl}morpholine-4-carboxylate (6.51 g (19.7 mmol), [3-(hydroxymethyl)phenyl]boronic acid (6.51 g 19.7 mmol), Pd(PPh₃)₄ (1.13 g, 1 mmol) and potassium carbonate (6.80 g, 49.2 mmol) in water were stirred at 80°C for 2 hours. After that, the reactant was cooled to room temperature, filtered, and extracted as dichloromethane (100 mL x 3). After that, the organic layers were combined, washed with brine (100 mL x 2), dried with sodium sulfate anhydrous, filtered and concentrated. The residue was purified with EA/Hex (50-100%) MPLC and the title compound (4.5 g) was obtained. MS (ESI, m/z): [M+H]⁺ = 402.5.

### 1-2) Preparation of t-butyl(2R)-2-({[2-(3-{[3-(3-cyanophenyl)-6-oxo-1,6-dihydropyridazin-1-yl]methyl}phenyl)pyrimidine-5-yl]oxy}methyl)morpholine-4-carboxylate

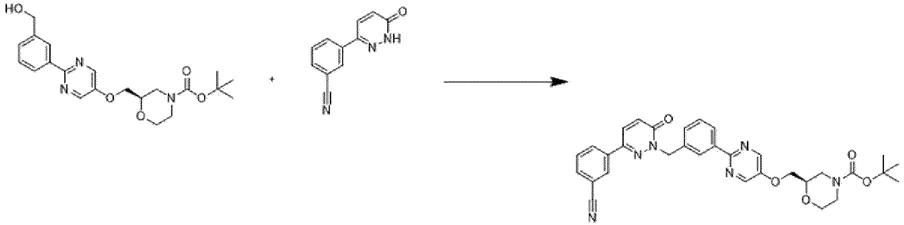

t-butyl(2R)-2-[({2-[3-(hydroxymethyl)phenyl]pyrimidine-5-yl}oxy)methyl]morpholine-4-carboxylate (3 g, 7.47 mmol) and 3-(6-oxo-1,6-dihydropyridazine-3-yl) benzonitrile (1.47 g, 7.47 mmol) were dissolved in THF (50 ml). Triphenylphosphine (2.35 g, 8.97 mmol) was slowly added to the mixture at 0°C. Subsequently, (E)-N-{[(propane-2-yloxy)carbonyl]imino}(propane-2-yloxy)formamide (1.81 g, 8.97 mmol) was added. Formamide was added and the reaction mixture immediately turned green. The reaction mixture was stirred at room temperature for 3 hours (color change to orange). The reaction mixture was then concentrated and dissolved in a solution (HX/EA EA 30%). To aid in the crystallization of triethylphosphine, triethylphosphine seed was added to the reaction mixture. Afterwards, the solidified triethylphosphine was filtered and the liquid product was concentrated. The residue was purified with EA/Hex (30-50%) MPLC and the title compound (3.5 g) was obtained. MS (ESI, m/z): [M+H]⁺ = 581.6.
¹H NMR (500 MHz, DMSO-*d*₆) δ 9.29 (br. s., 1 H) 9.21 (br. s., 1 H) 8.57 - 8.74 (m, 2 H) 8.37 (d, J=12.36 Hz, 1 H) 8.23 (d, J=7.48 Hz, 1 H) 8.16 (d, J=9.77 Hz, 1 H) 7.89 - 7.96 (m, 1 H) 7.71 (t, J=8.01 Hz, 1 H) 7.45 - 7.52 (m, 1 H) 7.16 (d, J=9.77 Hz, 1 H) 5.44 (s, 1 H) 4.23 - 4.36 (m, 2 H) 4.00 - 4.14 (m, 2 H) 3.78 (t, J=12.13 Hz, 1 H) 3.40 (d, J=12.36 Hz, 1 H) 3.25 (d, J=12.51 Hz, 1 H) 2.97 - 3.11 (m, 2 H) 2.89 (s, 1 H) 2.73 (s, 1 H) 2.48 - 2.53 (m, 1 H) 1.48 (m, 9 H)

### Example C-2: Preparation of (S)-3-(1-(3-(5-(morpholine-2-ylmethoxy)pyrimidine-2-yl)benzyl)-6-oxo-1,6-dihydropyridazine-3-yl)benzonitrile

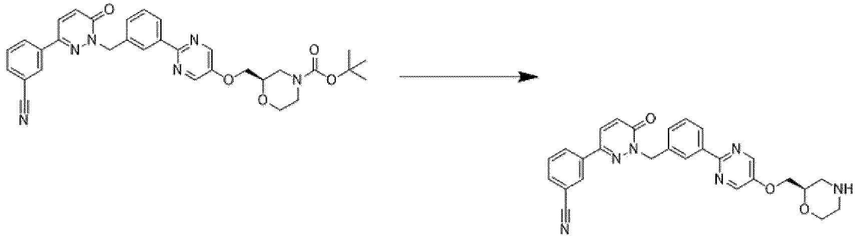

30% TFA was added to the solution of t-butyl(R)-2-(((2-(3-((3-(3-(3-cyanophenyl)-6-oxopyridazin-1(6H)-yl)methyl)phenyl)pyridine-5-yl)oxomethyl)morpholine-4-carboxylate (4.5g, 7.74mmol) solution in DCM. The reactants were stirred at room temperature for 2 hours. After the reaction was completed, the reactants were concentrated under decompression. The residues were purified by DCM/MeOH (0~5%) amine column chromatography, and the title compound (3.11 g) was obtained. MS (ESI, m/z): [M+H]⁺ = 481.5.
¹H NMR (500 MHz, DMSO-*d*₆) δ 9.29 (br. s., 1 H) 9.21 (br. s., 1 H) 8.57 - 8.74 (m, 2 H) 8.37 (d, J=12.36 Hz, 1 H) 8.23 (d, J=7.48 Hz, 1 H) 8.16 (d, J=9.77 Hz, 1 H) 7.89 - 7.96 (m, 1 H) 7.71 (t, J=8.01 Hz, 1 H) 7.45 - 7.52 (m, 1 H) 7.16 (d, J=9.77 Hz, 1 H) 5.44 (s, 1 H) 4.23 - 4.36 (m, 2 H) 4.00 - 4.14 (m, 2H) 3.78 (t, J=12.13 Hz, 1 H) 3.40 (d, J=12.36 Hz, 1 H) 3.25 (d, J=12.51 Hz, 1 H) 2.97 - 3.11 (m, 2 H) 2.89 (s, 1 H) 2.73 (s, 1 H) 2.48 - 2.53 (m, 1 H)

### Example C-3: Preparation of t-butyl 4-(2-{[2-(3-{[3-{3-(3-cyanophenyl)-6-oxo-1,6-dihydropyridazin-1-yl]methyl}phenyl)pyrimidine-5-yl]oxy}ethyl)piperazine-1-carboxylate

### 3-1) Preparation of Synthesis of t-butyl(2R)-2-[({2-[3-(hydroxymethyl)phenyl]pyrimidine-5-yl}oxy)methyl]morpholine-4-carboxylate

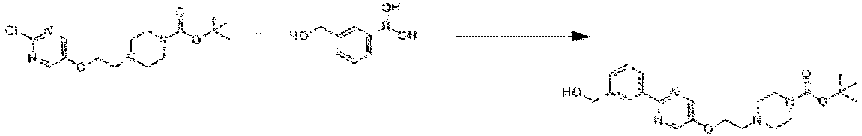

Under nitrogen atmospheric conditions, a mixture of 1.4-dioxane and t-butyl 4-{2-[(2-chloropyrimidine-5-yl)oxy]ethyl}piperazine-1-carboxylate (2.0 g, 5.8 mmol), [3-(hydroxymethyl)phenyl]boronic acid (0.87 g, 5.83 mmol), Pd(PPh₃)₄ (0.34 g, 0.29 mmol) and potassium carbonate (2.0 g, 14.6 mmol) in water were stirred at 80°C for 2 hours. After that, the reactant was cooled to room temperature, filtered, and extracted with dichloromethane (100mL x 3). After that, the organic layers were combined, washed with brine (100mL x 2), dried with anhydrous sodium sulfate, filtered and concentrated. The residue was purified with EA/Hex (50-100%) MPLC and the title compound (1.8 g) was obtained. MS(ESI, m/z): [M+H]⁺ = 415.5.

### 3-2) Preparation of tert-butyl 4-(2-(2-(3-((3-(3-(3-(3-cyanophenyl)-6-oxopyridazin-1(6H)-yl)methyl)phenyl)pyrimidine-5-yl)oxy)ethyl)piperazine-1-carboxylate

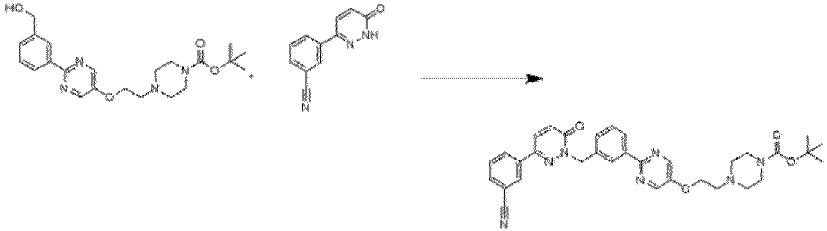

T-butyl 4-[2-({2-[3-(hydroxymethyl)phenyl]pyrimidine-5-yl}oxy)ethyl]piperazine-1-carboxylate (2.2 g, 5.31 mmol) and 3-(6-oxo-1,6-dihydropyridazine-3-yl) benzonitrile (1.05 g, 5.31 mmol) were dissolved in THF (50 ml). Triphenylphosphine (1.67 g, 6.37 mmol) was slowly added to the mixture. Subsequently, (E)-N-{[(propane-2-yloxy)carbonyl]imino}(propane-2-yloxy)formamide (1.05 g, 6.37 mmol) was dropped. Formamide was added and immediately turned green. The reaction mixture was stirred at room temperature for 3 hours (color change to orange). The reaction mixture was then concentrated and dissolved in a solution (HX/EA EA 30%). To aid in the crystallization of triethylphosphine, triethylphosphine seed was added to the reaction mixture. Afterwards, the solidified triethylphosphine was filtered and the liquid product was concentrated. The residual sand was purified with EA/Hex (30-50%) MPLC and the title compound (3.1 g) was obtained. MS (ESI, m/z): [M+H]⁺ = 594.7.
¹H NMR (500 MHz, DMSO-*d*₆) δ 8.66 (s, 3 H) 8.38 (d, J=6.56 Hz, 2 H) 8.17 - 8.27 (m, 3 H) 7.90 - 7.97 (m, 1 H) 7.68 - 7.74 (m, 1 H) 7.43 - 7.52 (m, 2 H) 7.17 (d, J=9.77 Hz, 1 H) 5.45 (s, 2 H) 4.75 - 4.89 (m, 1 H) 4.53 (d, J=4.42 Hz, 3 H) 3.51 (br. s., 2 H) 1.13 - 1.26 (m, 5 H) 1.42 (s, 9 H)

### Example C-4: Preparation of 3-(6-oxo-1-(3-(5-(2-(piperazine-1-yl)ethoxy)pyrimidine-2-yl)benzyl)-1,6-dihydropyridazine-3-yl)benzonitrile

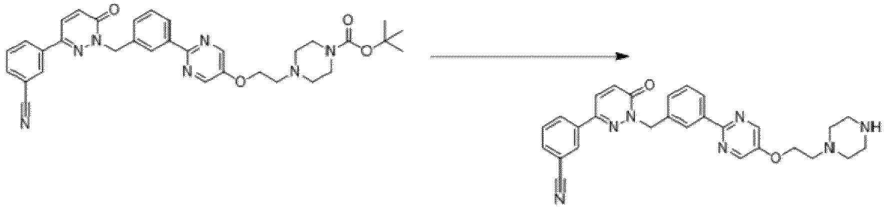

30% TFA was applied to a solution of t-butyl 4-(2-{[2-(3-{[3-(3-cyanophenyl)-6-oxo-1,6-dihydropyridazin-1-yl]methyl}phenyl)pyrimidine-5-yl]oxy}ethyl)piperazine-1-carboxylate (1.5 g, 2.53 mmol) in DCM. The reactants were stirred at room temperature for 2 hours. After the reaction was completed, the reactants were concentrated under decompression. The residues were purified by DCM/MeOH (0~5%) amine column chromatography, and the title compound (1.22 g) was obtained. MS (ESI, m/z): [M+H]⁺ = 494.6.

### Example C-5: Preparation of t-butyl 4-(2-(3-((3-(3-(3-cyanophenyl)-6-oxopyridazine-1(6H)-yl)methyl)phenyl)pyrimidine-5-yl)piperazine-1-carboxylate

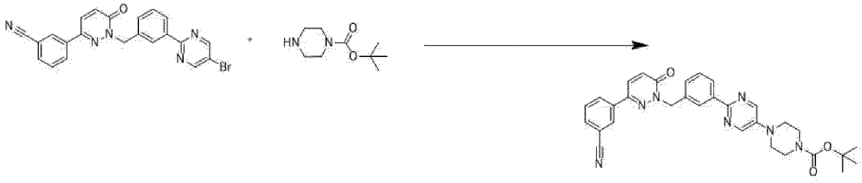

A mixture of 3-(1-{[3-(5-bromolimidine-2-yl)phenyl]methyl}-6-oxo-1,6-dihydropyridazin-3-ylbenzonitrile (0.3 g, 0.67 mmol), t-butylpiperazine-1-carboxylate (0.17 g, 0.67 mmol), Pd2 (dba)3 (19.4 mg, 33.8 µmol), Xantphos (39.1 mg, 67.5 µmol), and Na-t-butoxide (162 mg, 1.69 mmol) in toluene (3 mL) were stirred in a microwave at 100°C for 2 hours. After the reaction was completed, the reaction mixture was filtered and concentrated under reduced pressure. The residue was purified with EA/Hex (50-100%) MPLC and the title compound was obtained (310 mg). MS (ESI, m/z): [M+H]⁺ = [560.7].
¹H NMR (500 MHz, DMSO-*d*₆) δ 8.66 (s, 3 H) 8.38 (d, J=6.56 Hz, 2 H) 8.17 - 8.27 (m, 3 H) 7.90 - 7.97 (m, 1 H) 7.68 - 7.74 (m, 1 H) 7.43 - 7.52 (m, 2 H) 7.17 (d, J=9.77 Hz, 1 H) 5.45 (s, 2 H) 4.75 - 4.89 (m, 1 H) 4.53 (d, J=4.42 Hz, 3 H) 3.51 (br. s., 2 H) 1.13 - 1.26 (m, 1 H) 1.42 (s, 9 H)

### Example C-6: Preparation of 3-(6-oxo-1-(3-(5-(piperazine-1-yl)pyrimidine-2-yl)benzyl)-1,6-dihydropyridazine-3-yl)benzonitrile

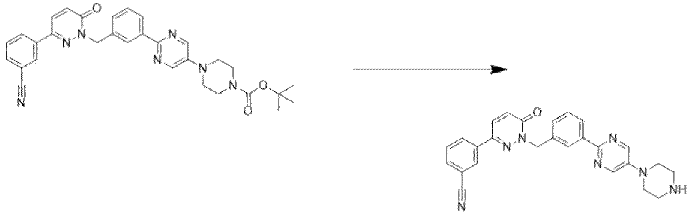

30% TFA was applied to a solution of t-butyl 4-(2-(3-(3-(3-(3-cyanophenyl)-6-oxopyridazine-1(6H)-yl)methyl)phenyl)pyrimidine-5-yl)piperazine-1-carboxylate (310 mg, 0.50 mmol) in DCM. The reactants were stirred at room temperature for 1 hour. After the end of the reaction, the residue was concentrated under reduced pressure, purified with DCM/MeOH (0~5%) amine column chromatography, and the title compound was obtained (250 mg). MS (ESI, m/z): [M+H]⁺ = [460.6].
¹H NMR (500 MHz, DMSO-*d*₆) δ 8.66 (s, 3 H) 8.38 (d, J=6.56 Hz, 2 H) 8.17 - 8.27 (m, 3 H) 7.90 - 7.97 (m, 1 H) 7.68 - 7.74 (m, 1 H) 7.43 - 7.52 (m, 2 H) 7.17 (d, J=9.77 Hz, 1 H) 5.45 (s, 2 H) 4.75 - 4.89 (m, 1 H) 4.53 (d, J=4.42 Hz, 3 H) 3.51 (br. s., 2 H) 1.13 - 1.26 (m, 1 H)

### Example D: Preparation of a PROTAC targeting an ABN derivative-based cMET protein

### Example D-1 : Preparation of 3-(6-(4-((1-(2-((S)-2-((5-(1-methyl-1H-pyrazole-4-yl)-1H-[1,2,3]triazolo[4,5-b]pyrazine-1-yl)methyl)morpholine-5-il)azetidin-3-il)methyl)piperazine-1-il)piperazine-1-il)1-oxopthalazine-2(1H)-day)piperidine-2,6-dione

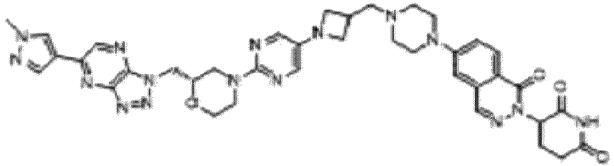

### 1-1) Preparation of 4-fluoro-2-formylbenzoate

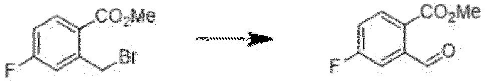

At room temperature, NMO (15.6 mg, 134 mmol) and 4Å molecular sieve were sequentially added to a solution of methyl 2-(bromomethyl)-4-fluorobenzoate (22.0 g, 89 mmol) in DCM (100 mL). The reactants were stirred at room temperature for 2 hours. The molecular sieve was filtered and washed with DCM (50ml). The DCM layer was washed with water (200ml), dried with MgSO₄, and concentrated under reduced pressure. The residue was purified by column chromatography, and the title compound was obtained as a white solid (12.0 g). MS (ESI, m/z): [M+H]⁺ = 183.2

### 1-2) Preparation of 4-fluoro-2-formylbenzoic acid

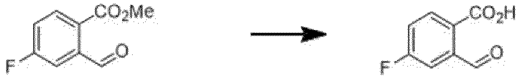

At room temperature, an aqueous solution (50 ml) of lithium (+1) hydroxide (13.8 g, 329 mmol) was added to a solution of methyl 4-fluoro-2-formylbenzoate (12.0 g, 65.9 mmol) in THF (50 mL). After 2 h of agitation, the reactants were decompressively concentrated to dry the THF. After cooling to 0°C, the reactants were acidified with 1N HCl and the pH was adjusted to 4. The reactants were extracted twice with ethyl acetate (50ml). The combined ethyl acetate layer was dried with MgSO4, concentrated under reduced pressure, and white crystals were obtained (10.0 g). MS (ESI, m/z): [M+H]+ = 169.2.

### 1-3) Preparation of 6-fluoro-1,2-dihydrophthalazine-1-one

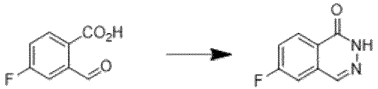

At room temperature, NH2NH2 monohydrate (2.02 mg, 40.3 mmol) was added to a solution of 4-fluoro-2-formylbenzoic acid (6.78 g, 40.3 mmol) in methanol (50 mL). After stirring for 30 minutes, the reactants were heated to 80°C for 2 hours. The reactants were cooled to room temperature and concentrated under reduced pressure. The residue was poured into water (150ml) and extracted twice with ethyl acetate (150ml). The combined ethyl acetate layer was dried with MgSO₄, concentrated under pressure, and white crystals were obtained (5.5 g). MS (ESI, m/z): [M+H]⁺ = 165.3.

### 1-4) Preparation of 3-(6-fluoro-1-oxopthalazine-2(1H)-yl)piperidine-2,6-dione

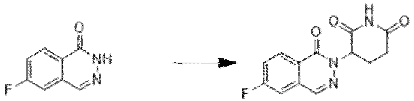

Sodium 2-methylpropane-2-oleate (175 mg, 0.91 mmol) was added to a 6-fluoro-1,2-dihydroptalazine-1-one (100 mg, 0.61 mmol) solution in DMF (2 mL) at 0°C. After stirring for 30 minutes, 3-bromoperidin-2,6-dione (90 mg, 0.47 mmol) was added to the reaction mixture, and stirred at room temperature for 6 hours. Water (20ml) was poured into the reaction mixture, and ethyl acetate (20ml) was extracted twice. The mixed ethyl acetate was dried with MgSO₄ and concentrated under reduced pressure. The residue was purified by column chromatography, and the title compound was obtained as white crystals (110.0 mg, 65.6%). MS (ESI, m/z): [M+H]⁺ = 276.6.
¹H NMR (400 MHz, DMSQ-*d*₆) δ 11.11 (s, 1H), 8.50 (s, 1H), 8.38 (dd, J=8.86, 5.44 Hz, 1H), 7.72 - 7.89 (m, 2H), 5.70 - 5.90 (m, 11H), 2.56 - 2.70 (m, 2H), 2.11 - 2.25 (m, 1H)

### 1-5) Preparation of t-butyl 4-(2-(2,6-dioxopiperidin-3-yl)-1-oxo-1,2-dihydroptalazine-6-yl)piperazine-1-carboxylate

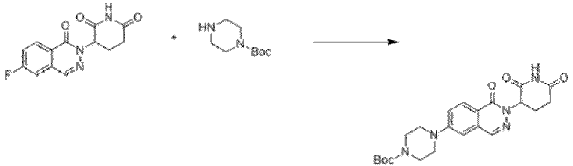

Ethylbis (propane-2-yl)amine (4.0 equivalent) was added to 3-(6-fluoro-1-oxophthalazine-2(1H)-yl) piperidine-2,6-dione (300 mg, 1.04 mmol) and t-butylpiperazine-1-carboxylate (386 mg, 2.07 mmol) solutions in NMP (3.0 ml). The reactants were stirred at 130 °C for 48 hours. After the reaction was completed, the reaction mixture was concentrated under reduced pressure. The residues were purified by MeOH/DCM (0-20%) column chromatography and the title compound was obtained (350 mg). MS (ESI, m/z): [M+H]⁺ = 442.4

### Preparation of 1-6) 3-(1-oxo-6-(piperazine-1-yl)phthalazine-2(1H)-yl)piperidine-2,6-dione

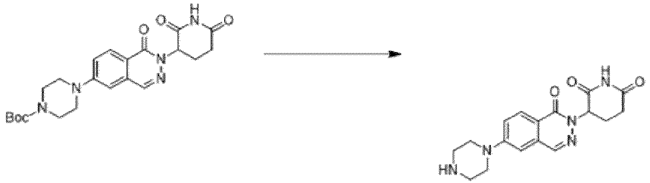

TFA (10 ml) was added to the t-butyl 4-(2-(2,6-dioxopiperidine-3-yl)-4-methyl-1-oxo-1,2-dihydroptalazin-6-yl) solution in DCM (30 ml). The reactants were stirred at room temperature for 1 hour. After the reaction was completed, the reaction mixture was concentrated under reduced pressure. The residues were purified by MeOH/DCM (0-20%) column chromatography and the title compound was obtained (200 mg). MS (ESI, m/z): [M+H]⁺ = 342.4

### 1-7) Preparation of 3-(6-(4-((1-(2-((S)-2-((5-(1-methyl-1H-pyrazole-4-yl)-1H-[1,2,3]triazolo[4,5-b]pyrazine-1-yl)methyl)morpholino)pyrimidin-5-il)azetidine-3-il)methyl)piperazine-1-yl)1-oxopthalazine-2(1H)-yl)piperidine-2,6-dione

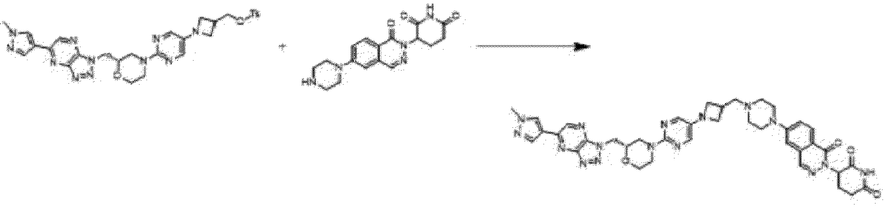

Potassium carbonate (10.1 mg, 0.07 mmol) and sodium iodide (4.73 mg, 0.032 mmol) were added to the solution of (1-{2-[(2S)-2-{[5-(1-methyl-1H-pyrazone-4-yl)-1H-[1,2,3]triazolo[4,5-b]pyrazine-1-yl}pyrimidine-4-yl}pyrimidin-5-yl}azetidine-3-yl)methyl 4-methylbenzene-1-sulfonate (15 mg, 0.02 mmol) and 3-[1-oxo-6-(piperazine-1-yl)-1,2-dihydroptalazine-2-yl]piperidine-2,6-dione (12.4 mg, 0.03 mmol). The reactants were stirred at 80 °C overnight. After the reaction was completed, the reactants were concentrated under decompression. The residues were purified by DCM/Me (0~3%) column chromatography to obtain the title compound (10 mg). MS (ESI, m/z): [M+H]⁺ = 787.9.
¹H NMR (500 MHz, DMSO-*d*₆) δ 11.02 (d, J=5.80 Hz, 1 H) 9.21 (s, 1 H) 8.62 - 8.70 (m, 1 H) 8.23 - 8.36 (m, 2 H) 8.11 (d, J=9.31 Hz, 1 H) 7.52 - 7.61 (m, 1 H) 7.50 (br. s., 1 H) 7.36 (br. s., 1 H) 7.27 (br. s, 1 H) 5.77 (br. s., 1 H) 5.33 (d, J=4.88 Hz, 1 H) 4.79 (br. s., 4H) 4.20 (br. s., 1 H) 4.12 (d, J=11.60 Hz, 1 H) 3.92 - 4.01 (m, 3 H) 3.89 (br. s., 1H) 3.65 (br. s, 2 H) 3.41 - 3.61 (m, 5 H) 2.91 (d, J=12.51 Hz, 2 H) 2.37 (br. s., 1 H) 2.20 - 2.34 (m, 5H) 1.84 - 2.06 (m, 4 H) 1.75 (br. s., 2 H)

### Example D-2 : Preparation of 3-(4-methyl-6-(4-((1-(2-((S)-2-((5-(1-(1H-pyrazole-4-yl)-1H-[1,2,3]triazolo[4,5-b]pyrazine-1-yl)methyl)morpholino)pyrimidine-5-il)azetidine-3-yl)methyl)piperazine-1-yl)1-oxopthalazine-2(1H)-yl)piperidine-2,6-dione

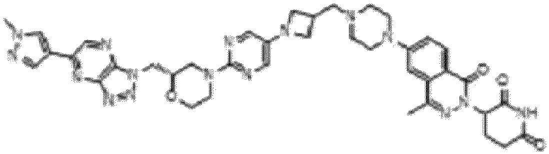

### 2-1) Preparation of methyl 2-acetyl-4-fluorobenzoate

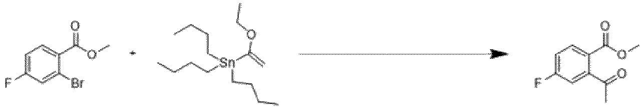

Triphenyl [tri(triphenyl-λ-phospanicyl)palladio]-λ-phosphorfan (4.98 g, 4.3 mmol) was added to a solution of methyl 2-bromo-4-fluorobenzoate (10 g, 43.0 mmol) and tributyl (1-ethoxyethethenyl)stannane (17 g, 47.2 mmol) in toluene (150 ml). The reactants were stirred at 100°C for 20 hours. After the reaction was completed, the reaction mixture was concentrated under reduced pressure. The residual acid was dissolved in 100 ml of THF and 40 ml of 2N HCl was added. The reactants were stirred at room temperature for 30 minutes. After the reaction was completed, the reaction mixture was concentrated under reduced pressure. The residues were purified by HX/EA (0-80%) column chromatography and the title compound was obtained (7.5 g). MS (ESI, m/z): [M+H]⁺ = 457.4.

### 2-2) Preparation of 6-fluoro-4-methyl-1,2-dihydrophthalazine-1-one

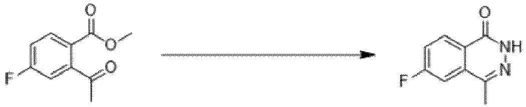

NH₂NH₂ hydrate was added to a solution of methyl 2-acetyl-4-fluorobenzoate (19 g, 97.1 mmol) in methanol (500 mL). The reactants were stirred at room temperature for 2 hours. The reaction mixture was stirred at 85°C for an additional 6 hours. After the end of the reaction, the reaction mixture was concentrated under reduced pressure, water (100 mL) was added and stirred for 3 hours, and the solids were filtered and vacuum-dried to obtain the heading compound (15 g). MS (ESI, m/z): [M+H]⁺ = 179.2.

### 2-3) Preparation of 3-(6-fluoro-4-methyl-1-oxo-1,2-dihydroptalazine-2-yl)piperidine-2,6-dione

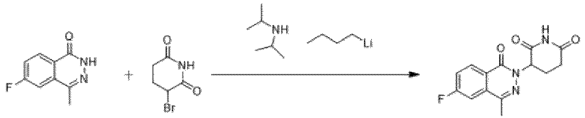

Butyllithium in 2.5M hexane (20.6ml) was added to a solution of bis(propane-2-yl)amine (7.27ml) in THF (75 mL) at -20°C. The reactants were stirred at 0°C for 30 minutes. 6-fluoro-4-methyl-1,2-dihydroptalazin-1-one (7.65 g) was added to the LDA solution and stirred at room temperature for 30 min. Subsequently, 3-bromoperidin-2,6-dione was added at the same temperature. It was returned for 2 hours to remove the solvent and washed with water. The solids were filtered and vacuum-dried to obtain the heading compound (7.9 g). MS (ESI, m/z): [M+H]⁺ = 290.2.

### 2-4) Preparation of t-butyl 4-(2-(2,6-dioxopiperidin-3-yl)-4-methyl-1-oxo-1,2-dihydroptalazine-6-yl)piperazine-1-carboxylate

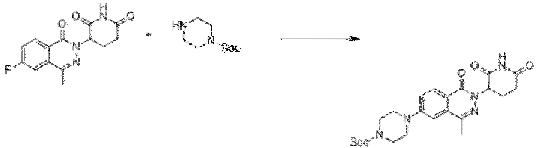

Ethylbis (propane-2-yl)amine (1.5 ml, 4.1 mmol) was added to 3-(6-fluoro-4-methyl-1-oxo-1,2-dihydroptallazine-2-yl) piperidine-2,6-dione (300 mg, 1.04 mmol) and t-butylpiperazine-1-carboxylate (386 mg, 2.07 mmol) solutions in NMP (3.0 ml). The reactants were stirred at 130°C for 48 hours. After the reaction was completed, the reaction mixture was concentrated under reduced pressure. The residues were purified by MeOH/DCM (0-20%) column chromatography and the title compound was obtained (350 mg). MS (ESI, m/z): [M+H]⁺ = 456.4.

### 2-5) Preparation of 3-(4-methyl-1-oxo-6-(piperazine-1-yl)phthalazine-2(1H)-yl)piperidine-2,6-dione

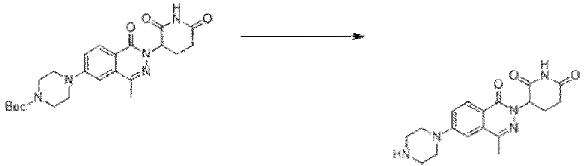

TFA (10 ml) was added to the t-butyl 4-(2-(2,6-dioxopiperidine-3-yl)-4-methyl-1-oxo-1,2-dihydroptalazin-6-yl) solution in DCM (30 ml). The reactants were stirred at room temperature for 1 hour. After the reaction was completed, the reaction mixture was concentrated under reduced pressure. The residues were purified by MeOH/DCM (0-20%) column chromatography and the title compound was obtained (200 mg). MS (ESI, m/z): [M+H]⁺ = 356.4.

### 2-6) Preparation of 33-(4-methyl-6-(4-((1-(2-((S)-2-((5-(1-methyl-1H-pyrazole-4-yl)-1H-[1,2,3]triazolo[4,5-b]pyrazine-1-yl)methyl)morpholino)pyrimidine-5-il)azetidine-3-il)methyl)piperazine-1-yl)1-oxopthalazine-2(1H)-yl)piperidine-2,6-dione

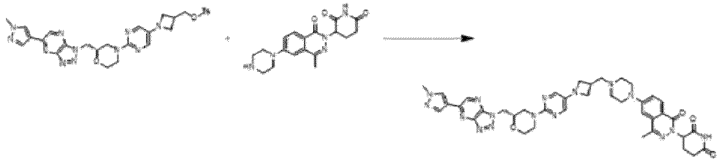

Potassium carbonate (10.1 mg, 0.03 mmol) and sodium iodide (4.73 mg, 0.03 mmol) were added to 1-{2-[(2S)-2-{[5-(1-methyl-1H-pyrazone-4-yl)-1H-[1,2,3]triazolo[4,5-b]pyrazine-1-yl]methyl}morpholine-4-yl]pyrimidin-5-yl}azetidine-3-yll)methyl 4-methylbenzene-1-sulfonate (15 mg, 0.02 mmol) and 3-[4-methyl-1-oxo-6-(piperazine-1-yl)-1,2-dihydropetalazine-2-yl]piperidine-2,6-dione (11.2 mg, 0.03 mmol). The reactants were stirred at 80°C overnight. After the reaction was completed, the reactants were stirred under decompression. The residues were purified by DCM/Me (0~3%) column chromatography and the title compound was obtained (10 mg). MS (ESI, m/z): [M+H]⁺ = 801.9.
¹H NMR (500 MHz, DMSO-*d*₆) δ 10.91 (s, 1 H) 9.09 - 9.19 (m, 1 H) 8.57 (s, 1 H) 8.24 (s, 1 H) 8.00 (d, J=9.00 Hz, 1 H) 7.71 - 7.84 (m, 2H) 7.43 (d, J=9.16 Hz, 1H) 7.02 (s, 1 H) 5.61 (d, J=6.87 Hz, 1 H) 4.78 - 4.90 (m, 2 H) 4.72 (br. s., 1 H) 4.38 (d, J=11.90 Hz, 1 H) 4.12 (d, J=4.58 Hz, 1 H) 4.04 (d, J=12.82 Hz, 1 H) 3.76 - 3.94 (m, 6 H) 3.34 - 3.48 (m, 2 H) 2.93 (d, J=6.10 Hz, 1 H) 2.77 - 2.89 (m, 3 H) 2.47 - 2.64 (m, 4 H) 1.82 - 1.95 (m, 4 H) 1.68 (br. s., 2 H) 1.38 (br. s., 2 H) 1.07 - 1.26 (m, 5 H)

### Example D-3 : Preparation of 3-((3-fluoro-4-(4-((1-(2-((S)-2-((5-(1-(1H-pyrazol-4-yl)-1H-[1,2,3]triazolo[4,5-b]pyrazine-1-yl)methyl)morphopolino)pyrimidine-5-il)azetidine-3-il)methyl)piperazine-1-il)phenyl)amino)piperidine-2,6-dione

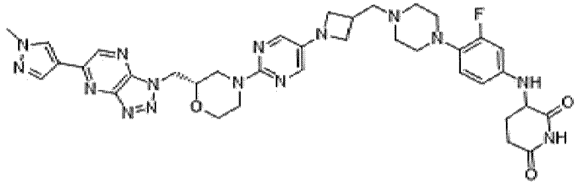

### 3-1) Preparation of t-butyl 4-(2-fluoro-4-nitrophenyl)piperazine-1-carboxylate

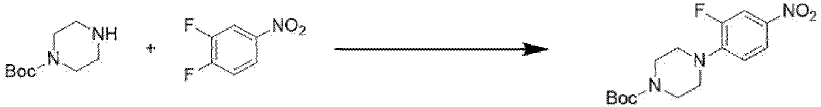

t-butylpiperazine-1-carboxylate (5.9 g, 31.4 mmol) and potassium carbonate (21.7 g, 157 mmol) were added to a solution of 1,2-difluoro-4-nitrobenzene (5 g, 31.4 mmol) in DMF (100 mL). The reactants were stirred at 80°C for 2 hours. After the reaction was completed, the reactant was poured into water and extracted twice with ethyl acetate. Dried over MgSO₄ and concentrated under reduced pressure. The residue was purified by hexane/ethyl acetate (0~30%) column chromatography to obtain the title compound (10.2 g). MS (ESI, m/z): [M+H]⁺ = 326.3.

### 3-2) Preparation of t-butyl 4-(4-amino-2-fluorophenyl)piperazine-1-carboxylate

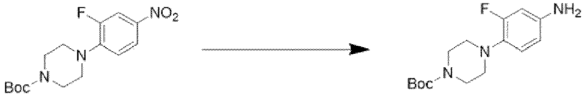

Iron powder (16.5 g, 295 mmol) was added to a solution of t-butyl 4-(2-fluoro-4-nitrophenyl)piperazine-1-carboxylate (4.8 g, 14.8 mmol) in saturated NH4Cl/THF (1:1) (100 mL). The reactants were stirred at 80 °C for 2 hours and used in the next process without further purification. ESI, m/z): [M+H]⁺ = 296.4.

### 3-3) Preparation of t-butyl 4-{4-[(2,6-dioxopiperidin-3-yl)amino]-2-fluorophenyl}piperazine-1-carboxylate preparation

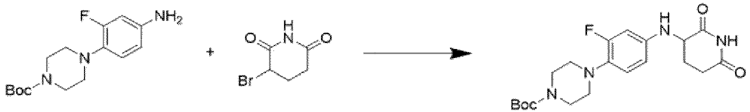

3-bromoperidin-2,6-dione (1.95 g, 10.2 mmol) was added to t-butyl 4-(4-amino-2-fluorophenyl)piperazine-1-carboxylate (1.5 g, 5.1 mmol) solution of t-butyl 4-(4-amino-2-fluorophenyl) piperazine-1-carboxylate (1.5 g, 5.1 mmol) in DMF (30 mL). The reactants were stirred at 85 °C for one night. After the reaction was completed, the reactant was poured into water and extracted twice with ethyl acetate. The residue was purified by hexane/ethyl acetate (0~100%) column chromatography and the title compound was obtained (2.1 g). MS (ESI, m/z): [M+H]⁺ = 407.5.

### 3-4) Preparation of 3-{[3-fluoro-4-(piperazine-1-yl)phenyl]amino}piperidine-2,6-dione manufacturing

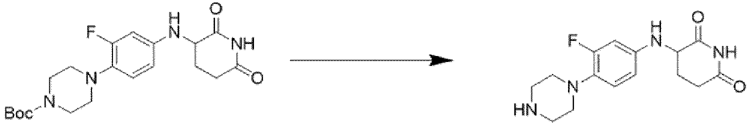

30% TFA was added to a solution of t-butyl 4-{4-[(2,6-dioxopiperidine-3-yl)amino]-2-fluorophenyl}piperazine-1-carboxylate (20 mg, 49.2 µmol) in DCM. The reactants were stirred at room temperature for 2 hours. After the end of the reaction, the reactants were stirred under decompression and used in the next process without further purification. MS (ESI, m/z): [M+H]⁺ = 307.3.

### 3-5) Preparation of 3-((3-fluoro-4-(4-((1-(2-((S)-2-((5-(1-(1H-pyrazole-4-yl)-1H-[1,2,3]triazolo[4,5-b]pyrazine-1-yl)methyl)morpholino)pyrimidine-5-il)azetidine-3-il)methyl)piperazine-1-yl)phenyl)amino)piperidine-2,6-dione

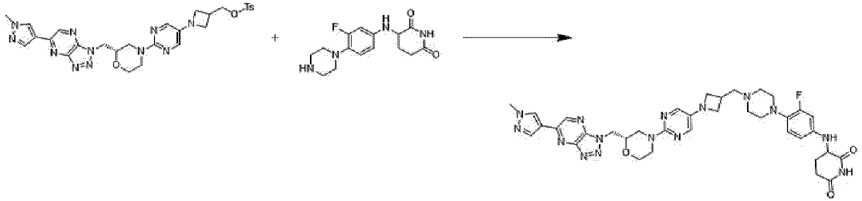

Potassium carbonate (10.1 mg, 0.03 mmol) and sodium iodide (4.73 mg, 0.03 mmol) were added to the solution of (1-{2-[(2S)-2-{[5-(1-methyl-1H-pyrazone-4-yl)-1H-[1,2,3]triazolo[4,5-b]pyrazine-1-yl]methyl}morpholine-4-yl}pyrimidin-5-yl}azetidine-3-yl)methyl 4-methylbenzene-1-sulfonate (15 mg, 0.02 mmol) and 3-{[3-fluoro-4-(piperazine-1-yl)phenyl}piperidine-2,6-dione (11.2 mg, 0.04 mmol). The reactants were stirred at 80 °C for one night. After the reaction was completed, the reactants were concentrated under decompression. The residues were purified by DCM/Me (0~3%) column chromatography and the title compound was obtained (10 mg). MS (ESI, m/z): [M+H]+ = 752.9.
¹H NMR (500 MHz, DMSO-*d*₆) δ 10.71 (s, 1 H) 9.13 (s, 1 H) 8.57 (s, 1H) 8.24 (s, 1 H) 7.69 - 7.81 (m, 2 H) 6.76 (t, J=9.23 Hz, 1 H) 6.39 - 6.51 (m, 1 H) 6.35 (d, J=7.48 Hz, 1 H) 5.74 (d, J=7.63 Hz, 1 H) 4.76 - 4.90 (m, 2 H) 4.71 (br. s., 1H) 4.67 (s, 1 H) 4.38 (d, J=10.99 Hz, 1 H) 4.19 (br, s., 1 H) 4.12 (br. s., 1 H) 4.04 (d, J=13.12 Hz, 1 H) 3.75 - 3.95 (m, 6 H) 3.31 - 3.41 (m, 1 H) 2.80 - 2.92 (m, 4 H) 2.78 (br. s., 4 H) 2.02 (s, 2 H) 1.83 - 1.95 (m, 4 H) 1.68 (br s., 2 H)

### Example D-4: Preparation of 3-(4-methyl-6-(4-((1-((1-((1-(1-(2-((S)-2-((5-(1-(1H-pyrazol-4-yl)-1H-[1,2,3]triazolo[4,5-b]pyrazine-1-yl)methyl)morpholine-5-il)azetidin-3-il)methyl)piperidine-4-ilmethyl)piperazine-1-day)-1-oxopthalazine-2(1H)-yl)piperidine-2,6-dione

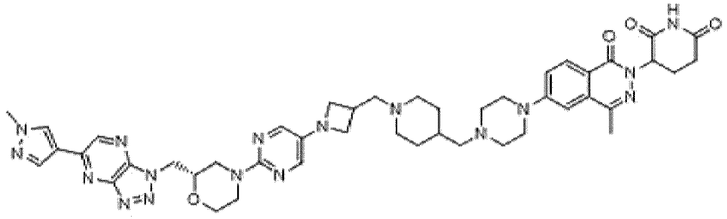

### 4-1) Preparation of t-butyl 4-((4-(2-(2,6-dioxopiperidin-3-yl)-4-methyl-1-oxo-1,2-dihydroptalazine-6-yl)piperazine-1-yl)methyl)piperidine-1-carboxylate

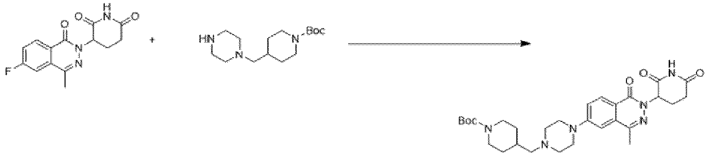

Ethylbis (propane-2-yl)amine (1 ml, 4.35 mmol) was added to 3-(6-fluoro-4-methyl-1-oxopthalazine-2(1H)-yl)piperidine-2,6-dione (300 mg, 1.09 mmol) and t-butyl 4-(piperazine-1-yl-methyl)piperidine-1-carboxylate (600 mg, 2.18 mmol) solutions in NMP (2.0 ml). The reactants were stirred at 130 °C for 48 hours. After the reaction was completed, the reactants were concentrated under decompression. The residues were purified by MeOH/DCM (0~20%) column chromatography and the title compound was obtained (400 mg). MS (ESI, m/z): [M+H]⁺ = 553.4.

### 4-2) Preparation of 3-(4-methyl-1-oxo-6-(4-(piperidine-4-ylmethyl)piperazine-1-yl)phthalazine-2(1H)-yl)piperidine-2,6-dione

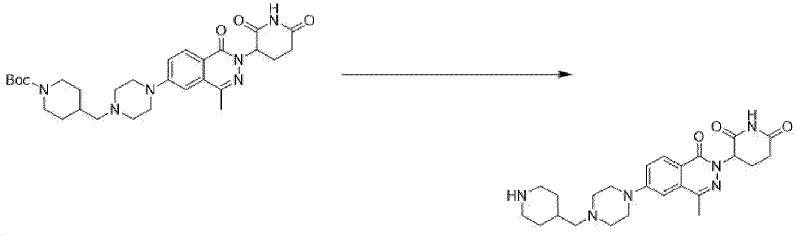

TFA (10 ml) was added to t-butyl 4-((4-(2-(2,6-dioxopiperine-3-yl)-4-methyl-1-oxo-1,2-dihydroptalazin-6-yl)piperazine-1-yl)methyl)piperidine-1-carboxylate (400 mg, 1.52 mmol) in DCM (30 ml). The reactants were stirred at room temperature for 1 hour. After the reaction was completed, the reaction mixture was concentrated under reduced pressure. The residues were purified by MeOH/DCM (0~20%) column chromatography and the title compound was obtained (400 mg). MS (ESI, m/z): [M+H]⁺ = 453.4.

### 4-3) Preparation of 33-(4-methyl-6-(4-((1-((1-(2-((S)-2-((5-(1-(1H)-1H-yl)-1H-[1,2,3]triazolo[4,5-b]pyrazine-1-yl)methyl)morpholino)pyrimidine-5-il)azetidin-3-il)methyl)piperidine-4-il)methyl)piperazine-1-day)-1-oxopthalazine-2(1H)-yl)piperidine-2,6-dione

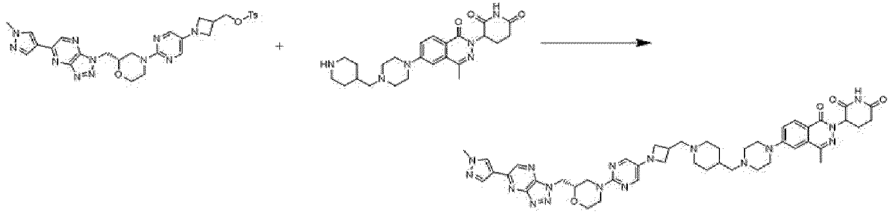

Potassium carbonate (10.1 mg, 0.073 mmol) and sodium iodide (4.73 mmol) in ACN (1 ml) in a solution of (1-{2-[(2S)-2-{[5-(1S)-2-{5-(1-methyl-1H)pyrazine-4-yl)-1H-[1,2,3]triazolo[4,5-b]pyrazine-1-yl]methyl}morpholine-4-yl}azetidine-3-yl)methyl 4-methylbenzene-1-sulfonate (15 mg, 0.02 mmol) and 3-(4-(4-methyl-4-yl)piperazine-1-yl)phthalazine-2(1H)-yl)piperidine-2,6-dione (16.5 mg, 0.03 mmol, 0.03 mmol). The reaction mixture was stirred at 80°C for one night. After the reaction was completed, the reactants were concentrated under decompression. The residues were purified by DCM/Me (0~3%) column chromatography and the title compound was obtained (11 mg). MS (ESI, m/z): [M+H]⁺ = 899.1.
¹H NMR (500 MHz, DMSO-*d*₆,) δ 10.98 (s, 1 H) 9.21 (s, 1 H) 8.65 (s, 1 H) 8.31 (s, 1H) 8.08 (d, J=9.00 Hz, 1 H) 7.85 (br. s., 2 H) 7.49 (dd, J=14.65, 8.24 Hz, 1 H) 7.05 - 7.16 (m, 1H) 5.69 (d, J=11.75 Hz, 1 H) 5.24 - 5.39 (m, 1 H) 4.84 - 4.97 (m, 2 H) 4.79 (br. s., 2 H) 4.41 - 4.52 (m, 1 H) 4.19 (br. s., 1 H) 4.12 (d, J=13.12 Hz, 1 H) 3.96 (s, 3 H) 3.88 (d, J=11.44 Hz, 2 H) 3.50 (d, J=14.95 Hz, 3 H) 3.44 (br. s., 3 H) 3.38 - 3.42 (m, 2 H) 2.87 - 3.01 (m, 3 H) 2.57 - 2.67 (m, 2 H) 2.23 (d, J=5.19 Hz, 2 H) 1.88 - 2.03 (m, 7 H) 1.75 (br. s., 3 H) 1.46 (br. s., 3 H) 1.24 (br. s,, 4 H)

### Example D-5: Preparation of 3-((3-fluoro-4-(4-((4-((4-((1-((S)-2-((5-(1-(1-(S)-2-((5-(1-methyl-1H-pyrazole-4-yl)-1H-[1,2,3]triazolo[4,5-b]pyrazine-1-yl)methyl)morpholino)pyrimidine-5-il)azetidine-3-il)methyl)piperazine-1-il)methyl)piperidine-1-il)phenyl)amino)piperidine-2,6-dione

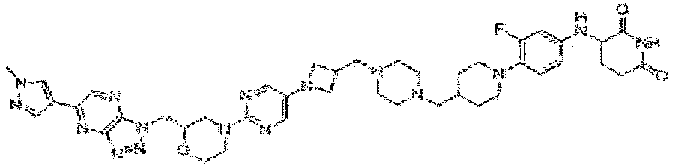

### 5-1) Preparation of t-butyl 4-{[1-(2-fluoro-4-nitrophenyl)piperidine-4-yl]methyl}Preparation of piperazine-1-carboxylate

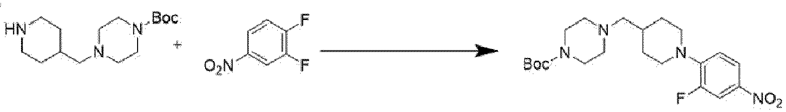

1,2-difluoro-4-nitrobenzene (5.61 g, 35.3 mmol) and potassium carbonate (24.4 g, 176 mmol) were added to a solution of t-butyl 4-[(piperidine-4-yl)methyl]piperazine-1-carboxylate (10 g, 35.3 mmol) in DMF (200 mL). The reactants were stirred at 80 °C for 2 hours. After the reaction was completed, the reactant was poured into water and extracted twice with ethyl acetate. Dried over MgSO₄ and concentrated under reduced pressure. The residues were purified by column chromatography and the title compound was obtained (11 g). MS (ESI, m/z): [M+H]⁺ = 423.5.

### 5-2) t-butyl 4-{[1-(4-amino-2-fluorophenyl)piperidine-4-yl]methyl}Preparation of piperazine-1-carboxylate

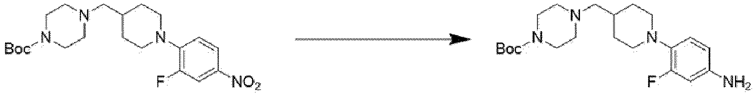

Iron powder (29.5 g, 521 mmol) was added to a solution of t-butyl 4-{[1-(2-fluoro-4-nitrophenyl)piperidine-4-yl]methyl}piperazine-1-carboxylate (11 g, 26 mmol) in saturated NH4Cl/THF (1:1) (200 mL). The reactants were stirred at 80 °C for 2 hours. After the end of the reaction, the reactants were purified and concentrated under reduced pressure, and used in the next process without further purification. MS (ESI, m/z): [M+H]⁺ = 393.5.

### 5-3) Preparation of t-butyl 4-[(1-{4-[(2,6-dioxopiperidin-3-yl)amino]-2-fluorophenyl}piperidine-4-yl)methyl]piperazine-1-carboxylate

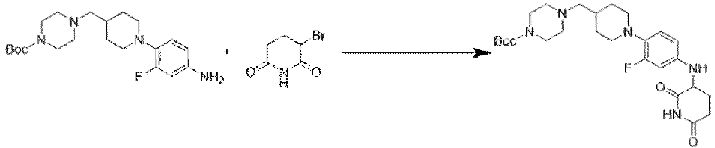

3-bromoperidin-2,6-dione (1.2 g, 6.1 mmol) was added to a solution of t-butyl 4-{[1-(4-amino-2-fluorophenyl)piperidine-4-yl]methyl}piperazine-1-carboxylate (1.2 g, 3.1 mmol) in DMF (25 mL). The reactants were stirred at 85 °C for one night. After the reaction was completed, the reactant was poured into water and extracted twice with ethyl acetate. Dried over MgSO₄ and concentrated under reduced pressure. The residues were purified by column chromatography and the title compound was obtained (1.3 g). MS (ESI, m/z): [M+H]⁺ = 504.6.

### 5-4) Preparation of 3-[(3-fluoro-4-{4-[(piperazine-1-yl)methyl]piperidine-1-yl}phenyl)amino]piperidine-2,6-dione preparation

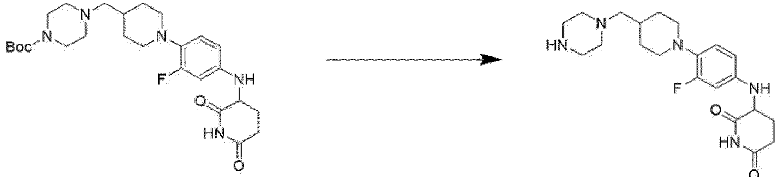

30% TFA was applied to a solution of t-butyl 4-[(1-{4-[(2,6-dioxopiperidine-3-yl)amino]-2-fluorophenyl}piperidine-4-yl)methyl]piperazine-1-carboxylate (650 mg, 1.29 mmol) in DCM. The reactants were stirred at room temperature for 2 hours. After the reaction was completed, the reactants were concentrated under decompression. The residues were purified by DCM/MeOH (5-10%) column chromatography and the title compound was obtained (485 mg). MS (ESI, m/z): [M+H]⁺ = 404.5.

### 5-5) Preparation of 3-((3-fluoro-4-(4-((4-((4-((1-(2-((S)-2-((5-(1-(1-methyl-1H-pyrazole-4-yl)-1H-[1,2,3]triazolo[4,5-b]pyrazine-1-yl)methyl)morpholino)pyrimidine-5-il)azetidine-3-il)methyl)piperazine-1-il)methyl)piperidine-1-il)phenyl)amino)piperidin-2,6-dione

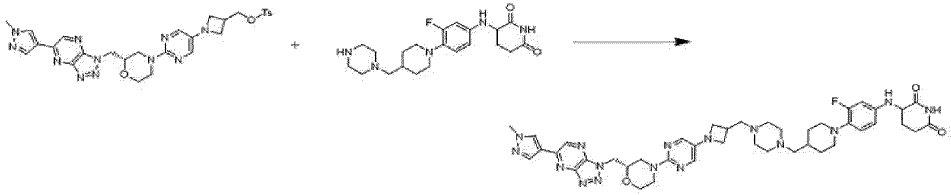

Potassium carbonate (10.1 mg, 0.073 mmol) and sodium iodide (4.73 mg) in ACN (1 ml) in a solution of (1-{2-[(2S)-2-{[5-(1S)-2-{5-{5-(1-methyl-1H-pyrazone-4-yl)-1H-[1,2,3]triazolo[4,5-b]pyrazine-1-yl]methyl}morpholine-4-yl}pyrimidine-4-yl}pyridine-2,6-dione (14.6 mg, 0.03 mmol) in ACN(1 ml). 0.03 mmol). The reactants were stirred at 80 °C for one night. After the reaction was completed, the reactants were concentrated under decompression. The residues were purified by DCM/Me (0~3%) column chromatography and the subject compound was obtained (12 mg). MS (ESI, m/z): [M+H]⁺ = 850.1.
¹H NMR (500 MHz, DMSO-*d*₆) δ 10.71 (s, 1 H) 9.13 (s, 1 H) 8.57 (s, 1H) 8.24 (s, 1 H) 7.75 (s, 2 H) 6.94 - 7.09 (m, 1 H) 6.64 - 6.82 (m, 1 H) 6.18 - 6.42 (m, 1 H) 4.72 - 4.89 (m, 2 H) 4.38 (d, J=11.75 Hz, 1 H) 4.11 (d, J=4.58 Hz, 1 H) 4.03 (d, J=12.97 Hz, 1 H) 3.88 (s, 3 H) 3.77 - 3.85 (m, 3 H) 3.46 - 3.64 (m, 4 H) 3.33 - 3.43 (m, 4 H) 3.01 (d, J=9.77 Hz, 2 H) 2.77 - 2.89 (m, 3 H) 2.57 (br. s., 1 H) 2.51 (br. s., 2 H) 2.25 - 2.38 (m, 6 H) 2.07 - 2.16 (m, 3 H) 1.73 - 1.92 (m, 2 H) 1.66 (d, J=10.68 Hz, 2 H) 1.49 (br, s., 1 H) 1.06 - 1.20 (m, 2 H)

### Example E: Preparation of a Tepotinib derivative-based PROTAC targeting a cMET protein

### Example E-1: Preparation of 3-(1-(3-(5-(((2R)-4-(2-(2-(2,6-dioxopiperidin-3-yl)-4-methyl-1-oxo-1,2-dihydropthalazine-6-yl)morpholine-2-yl)methoxy)pyrimidine-2-yl)benzyl)-6-oxo-1,6-dihydropyridazine-3-yl)benzonitrile

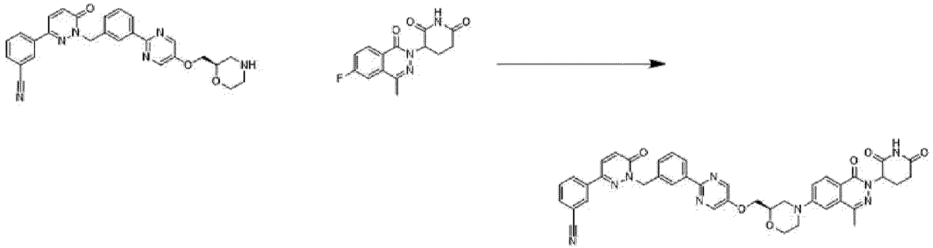

Ethylbis (50 ul, 0.5 mmol) was added to (R)-3-(1-(3-(5-(5-(morpholine-2-ylmethoxy)pyrimidine-2-yl)benzyl)benzonitrile (50 mg, 0.1 mmol) and 3-(6-fluoro-4-methyl-1-oxothalazine-2(1H)-yl)piperidine-2,6-dione (29 mg, 0.1 mmol) in NMP (2 ml). The reactants were stirred at 140 °C for one night. The reactants were poured into water and extracted with ethyl acetate. The organic layer was dried over MgSO₄ and concentrated under reduced pressure. Subsequently, MeOH/DCM (0-20%) column chromatography was purified and the title compound was obtained (48 mg). MS (ESI, m/z): [M+H]⁺ = 750.8.
¹H NMR (500 MHz, DMSO-*d*₆) δ 10.92 (s, 1 H) 8.66 (s, 2 H) 8.32 (d, J=8.55 Hz, 2 H) 8.18 (d, J=6.87 Hz, 2 H) 8.11 (d, J=9.61 Hz, 1 H) 8.05 (d, J=9.00 Hz, 1 H) 7.87 (d, J=7.78 Hz, 1 H) 7.65 (t, J=7.86 Hz, 1 H) 7.48 (dd, J=9.08, 1.75 Hz, 1 H) 7.41 - 7.45 (m, 2H) 7.03 - 7.14 (m, 2 H) 5.58 - 5.68 (m, 1 H) 5.38 (s, 2 H) 4.23 - 4.37 (m, 2 H) 3.92 - 4.08 (m, 3 H) 3.86 (d, J=11.75 Hz, 1H) 3.62 - 3.73 (m, 1 H) 2.76 - 2.97 (m, 3 H) 2.44 - 2.60 (m, 5 H) 1.96 - 2.06 (m, 1 H)

### Example E-2: Preparation of 3-(1-(3-(5-(((2R)-4-(2-(2,6-dioxopiperidin-3-yl)-1-oxo-1,2-dihydrophthalazine-6-yl)morpholine-2-yl)methoxy)pyrimidin-2-yl)benzyl)6-oxo-1,6-dihydropyridazine-3-il)benzonitrile

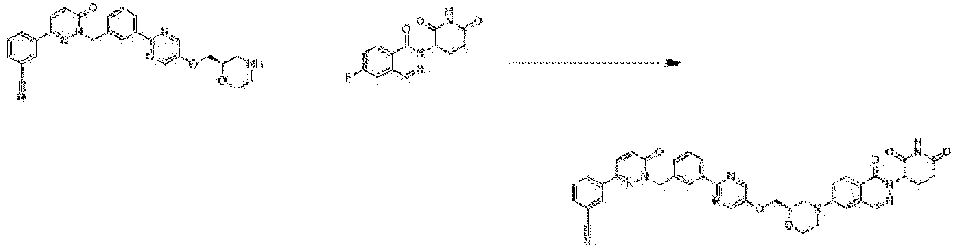

Ethylbis (50 ul, 0.5 mmol) was added to (R)-3-(1-(3-(5-(5-(morpholine-2-yl methoxy-2-yl)benzyl)benzonitrile (50 mg, 0.1 mmol) and 3-(6-fluoro-1-oxopthalazine-2(1H)-yl)piperidine-2,6-dione (27 mg, 0.1 mmol) in NMP (2 ml). The reactants were poured into water and extracted with ethyl acetate. Organic layers were combined, dried with MgSO₄ , and concentrated under reduced pressure. Afterwards, MeOH/DCM (0~20%) column chromatography was purified and the title compound was obtained (35 mg). MS (ESI, m/z): [M+H]⁺ = 736.7.
¹H NMR (500 MHz, DMSO-*d*₆) δ 10.95 (s, 1 H) 8.65 (s, 2 H) 8.32 (d, J=8.09 Hz, 2 H) 8.15 - 8.25 (m, 3 H) 8.11 (d, J=9.77 Hz, 1 H) 8.02 (d, J=9.00 Hz, 1 H) 7.87 (d, J=7.63 Hz, 1 H) 7.65 (t, J=7.86 Hz, 1 H) 7.50 (dd, J=9.16, 1.98 Hz, 1 H) 7.37 - 7.47 (m, 2 H) 7.21 - 7.28 (m, 1 H) 7.10 (d, J=9.77 Hz, 1 H) 5.70 (dd, J=11.98, 5.26 Hz, 1 H) 5.38 (s, 2 H) 4.23 - 4.36 (m, 2 H) 3.91 - 4.05 (m, 3 H) 3.79 (d, J=11.90 Hz, 1 H) 3.61 - 3.72 (m, 1 H) 2.76 - 2.97 (m, 3 H) 2.45 - 2.62 (m, 2 H) 1.99 - 2.07 (m, 1 H)

### Example E-3: Preparation of 3-(1-(3-(5-(((2R)-4-(4-((2,6-dioxopiperidin-3-yl)amino)-2-fluorophenyl)morpholine-2-yl)methoxy)pyrimidine-2-yl)benzyl)6-oxo-1,6-dihydropyridazine-3-il)benzonitrile

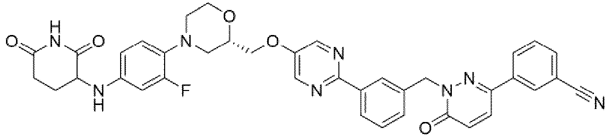

### 3-1) Preparation of (R)-3-(1-(3-(5-((4-(2-fluoro-4-nitrophenyl)morpholine-2-yl)methoxy)pyrimidine-2-yl)benzyl)-6-oxo-1,6-dihydropyridazine-3-yl)benzonitrile preparation

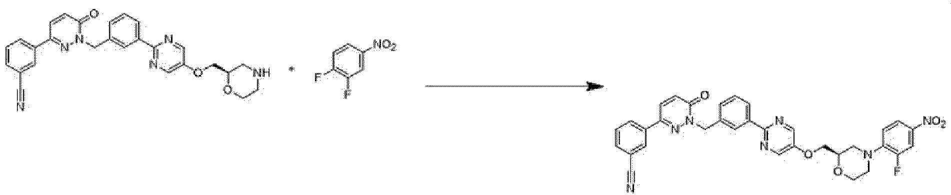

Ethylbis (224 mg, 1.73 mmol) was added to a solution of (R)-3-(1-(3-(5-(5-(morpholine-2-ylmethoxy)pyrimidin-2-yl)benzyl)-6-oxo-1,6-dihydropyridazin-3-yl) benzonitrile (180 mg, 0.38 mmol) and 1,2-difluoro-4-nitrobenzene (55.2 mg, 0.38 mmol) in acetonitrile (2 ml). The reactants were stirred at 100°C for 2 hours. After cooling to room temperature, the reactants were concentrated under reduced pressure, then purified by column chromatography and subjected compounds (110 mg). MS (ESI, m/z): [M+H]⁺ = 620.7.

### 3-2) Preparation of (R)-3-(1-(3-(5-((4-(4-amino-2-fluorophenyl)morpholine-2-yl)methoxy)pyrimidine-2-yl)benzyl)-6-oxo-1,6-dihydropyridazine-3-yl)benzonitrile preparation

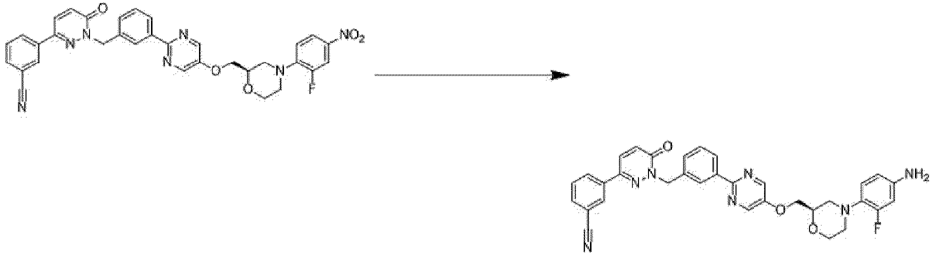

THF (2 ml) was added to 2 ml of NH₄Cl saturated with a solution of (R)-3-(1-(3-(5-((4-(2-(2-fluoro-4-nitrophenyl)morpholine-2-yl)methoxy)pyrimidin-2-yl)benzonitrile (110 mg, 0.18 mmol) and iron (187 mg, 3.34 mmol) solution. The reactants were stirred at 100°C for 2 hours. After cooling to room temperature, the reactants were purified, then poured into water and extracted with ethyl acetate. The organic layer was dried with MgSO₄, concentrated under reduced pressure, and then purified by column chromatography to obtain the heading compound (92 mg). MS (ESI, m/z): [M+H]⁺ = 590.7.

### 3-3) Preparation of 3-(1-(3-(5-(2-(4-(4-((2,6-dioxopiperidin-3-yl)amino)2-fluorophenyl)piperazine-1-yl)ethoxy)pyrimidine-2-yl)benzyl)6-oxo-1,6-dihydropyridazine-3-yl)benzonitrile

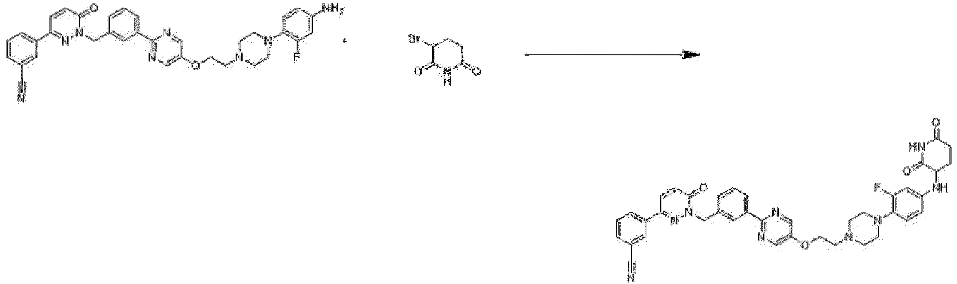

NaHCO₃ (88.1 mg, 0.64 mmol) was added to (R)-3-(1-(3-(5-((4-(4-amino-2-fluorophenyl)morpholine-2-yl)methoxy)pyrimidin-2-yl)benzonitrile-6-oxo-1,6-dihydropyridazine-3-yl) benzonitrile (80 mg, 0.14 mmol) and 3-bromomorpheridine-2,6-dione (73.4 mg, 0.38 mmol) in DMF (1 ml) at room temperature. The reactants were concentrated at 65°C for 16 hours. The reactants were poured into water, extracted with ethyl acetate, dried with MgSO₄, and concentrated under reduced pressure. The residue was purified with MeOH/DCM (0~10%) MPLC and the title compound was obtained (65 mg). MS (ESI, m/z): [M+H]⁺ = 701.7.
¹H NMR (500 MHz, DMSO-*d*₆) δ 10.91 (s, 1 H) 8.61 (s, 2 H) 8.32 (br. s., 2 H) 8.13 - 8.22 (m, 2 H) 8.11 (d, J=9.77 Hz, 1 H) 7.87 (d, J=7.78 Hz, 1 H) 7.65 (t, J=7.86 Hz, 1 H) 7.36 - 7.49 (m, 2 H) 7.10 (d, J=9.77 Hz, 1 H) 6.80 (t, J=9.38 Hz, 1 H) 6.47 (d, J=16.63 Hz, 1 H) 6.38 (d, J=8.55 Hz, 1 H) 5.81 (d, J=7.78 Hz, 1 H) 5.38 (s, 2 H) 4.09 - 4.29 (m, 3 H) 3.93 (br. s., 1 H) 3.86 (d, J=11.14 Hz, 1 H) 3.65 (t, J=10.91 Hz, 1 H) 3.10 (d, J=10.83 Hz, 1 H)} 2.92 (d, J=10.99 Hz, 1 H) 2.55 - 2.73 (m, 3 H) 2.46 - 2.54 (m, 1 H) 1.97 - 2.09 (m, 1 H) 1.73 - 1.94 (m, 1 H)

### Example E-4: Preparation of 3-(1-(3-(5-(2-(4-(2-(2-(2,6-dioxopiperidine-3-yl)-1-oxo-1,2-dihydropetalazine-6-yl)piperazine-1-il)ethoxy)pyrimidine-2-yl)benzyl)6-oxo-1,6-dihydropyridazine-3-yl)benzonitrile

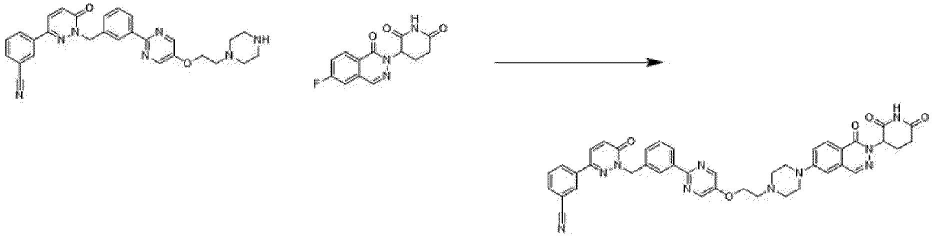

Ethylbis (50 ul, 0.5 mmol) was added to 3-(6-oxo-1-(3-(5-(2-(piperazine-1-yl)ethoxy-pyrimidine-2-yl)benzyl)benzonitrile (50 mg, 0.1 mmol) and 3-(6-fluoro-1-oxopthalazine-2(1H)-yl)piperidine-2,6-dione (27 mg, 0.1 mmol) solutions in NMP (2 ml). The reactants were stirred at 140°C for one night. The reactants were poured into water and extracted with ethyl acetate. The organic layer was dried over MgSO₄ and concentrated under reduced pressure. Afterwards, MeOH/DCM (0~20%) column chromatography was purified and the title compound was obtained (38 mg). MS (ESI, m/z): [M+H]⁺ = 749.8.
¹H NMR (500 MHz, DMSO-*d*₆) δ 10.94 (s, 1 H) 8.62 (s, 2 H) 8.28 - 8.37 (m, 2 H) 8.14 - 8.23 (m, 3 H) 8.11 (d, J=9.77 Hz, 1 H) 7.97 (d, J=9.00 Hz, 1 H) 7.87 (d, J=7.78 Hz, 1 H) 7.65 (t, J=7.86 Hz, 1 H) 7.38 - 7.51 (m, 3 H) 7.20 (d, J=2.14 Hz, 1 H) 7.10 (d, J=9.77 Hz, 1 H) 5.68 (dd, J=12.21, 5.49 Hz, 1 H) 5.38 (s, 2 H) 4.30 (t, J=5.42 Hz, 2 H) 3.36 (br. s., 4 H) 2.80 - 2.92 (m, 1 H) 2.76 (t, J=5.34 Hz, 2 H) 2.60 - 2.66 (m, 3 H) 2.46 - 2.60 (m, 3 H) 1.97 - 2.09 (m, 1 H)

### Example E-5: Preparation of 3-(1-(3-(5-(2-(4-(2-(2-(2-(2-(2,6-dioxopiperidin-3-yl)-4-methyl-1-oxo-1,2-dihydrophthalazine-6-yl)piperazine-1-yl)ethoxy)pyrimidine-2-yl)benzyl)-6-oxo-1,6-dihydropyridazine-3-yl)benzonitrile

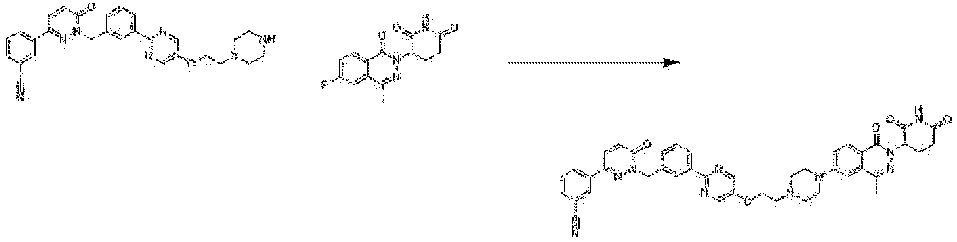

Ethylbis (50 ul, 0.5 mmol) was added to 3-(6-oxo-1-(3-(5-(2-(piperazine-1-yl)ethoxy)pyrimidin-2-yl)benzyl)-1,6-dihydropyridazine-3-yl) benzonitrile (50 mg, 0.1 mmol) and 3-(6-fluoro-4-methyl-1-oxopthalazine-2(1H)-yl)piperidine-2,6-dione (29 mg, 0.1 mmol) solutions in NMP (2 ml). The reactants were stirred at 140°C for one night. The reactants were poured into water and extracted with ethyl acetate. The organic layer was dried over MgSO₄ and concentrated under reduced pressure. Afterwards, MeOH/DCM (0~20%) column chromatography was purified and the title compound was obtained (35 mg). MS (ESI, m/z): [M+H]⁺ = 763.8.
¹H NMR (500 MHz, DMSO-*d*₆) δ 10.91 (s, 1 H) 8.55 - 8.69 (m, 2 H) 8.32 (d, J=5.49 Hz, 2 H) 8.14 - 8.24 (m, 2 H) 8.11 (d, J=9.77 Hz, 1 H) 7.99 (d, J=9.00 Hz, 1 H) 7.87 (d, J=7.63 Hz, 1 H) 7.65 (t, J=7.86 Hz, 1 H) 7.37 - 7.50 (m, 3 H) 7.10 (d, J=9.77 Hz, 1 H) 6.93 - 7.06 (m, 1 H) 5.62 (d, J=11.60 Hz, 1 H) 5.38 (s, 2 H) 4.30 (t, J=5.42 Hz, 2 H) 3.39 (br. s., 4 H) 2.78 - 2.89 (m, 1 H) 2.76 (t, J=5.42 Hz, 2 H) 2.62 (br. s., 6 H) 2.47 - 2.59 (m, 3 H) 1.92 - 2.05 (m, 1 H)

### Example E-6: Preparation of 3-(1-(3-(5-(2-(4-(4-((2,6-dioxopiperidin-3-yl)amino)2-fluorophenyl)piperazine-1-yl)ethoxy)pyrimidine-2-yl)benzyl)6-oxo-1,6-dihydropyridazine-3-yl)benzonitrile

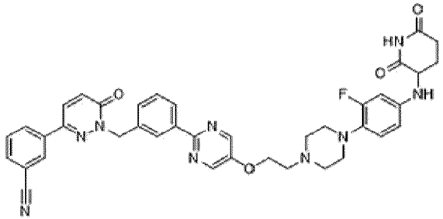

### 6-1) Preparation of 3-(1-(3-(5-(2-(4-(2-(2-(2-(2-(2-nitrophenyl)piperazine-1-yl)ethoxy)pyrimidin-2-yl)benzyl)-6-oxo-1,6-dihydropyridazine-3-yl)benzonitrile manufacturing

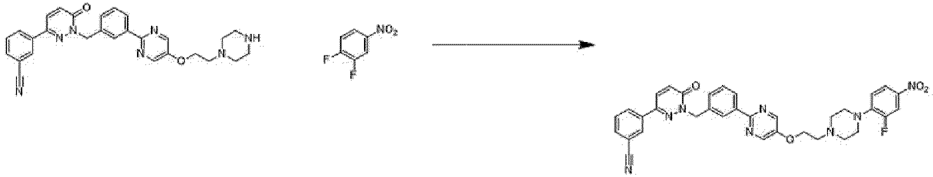

Ethylbis (224 mg, 1.73 mmol) was added to a solution of 3-(6-oxo-1-(3-(5-(2-(piperazine-1-yl)ethoxy)pyrimidin-2-yl)benzyl)-1,6-dihydropyridazine-3-yl)benzonitrile (180 mg, 0.35 mmol) and 1,2-difluoro-4-nitrobenzene (55.2 mg, 0.35 µmol) in acetonitrile (2 ml). The reactants were stirred at 100°C for 2 hours. After cooling to room temperature, it was concentrated under reduced pressure, then purified by column chromatography and subjected compounds (110 mg). MS (ESI, m/z): [M+H]⁺ = 633.7.

### 6-2) Preparation of 3-(1-(3-(5-(2-(4-(4-amino-2-fluorophenyl)piperazine-1-yl)ethoxy)pyrimidine-2-yl)benzyl)-6-oxo-1,6-dihydropyridazine-3-yl)benzonitrile preparation

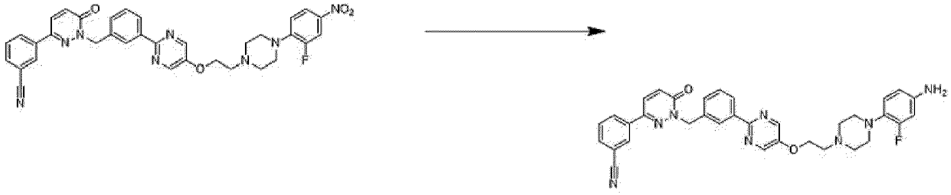

2 ml of NH₄Cl was saturated with a solution of 3-(1-(3-(5-(2-(4-(2-(2-(2-(2-(2-(2-fluoro-4-nitrophenyl)piperazine-1-yl)ethoxy)pyrimidine-2-yl)benzyl)-6-oxo-1,6-dihydropyridazine-3-yl)benzonitrile (110 mg, 0.18 mmol) and iron (187 mg, 3.34 mmol) in THF (2 ml). The reactants were stirred at 100°C for 2 hours. After cooling to room temperature, the reactants were purified, poured into water, and extracted with ethyl acetate. The organic layer was dried with MgSO₄, concentrated under reduced pressure, and then purified by column chromatography to obtain the heading compound (92 mg). MS (ESI, m/z): [M+H]⁺ = 603.7.

### 6-3) Preparation of 3-(1-(3-(5-(2-(4-(4-((2,6-dioxopiperidin-3-yl)amino)2-fluorophenyl)piperazine-1-yl)ethoxy)pyrimidine-2-yl)benzyl)6-oxo-1,6-dihydropyridazine-3-yl)benzonitrile

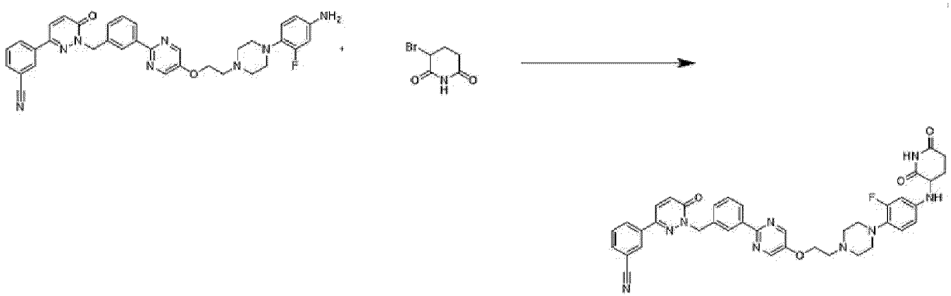

NaHCO₃ (88.1 mg, 0.64 mmol) was added to a solution of 3-(1-(3-(5-(2-(4-(4-amino-2-fluorophenyl)piperazine-1-(4-amino-2-fluorophenyl)piperazine-1-yl)ethoxy)pyrimidine-2-yl)benzonitrile (80 mg, 0.14 mmol) and 3-bromoperidin-2,6-dione (73.4 mg, 0.38 mmol) in 1 ml of DMF at room temperature. The reactants were concentrated at 65°C for 16 hours. The reactants were poured into water, extracted with ethyl acetate, dried with MgSO₄, and concentrated under reduced pressure. The residue was purified with MeOH/DCM (0~10%) MPLC and the title compound was obtained (65 mg). MS (ESI, m/z): [M+H]⁺ = 714.7 ¹H NMR (500 MHz, DMSO-*d*₆) δ 10.73 - 10.84 (m, 1 H) 8.68 (s, 2 H) 8.39 (d, J=4.88 Hz, 2 H) 8.21 - 8.30 (m, 2 H) 8.18 (d, J=9.77 Hz, 1 H) 7.94 (d, J=7.63 Hz, 1H) 7.73 (t, J=7.86 Hz, 1 H) 7.45 - 7.55 (m, 2 H) 7.17 (d, J=9.77 Hz, 1 H) 6.83 (t, J=9.88 Hz, 1 H) 6.48 - 6.59 (m, 1 H) 6.42 (d, J=8.39 Hz, 1 H) 5.83 (d, J=7.48 Hz, 1 H) 5.45 {s, 2 H) 4.80 (d, J=11.14 Hz, 1 H) 4.35 (br. s., 2 H) 4.19 - 4.31 (m, 1 H) 2.88 (br. s., 4 H) 2.67 - 2.79 (m, 2 H) 2.64 (br. s., 2 H) 2.58 (d, J=4.43 Hz, 1 H) 2.02 - 2.15 (m, 1 H) 1.78 - 2.00 (m, 3 H)

### Example E-7: Preparation of 3-(1-(3-(5-(4-((1-(4-(1-((2,6-dioxopiperidin-3-yl)amino)-2-fluorophenyl)azetidin-3-yl)methyl)piperazine-1-il)pyrimidine-2-yl)benzyl)6-oxo-1,6-dihydropyridazine-3-yl)benzonitrile

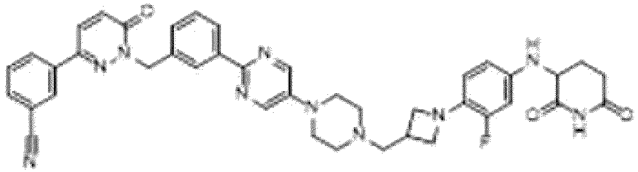

### 7-1) Preparation of t-butyl 3-({4-[2-(3-{[3-(3-cinaophenyl)-6-oxo-1,6-dihydropyridazin-1-yl]methyl}phenyl)pyrimidine-5-yl]piperazine-1-yl}methyl)azetidine-1-carboxylate

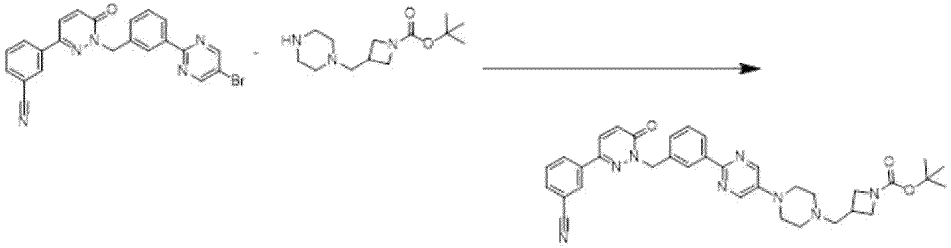

A mixture of 3-(1-{[3-(5-bromoprimidine-2-yl)phenyl]methyl}-6-oxo-1,6-dihydropyridazine-3-yl)benzonitrile (0.3 g, 0.67 mmol), tert-butyl 3-[(piperazine-1-yl)methyl]azetidine-1-carboxylate (0.17 g, 0.67 mmol), Pd₂(dba)₃ (19.4 mg, 33.8 µmol), Xantphos (39.1 mg, 67.5 µmol), and Na-t-butoxide (162 mg, 1.69 mmol) in a microwave sub-microwave 100°C for 2 hours. After the reaction was completed, the reaction mixture was purified and concentrated under reduced pressure. The residue was purified with EA/Hex (50~100%) MPLC and the title compound was obtained (310 mg). MS (ESI, m/z): [M+H]⁺ = [619.7].

### 7-2) Preparation of 3-(1-{[3-(5-{4-[(azetidin-3-yl)methyl]piperazine-1-il}pyrimidine-2-yl)phenyl]methyl}-6-oxo-1,6-dihydropyridazine-3-yl)benzonitrile

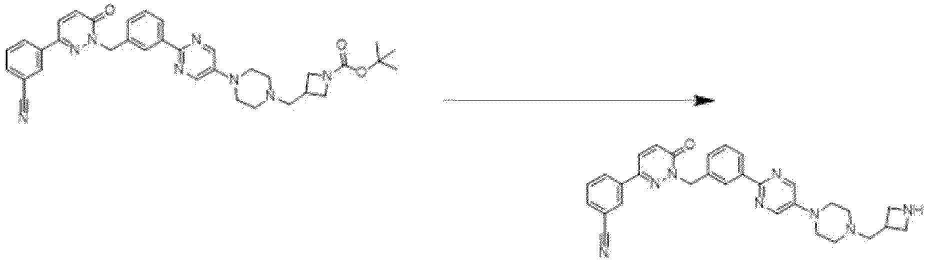

30% TFA was added to a solution of t-butyl 4-(2-{[2-(3-{[3-(3-cyanophenyl)-6-oxo-1,6-dihydropyridazine-1-yl]methyl}phenyl)pyrimidine-5-yl]oxy}ethyl)piperazine-1-carboxylate (310 mg, 0.50 mmol) in DCM. The reactants were stirred at room temperature for 1 hour. After the reaction was completed, the reactants were concentrated under decompression. The residue was purified by DCM/MeOH (0~5%) amine column chromatography and the title compound was obtained (270 mg). MS (ESI, m/z): [M+H]⁺ = [519.6].

### 7-3) Preparation of 3-[1-({3-[5-(4-{[1-(2-fluoro-4-nitrophenyl)azetidine-3-yl]methyl}piperazine-1-yl)pyrimidine-2-yl]phenyl}methyl)6-oxo-1,6-dihydropyridazine-3-yl]benzonitrile preparation

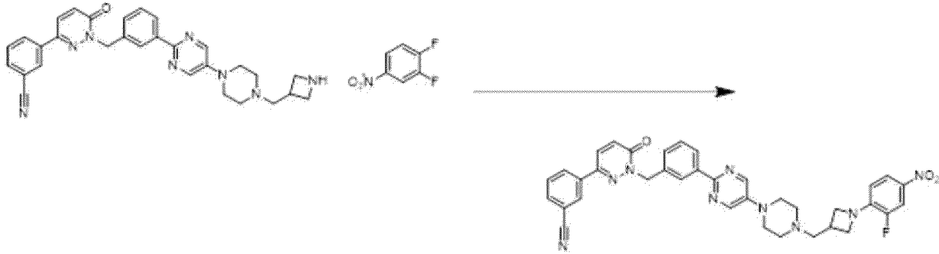

Ethylbis (224 mg, 1.73 mmol) was added to a solution of acetonitrile (2 ml) in a solution of 3-(1-{3-(5-{4-[(azetidine-3-yl)methyl]piperazine-1-yl}pyrimidin-2-yl)phenyl}methyl}-6-oxo-1,6-dihydropyridazin-3-yl) benzonitrile (180 mg, 347 µmol) and 1,2-difluoro-4-nitrobenzene (55.2 mg, 347 µmol). The reactants were stirred at 100°C for 2 hours. After cooling to room temperature, the reactants were concentrated under reduced pressure. Purified by column chromatography, the title compound was obtained (110 mg). MS (ESI, m/z): [M+H]⁺ = [658.7].

### 7-4) Preparation of 3-[1-({3-[5-(4-{[1-(4-amino-2-fluorophenyl)azetidine-3-yl]methyl}piperazine-1-yl)pyrimidine-2-yl]phenyl}methyl)-6-oxo-1,6-dihydropyridazine-3-yl]benzonitrile preparation

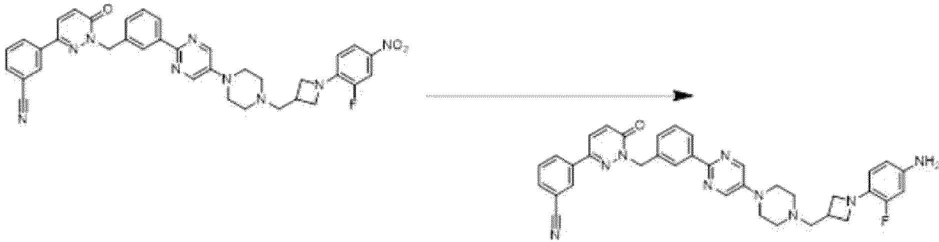

THF (2 ml) of 3-[1-({3-[5-(4-{[1-(2-fluoro-4-nitrophenyl)azetidine-3-yl]methyl}piperazine-1-yl)pyrimidine-2-yl]phenyl}methyl)6-oxo-1,6-dihydropyridazine-3-yl]benzonitrile (110 mg, 167 µmol) and iron (187 mg, 3.34 mmol) solutions were saturated with NH4Cl. The reactants were stirred at 100°C for 2 hours. After cooling to room temperature, the reactants were purified, the purified water was poured into water, and extracted with ethyl acetate. The organic layer was dried with MgSO4, concentrated under reduced pressure, and the residue was purified by column chromatography and the title compound was obtained (92 mg). MS (ESI, m/z): [M+H]⁺ = [628.7].

### 7-5) Preparation of 3-(1-(3-(5-(4-((1-(4-(4-((2,6-dioxopiperidin-3-yl)amino)2-fluorophenyl)azetidin-3-yl)methyl)piperazine-1-il)pyrimidine-2-yl)benzyl)6-oxo-1,6-dihydropyridazine-3-yl)benzonitrile

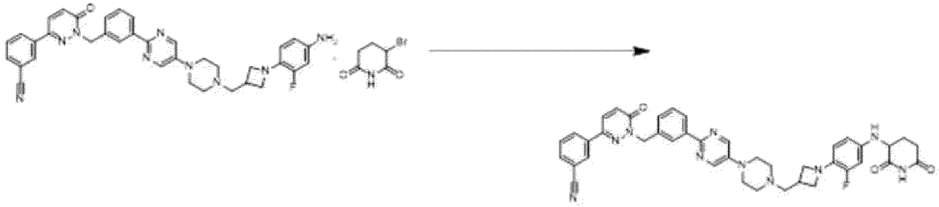

NaHCO 3 (88.1 mg, 637 umol) was added to a solution of 3-[1-({3-[5-(4-{[1-(4-amino-2-fluorophenyl)azetidine-3-yl]methyl}piperazine-1-yl]phenyl}methyl)-6-oxo-1,6-dihydropyridazin-3-yl]benzonitrile (80 mg, 127 umol) and 3-bromoperidin-2,6-dione (73.4 mg, 382 umol) in 1 ml of DMF at room temperature. The reactants were stirred at 65°C for 16 hours. The reactants were poured into water, extracted with ethyl acetate, dried with MgSO₄, and concentrated under reduced pressure. The residue was purified with MeOH/DCM (0~10%) MPLC and the title compound was obtained (65 mg). MS (ESI, m/z): [M+H]⁺ = 739.8.
¹H NMR (500 MHz, DMSO-*d*₆) δ 10.70 (s, 1 H) 8.47 - 8.56 (m, 2 H) 8.31 (s, 1 H) 8.28 (s, 1 H) 8.18 (d, J=8.09 Hz, 1 H) 8.06 - 8.15 (m, 2 H) 7.87 (d, J=7.63 Hz, 1 H) 7.65 (t, J=7.86 Hz, 1 H) 7.31 - 7.44 (m, 2 H) 7.10 (d, J=9.77 Hz, 1 H) 6.42 (d, J=15.41 Hz, 1 H) 6.24 - 6.38 (m, 1 H) 5.48 (d, J=7.48 Hz, 1 H) 5.37 (s, 2 H) 4.06 - 4.19 (m, 1 H) 3.80 (t, J=6.94 Hz, 2 H) 3.34 (t, J=6.18 Hz, 5 H) 3.24 (br. s., 4 H) 2.81 (dt, J=13.66, 6.90 Hz, 1 H) 2.65 (ddd, J=17.51, 12.02, 5.26 Hz, 1 H) 2.48 - 2.59 (m, 6 H) 1.95 - 2.07 (m, 1 H)

### Example E-8: Preparation of 3-(1-(3-(5-(4-((1-(4-((1-(4-((2,6-dioxopiperidin-3-yl)amino)-2-fluorophenyl)piperidine-4-yl)methyl)piperazine-1-il)pyrimidine-2-yl)benzyl)6-oxo-1,6-dihydropyridazine-3-yl)benzonitrile

### 8-1) Preparation of 3-(6-oxo-1-(3-(5-(4-(piperidine-4-ylmethyl)piperazine-1-yl)pyrimidine-2-yl)benzyl)-1,6-dihydropyridazine-3-yl)benzonitrile

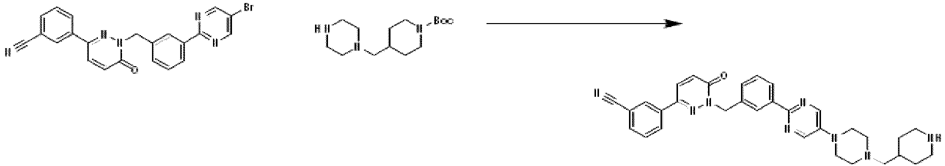

Tris (1,5-diphenylpenta-1,4-diene-3-one) dipalladium (144 mg, 158 µmol) and [5-(diphenylphosphony)9,9-dimethyl-9H-xanthenene-4-yl]diphenylphospan (91.2 mg, 158 µmol) were added to a solution of 3-(1,5-diphenylphosphony)-9,9-dimethyl-9H-xantene-4-yl]diphenylphospan (91.2 mg, 158 µmol) in a solution of toluene (10 ml), followed by K2CO3 (528 mg, 3.94 mmol). The reactants were stirred at 110°C for 5 hours. After cooling to room temperature, the reactants were concentrated under reduced pressure and purified by column chromatography to obtain BOC-protected compounds. The compound was treated with 4.0 M HCl in dioxane (5 mL) and stirred for 5 hours. The reactants were concentrated under reduced pressure, passed through a basic silica pad, and the subject compound was obtained (521 mg). MS (ESI, m/z): [M+H]⁺ = 547.3.

### 8-2) Preparation of 3-(1-(3-(5-(4-((1-(2-fluoro-4-nitrophenyl)piperidine-4-yl)methyl)piperazine-1-il)pyrimidine-2-yl)benzyl)6-oxo-1,6-dihydropyridazine-3-yl)benzonitrile

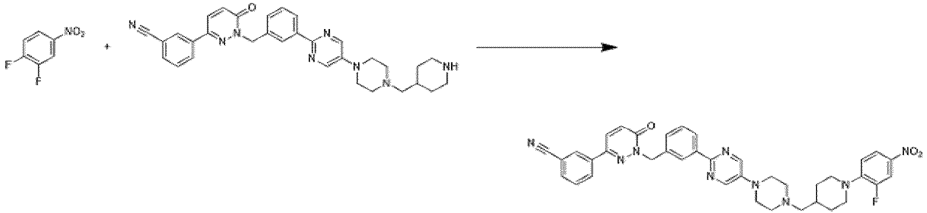

Ethylbis (propane-2-yl)amine (47.3 mg, 366 µmol) was added to a solution of 3-(6-oxo-1-{{5-{4-[(piperidine-4-yl)methyl]piperazine-1-yl}pyrimidine-2-yl)phenyl]methyl}-1,6-dihydropyridazin-3-yl)benzonitrile (100 mg, 183 µmol) and 1,2-difluoro-4-nitrobenzene (29.1 mg, 1839 µmol) in acetonitrile (2 ml). Ethylbis (propane-2-yl)amine (47.3 mg, 366 µmol) was added to a solution of 3-(6-oxo-1-{5-{4-[(piperidine-4-yl)methyl]piperazine-1-yl}pyridine-2-yl}pyridine-2-yl}, after cooling to room temperature, and purified by column chromatography to obtain the heading compound (110 mg). MS (ESI, m/z): [M+H]⁺ = 686.7.

### 8-3) Preparation of 3-(1-(3-(5-(4-((1-(4-(4-(4-amino-2-fluorophenyl)piperidine-4-yl)methyl)piperazine-1-il)pyrimidine-2-yl)benzyl)6-oxo-1,6-dihydropyridazine-3-yl)benzonitrile

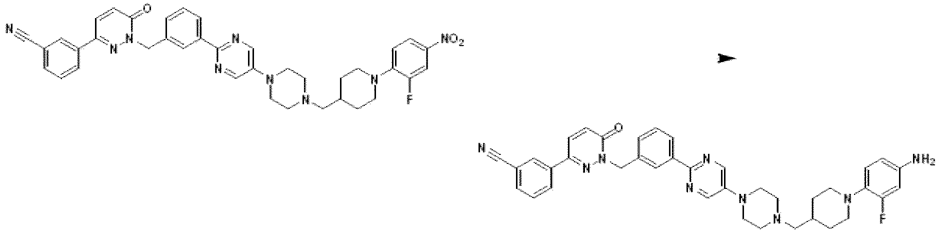

2 mL of saturated NH₄Cl was added to a solution of 3-(1-(3-(5-(4-((1-(2-fluoro-4-nitrophenyl)piperidine-4-yl)methyl)piperazine-1-il)pyrimidine-2-yl)benzyl)-6-oxo-1,6-dihydropyridazine-3-yl) benzonitrile (100 mg, 146 µmol) and iron (40.7 mg, 729 µmol) in a solution. The reactants were stirred at 100°C for 2 hours. After cooling to room temperature, the reactants were filtered, the filtrate was poured into water, and extracted with ethyl acetate. The organic layer was dried with MgSO4, concentrated under reduced pressure, purified by column chromatography, the title compound was obtained (79 mg). MS (ESI, m/z): [M+H]⁺ = 656.7.

### 8-4) Preparation of 3-(1-(3-(5-(4-((1-(4-(1-((2,6-dioxopiperidine-3-yl)amino)2-fluorophenyl)piperidine-4-yl)methyl)piperazine-1-yl)pyrimidine-2-yl)benzyl)-6-oxo-1,6-dihydropyridazine-3-yl)benzonitrile

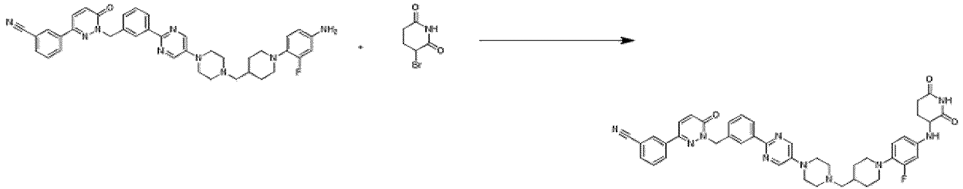

Ethylbis (propane-2-yl)amine (47.3 mg, 366 µmol) was added to 3-[1-({3-[5-(4-{[1-(2-fluoro-4-nitrophenyl)piperidine-4-yl]methyl}piperazine-1-yl)pyrimidin-2-yl]phenyl}methyl)-6-oxo-1,6-dihydropyridazine-3-yl]benzonitrile (100 mg, 146 µmol) and 3-bromomorphidine-2,6-dione solution in acetonitrile (2 ml). The reactants were stirred at 100°C for 2 hours. After cooling to room temperature, the reactants were concentrated under reduced pressure, purified by column chromatography, and the title compound (110 mg) was obtained. MS (ESI, m/z): [M+H]⁺ = 767.9.

1H NMR (500 MHz, DMSO-d6) δ ppm 10.72 (s, 1 H) 8.52 (s, 3 H) 8.32 (s, 2 H) 8.28 (s, 1 H) 8.18 (d, J=8.09 Hz, 1 H) 8.09 - 8.14 (m, 3 H) 7.87 (d, J=7.78 Hz, 1 H) 7.65 (t, J=7.93 Hz, 1 H) 7.36 - 7.40 (m, 2 H) 7.10 (d, J=9.61 Hz, 1 H) 6.76 (t, J=9.31 Hz, 1 H) 6.40 - 6.48 (m, 1 H) 6.35 (d, J=8.70 Hz, 1 H) 5.72 (d, J=7.78 Hz, 1 H) 5.37 (s, 3 H) 4.15 - 4.23 (m, 1 H) 2.99 - 3.09 (m, 2 H) 2.58 - 2.73 (m, 1 H) 2.48 - 2.53 (m, 3 H) 2.17 (br. s., 1 H) 1.96 - 2.06 (m, 1 H) 1.82 - 1.92 (m, 1 H) 1.68 - 1.79 (m, 3 H) 1.15 - 1.25 (m, 3 H) 1.11 (t, J=7.17 Hz, 1 H)

### Example 1: Cell line culture and preparation of compounds

The c-MET overexpressing human gastric cancer cell lines MKN45 and SNU638 were cultured in RPMI medium with 10% fetal bovine serum, penicillin (100 U/ml), and streptomycin (100 mg/ml), and the c-MET exon 14 skipping mutant human gastric cancer cell line Hs746T was cultured in DMEM media. The c-MET overexpressing human lung cancer cell line EBC-1 was cultured in MEM media with the addition of 10% fetal bovine serum, penicillin (100U/ml), and streptomycin (100mg/ml). The normal cell line WI-38 derived from human lung tissue was cultured in EMEM medium with the addition of 10% fetal bovine serum, penicillin (100U/ml), and streptomycin (100mg/ml).

The compound synthesized in accordance with the present invention was prepared with a raw material solution of 10 mM dissolved in DMSO (dimethyl sulfoxide), and was sequentially diluted and processed.

### Example 2: Evaluation of cancer cell anti proliferative activity of ligand compounds targeting cMET

In order to evaluate the inhibitory activity on cell growth, cells were dispensed in a 96-well plate at a concentration of 5,000 cells/well and then treated with the compound of the present invention prepared at various concentrations for 72 hours. The absorbance measurement at 450 nm was calculated using the GraphicPad Prism 8.0 software. The IC₅₀ value (concentration of the compound achieving 50% cytostatic inhibition) is the average value of the measured results. The results are shown in Tables 1 and 2. As shown in the experimental results, the compounds of the present invention showed the effect of inhibiting the proliferation of cancer cells, and in particular, the embodiment of effective degradation of c-MET is as follows.

**[Table 1]**

| IC₅₀ value of cMET target ligand compounds based on ABN derivatives | | | |
|---|---|---|---|
| | Structure | In vitro cell viability assay (IC₅₀, nM) | |
| | | SNU-638 | EBC-1 |
| ABN401 | 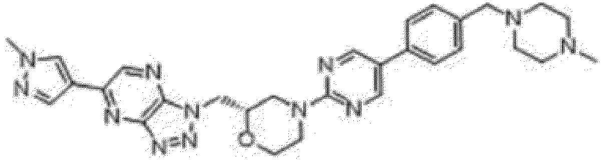 | A | A |
| Example B-1 | 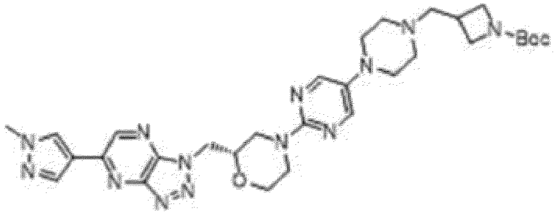 | A | A |
| Example B-2 | 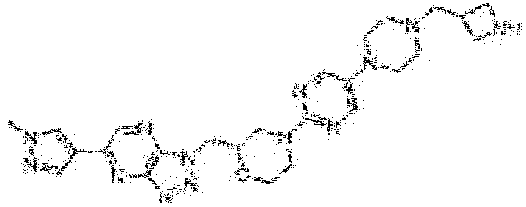 | A | A |
| Example B-3 | 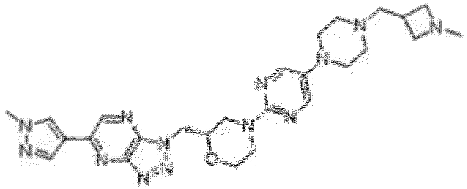 | A | A |
| Example B-4 | 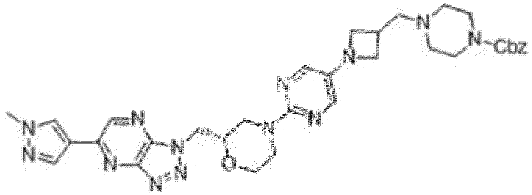 | A | A |
| Example B-5 | 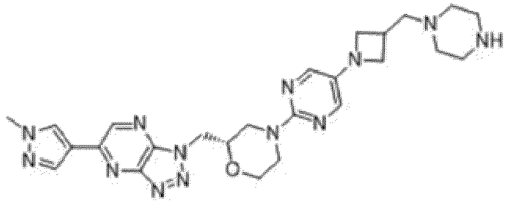 | A | A |
| Example B-6 | 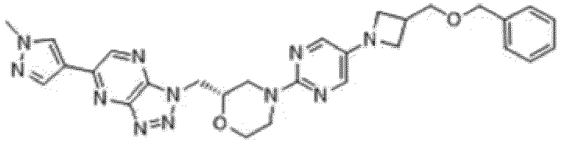 | A | A |
| Example B-7 | 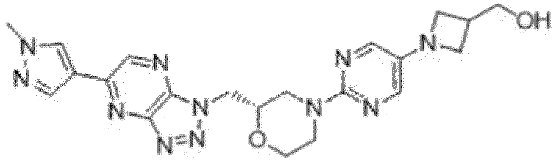 | A | A |
| Example B-8 | 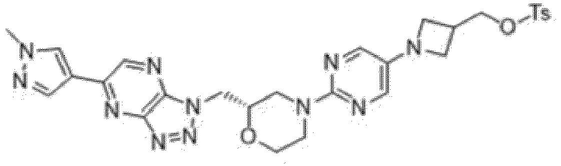 | A | A |
| Example B-9 | 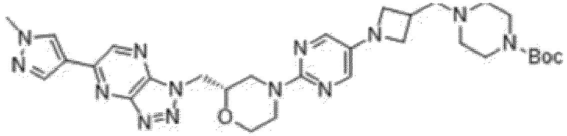 | A | A |
| Example B-10 | 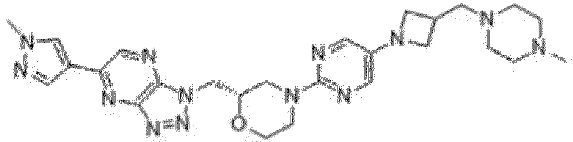 | A | A |
| Example B-11 | 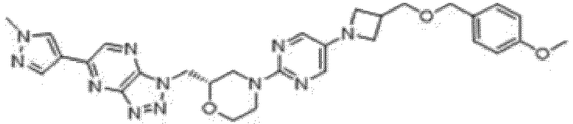 | A | A |
| Example B-12 | 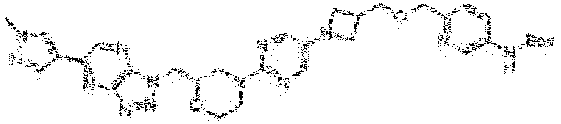 | B | B |
| Example B-13 | 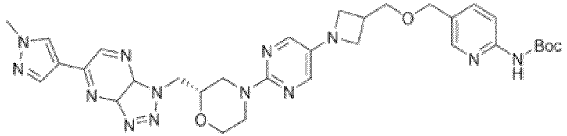 | B | B |

| | | | |
|---|---|---|---|
| A: IC₅₀ ≤ 100 nM, B: IC₅₀ ≤ 1 uM, C: IC₅₀ > 1 uM | | | |

**[Table 2]**

| IC 50 value of cMET target ligand compounds based on Tepotinib derivative | | | |
|---|---|---|---|
| | Structure | In vitro cell viability assay (IC₅₀, nM) | |
| | | SNU-638 | EBC-1 |
| Example C-1 | 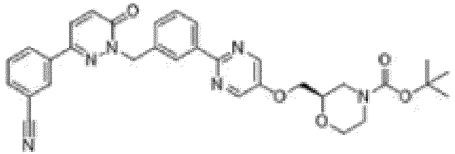 | A | A |
| Example C-2 | 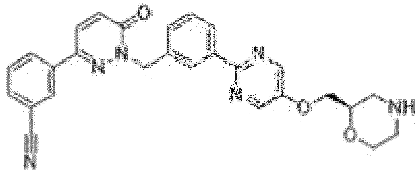 | A | A |
| Example C-3 | 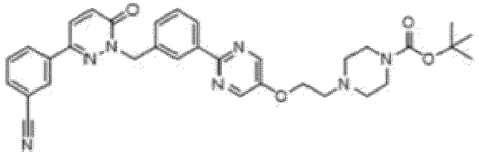 | A | A |
| Example C-4 | 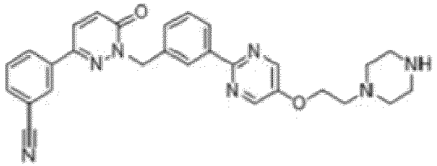 | A | A |
| Example C-5 | 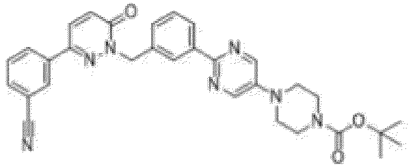 | A | A |
| Example C-6 | 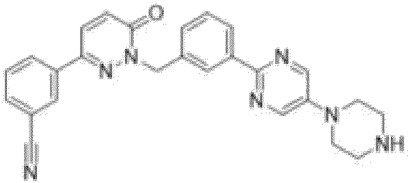 | A | A |

| | | | |
|---|---|---|---|
| A: IC₅₀ ≤ 100 nM, B: IC₅₀ ≤ 1 uM, C: IC₅₀ > 1 uM | | | |

### Example 3: Evaluation of c-MET protein degradation efficacy of PROTAC targeting cMET protein

To evaluate the degradation efficacy of the compounds of the present invention for c-MET expressed in cells, cells were dispensed in a 12-well plate at a concentration of 2 × 105 cells/well, and then gastric cancer cell lines were treated with samples for 24 hours and lung cancer cell lines were treated for 48 hours.

To perform a western blotting assay to confirm protein expression, cells treated with a compound treated with 40 µL of RIPA buffer with a protease inhibitor for 30 minutes, collected cell lysate using a scraper on ice, and centrifuged at 4°C 15,000 rpm for 30 min to obtain a protein-containing supernatant.

Protein expression was then measured using the Simple Western Automated Western Blot System Abby instrument using 3 µg of protein for each sample. SDS and heat-induced denaturation proteins were separated by molecular weight, attached to the capillary, reacted with anti-c-MET or anti-GAPDH antibodies, which are primary antibodies, and then reacted with HRP-conjugated secondary antibodies, and analyzed the protein expression signal with compass software.

Protein expression was normalized based on the expression value of GAPDH, and the protein expression amount in each sample was measured as a percentage (%) based on 100% of samples treated with 0.1% DMSO carrier, and DC₅₀ (concentration of a compound where 50% protein inhibition is achieved) was calculated from the change in protein expression amount due to compound treatment.

The results are shown in Tables 3 and 4.

**[Table 3]**

| IC₅₀ and DC₅₀ values of PROTAC targeting ABN derivative-based cMET proteins | | | | | | | |
|---|---|---|---|---|---|---|---|
| | Structure | In vitro cell viability assay (nM) | | | | | |
| | | SNU-638 | | EBC-1 | | Hs746T | |
| | | DC₅₀ | IC₅₀ | DC₅₀ | IC₅₀ | DC₅₀ | IC₅₀ |
| Example D-1 | 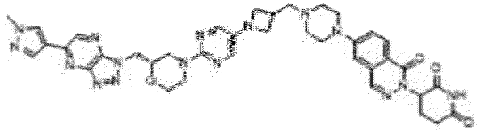 | C | A | A | A | A | A |
| Example D-2 | 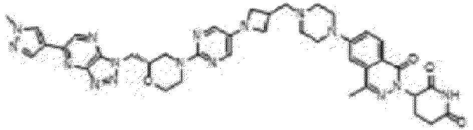 | C | A | A | A | A | A |
| Example D-3 | 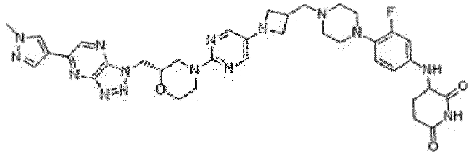 | A | A | A | A | A | A |
| Example D-4 | 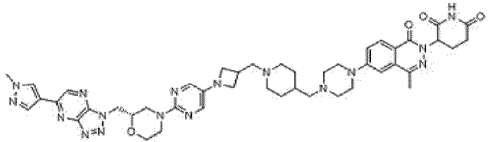 | B | A | A | A | A | A |
| Example D-5 | 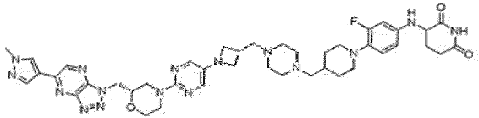 | C | A | C | A | C | C |

| | | | | | | | |
|---|---|---|---|---|---|---|---|
| A: DC₅₀ or IC₅₀ ≤ 100 nM, B: DC₅₀ or IC₅₀ ≤ 1 uM, C: DC₅₀ or IC₅₀ > 1 uM | | | | | | | |

**[Table 4]**

| IC₅₀ and DC₅₀ values of Tepotinib derivative-based PROTAC targeting cMET proteins | | | | | | | |
|---|---|---|---|---|---|---|---|
| | Structure | In vitro cell viability assay (nM) | | | | | |
| | | SNU-638 | | EBC-1 | | Hs746T | |
| | | DC₅₀ | IC₅₀ | DC₅₀ | IC₅₀ | DC₅₀ | IC₅₀ |
| Example E-1 | 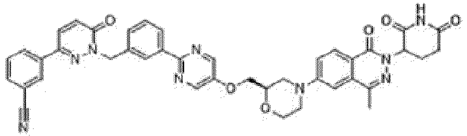 | C | A | A | A | A | A |
| Example E-2 | 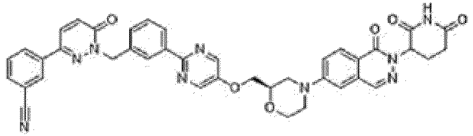 | - | - | A | A | A | A |
| Example E-3 | 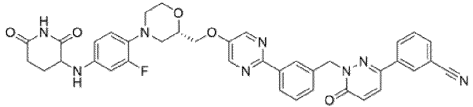 | C | A | C | A | - | - |
| Example E-4 | 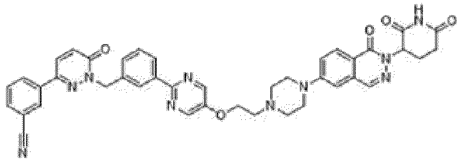 | C | A | A | A | A | A |
| Example E-5 | 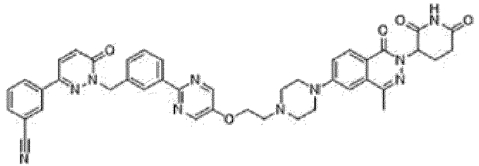 | A | A | A | A | A | A |
| Example E-6 | 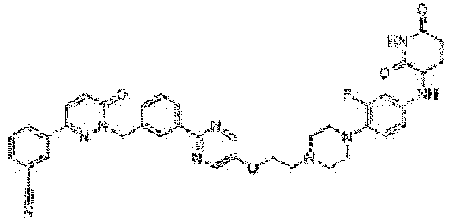 | C | A | C | A | - | - |
| Example E-7 | 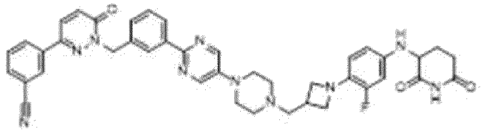 | A | A | A | A | - | - |
| Example E-8 | 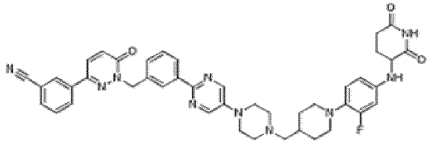 | C | A | A | A | A | A |

| | | | | | | | |
|---|---|---|---|---|---|---|---|
| A: DC₅₀ or IC₅₀ ≤ 100 nM, B: DC₅₀ or IC₅₀ ≤ 1 uM, C: DC₅₀ or IC₅₀ > 1 uM | | | | | | | |

### [Industrial Applicability]

The PROTAC compound according to the present invention can be used as an anticancer drug.

## Claims

1. A compound represented by the following Chemical Formula 1:
[Chemical Formula 1] cMET Targeted Protein-Binding Moiety (P)-{Linker (L)}p-E3 Ligase-Binding Moiety (E)
wherein,
E3 Ligase-Binding Moiety (E) is a compound represented by the following Chemical Formula 2; and
cMET Targeted Protein-Binding Moiety (P) is a compound represented with any of the following formulas 3 to 7; and
p is an integer of 0 or 1: 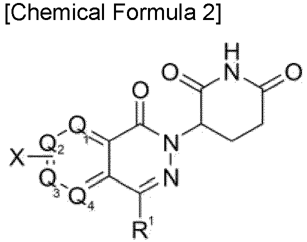 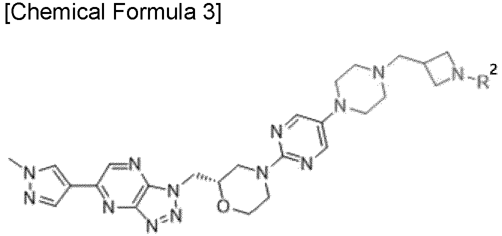 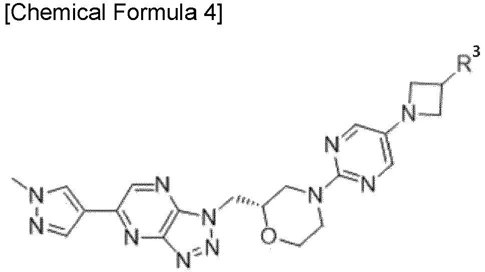 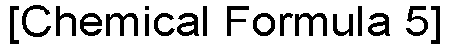 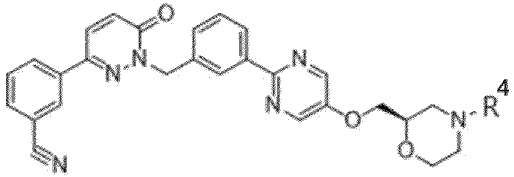 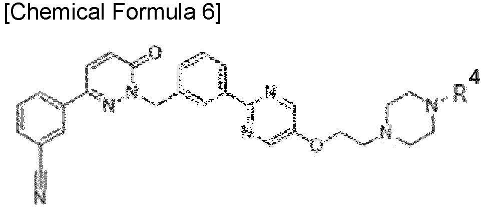 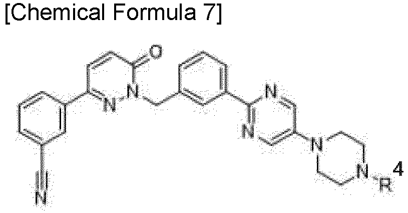 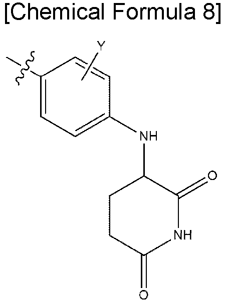
wherein,
X is hydrogen, halogen, amino, nitro, hydroxy, C1 to C6 straight or branched alkyl, or a 4 to 8 atom heterocyclic containing oxygen or nitrogen;
Q₁ to Q₄ are each independently C-F, C-Cl, C-H, C-X, or N, and at least any one of Q₁ to Q₄ is C-X;
Y is hydrogen, halogen, or a C1 to C6 a straight, branched or cyclic alkyloxy unsubstituted or substituted with halogen;
R¹ is hydrogen, nitro, amino, carbonyl, C1 to C6 straight, branched or cyclic alkyl, or C1 to C6 straight, branched, or cyclic alkyl substituted with halogen; 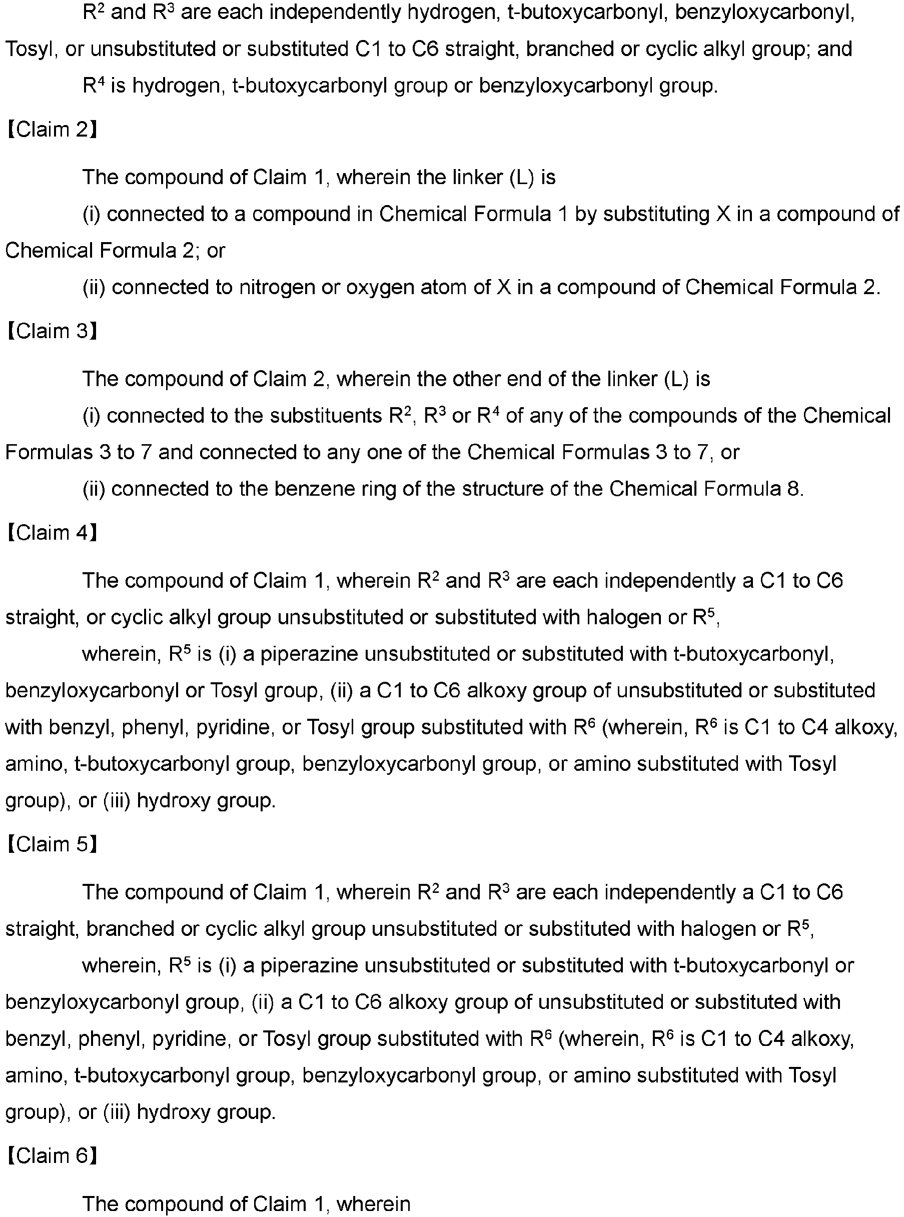
R² is hydrogen, or a C1 to C6 straight or branched alkyl chain or cyclic alkyl group,
R³ is a straight or branched, or cyclic alkyl group of C1 to C4 unsubstituted or substituted with R⁵,
wherein, R⁵ may be (i) a piperazine, (ii) an C1 to C4 alkoxy group unsubstituted or substituted with benzyl, phenyl, or pyridine unsubstituted or substituted with R⁶ (wherein, R⁶ is an C1 to C4 alkoxy group or amino group of), or (iii) a hydroxy group.

2. The compound of Claim 1, wherein
the linker (L) is
-(CH₂)ₘ-R⁷-(CH₂)ₙ- 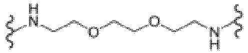 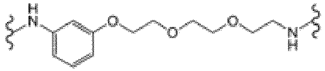 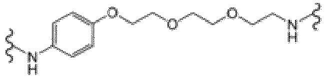 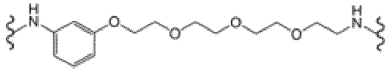 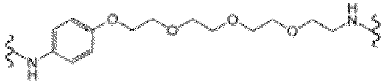
or 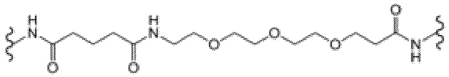
wherein, R⁷ is piperazine, piperidine, azetidine, benzene, triazole or pyrrolidine; and
m and n are each independently an integer selected from 0 to 5.

3. The compound of Claim 1, wherein
the linker (L) is 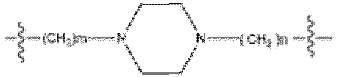 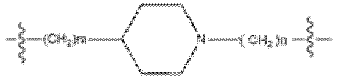 or 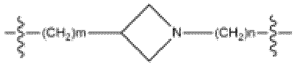
wherein, m and n are each independently an integer selected from 0 to 5.

4. A compound selected from the group consisting of the following compounds or a pharmaceutically acceptable salt thereof:
3-(6-(4-((1-(2-((S)-2-((5-(1-methyl-1H-pyrazole-4-yl)-1H-[1,2,3]triazolo[4,5-b]pyrazine-1-yl)methyl)morpholino)pyrimidine-5-il)azetidin-3-il)methyl)piperazine-1-yl)1-oxopthalazine-2(1H)-yl)piperidine-2,6-dione;
3-(4-methyl-6-(4-((1-(2-((S)-2-((5-(1-methyl-1H-pyrazole-4-yl)-1H-[1,2,3]triazolo[4,5-b]pyrazine-1-yl)methyl)morpholino)pyrimidine-5-il)azetidine-3-yl)methyl)piperazine-1-yl) 1-oxopthalazine-2(1H)-yl)piperidine-2,6-dione;
3-((3-fluoro-4-(4-((1-(2-((S)-2-((5-(1-methyl-1H-pyrazole-4-yl)-1H-[1,2,3]triazolo[4,5-b]pyrazine-1-yl)methyl)morpholino)pyrimidine-5-il)azetidin-3-il)methyl)piperazine-1-il)phenyl)amino)piperidine-2,6-dione;
3-(4-methyl-6-(4-((1-((1-(1-(2-((S)-2-((5-(1-methyl-1H-pyrazole-4-yl)-1H-[1,2,3]triazolo[4,5-b]pyrazin-1-yl)methyl)morpholino)pyrimidine-5-il)azetidin-3-il)methyl)piperidine-4-il)methyl)piperazine-1-day)-1-oxopthalazine-2(1H)-yl)piperidine-2,6-dione;
3-((3-fluoro-4-(4-((4-((1-(2-((S)-2-((5-(1-(1H-pyrazole-4-yl)-1H-[1,2,3]triazolo[4,5-b]pyrazine-1-yl)methyl)morpholino)pyrimidine-5-il)azetidine-3-il)methyl)piperazine-1-il)methyl)piperidine-1-il)phenyl)amino)piperidine-2,6-dione;
3-(1-(3-(5-(((2R)-4-(2-(2-(2,6-dioxopiperidine-3-yl)-4-methyl-1-oxo-1,2-dihydropthalazine-6-yl)morpholine-2-yl)methoxy)pyrimidine-2-yl)benzyl)-6-oxo-1,6-dihydropyridazine-3-yl)benzonitrile;
3-(1-(3-(5-(((2R)-4-(2-(2-(2,6-dioxopiperidin-3-yl)-1-oxo-1,2-dihydroptalazine-6-yl)morpholine-2-yl)methoxy)pyrimidine-2-yl)benzyl)6-oxo-1,6-dihydropyridazine-3-il)benzonitrile;
3-(1-(3-(5-(((2R)-4-(4-((2,6-dioxopiperidin-3-yl)amino)-2-fluorophenyl)morpholine-2-yl)methoxy)pyrimidine-2-yl)benzyl)6-oxo-1,6-dihydropyridazine-3-yl)benzonitrile;
3-(1-(3-(5-(2-(4-(2-(2,6-dioxopiperidine-3-yl)-1-oxo-1,2-dihydroptalazine-6-yl)piperazine-1-yl)ethoxy)pyrimidine-2-yl)benzyl)6-oxo-1,6-dihydropyridazine-3-yl)benzonitrile;
3-(1-(3-(5-(2-(4-(2-(2-(2,6-dioxopiperidine-3-yl)-4-methyl-1-oxo-1,2-dihydroptalazine-6-yl)piperazine-1-yl)ethoxy)pyrimidine-2-yl)benzyl)6-oxo-1,6-dihydropyridazine-3-yl)benzonitrile;
3-(1-(3-(5-(2-(4-((2,6-dioxopiperidin-3-yl)amino)-2-fluorophenyl)piperazine-1-yl)ethoxy)pyrimidine-2-yl)benzyl)6-oxo-1,6-dihydropyridazine-3-yl)benzonitrile;
3-(1-(3-(5-(4-((1-(4-((2,6-dioxopiperidine-3-yl)amino)2-fluorophenyl)azetidine-3-yl)methyl)piperazine-1-yl)pyrimidine-2-yl)benzyl)-6-oxo-1,6-dihydropyridazine-3-yl)benzonitrile; and
3-(1-(3-(5-(4-((1-(4-((2,6-dioxopiperidine-3-yl)amino)2-fluorophenyl)piperidine-4-yl)methyl)piperazine-1-il)pyrimidine-2-yl)benzyl)-6-oxo-1,6-dihydropyridazine-3-yl)benzonitrile.

5. A compound represented by Chemical Formulas 3 or 4 or a pharmaceutically acceptable salt thereof: 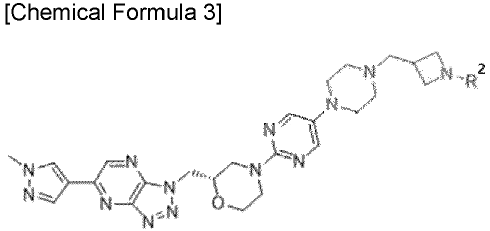 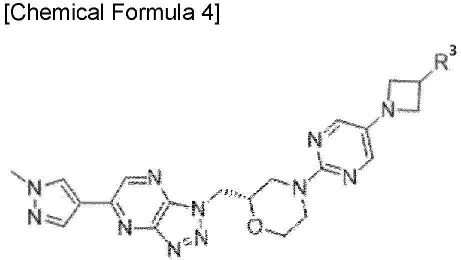 wherein, R² and R³ are each independently hydrogen, t-butoxycarbonyl, benzyloxycarbonyl, Tosyl, or substituted or non-transmuted C1 to C6 straight or branched, or cyclic alkyl group.

6. The compound of Claim 10, wherein R² and R³ are each independently a C1 to C6 straight, branched chain or cyclic alkyl group unsubstituted or substituted with halogen or R⁵,
wherein, R⁵ is (i) a piperazine substituted or non-t-butoxycarbonyl, benzyloxycarbonyl, or Tosyl, (ii) a C1 to C6 alkoxy group of unsubstituted or substituted with benzyl, phenyl, pyridine, or Tosyl group substituted with R⁶ (wherein, R⁶ is C1 to C6 alkoxy, amino, t-butoxycarbonyl group, benzyloxycarbonyl group, or amino substituted with Tosyl group), or (iii) hydroxy group.

7. The compound of Claim 1, wherein R² and R³ are each independently a C1 to C6 straight, branched chain or cyclic alkyl group unsubstituted or substituted with halogen or R⁵,
wherein, R⁵ is (i) a piperazine substituted or non-t-butoxycarbonyl or benzyloxycarbonyl, (ii) a C1 to C6 alkoxy group unsubstituted or substituted with benzyl, phenyl, pyridine, or Tosyl group substituted with R⁶ (wherein, R⁶ is C1 to C6 alkoxy, amino, t-butoxycarbonyl group, benzyloxycarbonyl group, or amino substituted with Tosyl group), or (iii) hydroxy group.

8. The compound of Claim 1, wherein R² is hydrogen or a C1 to C6 straight or branched alkyl chain or cyclic alkyl group; and
R³ is a straight or branched, or cyclic alkyl group of C1 to C4 unsubstituted or substituted with R⁵,
wherein, R⁵ may be (i) a piperazine, (ii) an C1 to C4 alkoxy group unsubstituted or substituted with a benzyl, phenyl, or pyridine unsubstituted or substituted with R⁶ (wherein, R⁶ is an C1 to C4 alkoxy group or amino group of), or (iii) a hydroxy group.

9. A compound selected from the group of the following compounds or a pharmaceutically acceptable salt thereof:
tert-butyl(S)-3-((4-(2-(2-((5-(1-methyl-1H-pyrazole-4-yl)-1H-[1,2,3]triazolo[4,5-b]pyrazine-1-yl)methyl)morpholino)pyrimidine-5-il)ppiperazine-1-yl)methyl)azetidine-1-carboxylate;
(S)-4-(5-(4-(azetidine-3-ylmethyl)piperazine-1-yl)pyrimidine-2-yl)-2-((5-(1-methyl-1H-pyrazole-4-yl)-1H-[1,2,3]triazolo[4,5-b]pyrazine-1-yl)methyl)morpholine;
(S)-2-((5-(1-methyl-1H-pyrazole-4-yl)-1H-[1,2,3]triazolo[4,5-b]pyrazine-1-yl)methyl)-4-(5-(4-((1-methylazetidine-3-yl)methyl)piperazine-1-yl)pyrimidine-2-yl)morpholine;
Benzyl(S)-4-((1-(2-(2-((5-(1-(1H-pyrazole-4-yl)-1H-[1,2,3]triazolo[4,5-b]pyrazine-1-yl)methyl)morpholino)pyrimidine-5-yl)azetidine-3-yl)methyl)piperazine-1-carboxylate;
(S)-2-((5-(1-methyl-1H-pyrazole-4-yl)-1H-[1,2,3]triazolo[4,5-b]pyrazine-1-yl)methyl)-4-(5-(3-(piperazine-1-ylmethyl)azetidine-1-yl)pyrimidine-2-yl)morpholine ;
(S)-4-(5-(3-((benzyloxy)methyl)azetidine-1-yl)pyrimidine-2-yl)-2-((5-(1-methyl-1H-pyrazole-4-yl)-1H-[1,2,3]triazolo[4,5-b]pyrazine-1-yl)methyl)morpholine;
(S)-(1-(1-(2-(2-((5-(1-methyl-1H-pyrazole-4-yl)-1H-[1,2,3]triazolo[4,5-b]pyrazine-1-yl)methyl)morpholino)pyrimidine-5-il)azetidine-3-yl)methanol;
(S)-(1-(2-(2-((5-(1-methyl-1H-pyrazole-4-yl)-1H-[1,2,3]triazolo[4,5-b]pyrazin-1-yl)methyl)morpholino)pyrimidine-5-yl)azetidine-3-yl)methyl 4-methylbenzenesulfonate;
tert-butyl(S)-4-((1-(2-(2-((5-(1-(1-methyl-1H-pyrazole-4-yl)-1H-[1,2,3]triazolo[4,5-b]pyrazine-1-yl)methyl)morpholino)pyrimidine-5-il)azetidine-3-yl)methyl)piperazine-1-carboxylate;
(S)-2-((5-(1-methyl-1H-pyrazole-4-yl)-1H-[1,2,3]triazolo[4,5-b]pyrazin-1-yl)methyl)-4-(5-(3-((4-methylpiperazine-1-yl)methyl)azetidine-1-yl)pyrimidine-2-yl)morpholine;
(S)-4-(5-(3-(((4-methoxybenzyl)oxy)methyl)azetidin-1-yl)pyrimidine-2-yl)-2-((5-(1-methyl-1H-pyrazole-4-yl)-1H-[1,2,3]triazolo[4,5-b]pyrazine-1-yl)methyl)morpholine;
tert-butyl(S)-(4-(((1-(2-(2-((5-(1-(1H-pyrazole-4-yl)-1H-[1,2,3]triazolo[4,5-b]pyrazine-1-yl)methyl)morpholino)pyrimidine-5-il)azetidine-3-il)methoxy)methyl)phenyl)carbamate; and
tert-butyl(S)-(5-(((1-(2-(2-((5-(1-(1H-pyrazol-4-yl)-1H-[1,2,3]triazolo[4,5-b]pyrazine-1-yl)methyl)morpholino)pyrimidine-5-il)azetidin-3-il)methoxy)methyl)pyridine-2-yl)carbamate.

10. A compound represented by any one of the following Chemical Formulas 5 to 7 or a pharmaceutically acceptable salt thereof: 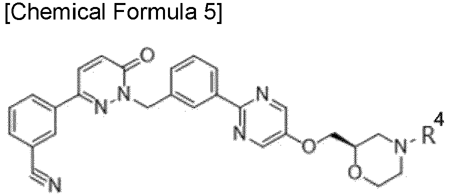 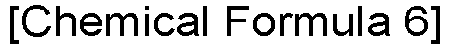 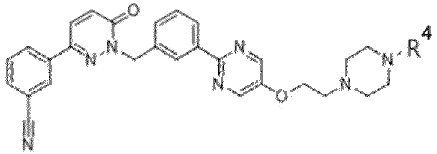 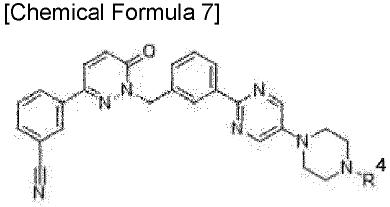 wherein,
R⁴ is hydrogen, t-butoxycarbonyl group or benzyloxycarbonyl group.

11. A compound selected from the group consisting of the following compounds or a pharmaceutically acceptable salt thereof:
tert-butyl(2R)-2-(((2-(3-((3-(3-cyanophenyl)-6-oxopridazine-1(6H)-yl)methyl)phenyl)pyrimidine-5-yl)oxy)methyl)morpholine-4-carboxylate;
(S)-3-(1-(3-(5-(morpholine-2-ylmethoxy)pyrimidine-2-yl)benzyl)-6-oxo-1,6-dihydropyridazine-3-yl)benzonitrile;
t-butyl-4-(2-{[2-(3-{[3-(3-cyanophenyl)-6-oxo-1,6-dihydropyridazine-1-yl]methyl}phenyl)pyrimidine-5-yl]oxy}ethyl)piperazine-1-carboxylate;
3-(6-oxo-1-(3-(5-(2-(piperazine-1-yl)ethoxy)pyrimidine-2-yl)benzyl)-1,6-dihydropyridazine-3-yl)benzonitrile;
t-butyl 4-(2-(3-((3-(3-(3-cyanophenyl)-6-oxopyridazine-1(6H)-yl)methyl)phenyl)pyrimidine-5-yl)piperazine-1-carboxylate; and
Preparation of 3-(6-oxo-1-(3-(5-(piperazine-1-yl)pyrimidin-2-yl)benzyl)-1,6-dihydropyridazine-3-yl)benzonitrile.

12. An anticancer composition comprising a compound of any of the claims 1 to 16.
